# EUROPEAN PATENT SPECIFICATION

(11) **EP 1 427 856 B1**
(45) Date of publication and mention of the grant of the patent: **11.05.2011**
(21) Application number: 02767487.8
(22) Date of filing: 13.09.2002
(51) Int. Cl.: C12Q 1/68, C12N 15/62, C12N 15/63, C12N 5/10, A01H 5/00, A01K 67/027, G01N 33/68, C12Q 1/25

(54) **FUSION PROTEINS USEFUL FOR DETECTING ANALYTES**
FUSIONSPROTEINE ZUR DETEKTION VON ANALYTEN
PROTEINES HYBRIDES DESTINEES A LA DETECTION D'ANALYTES

(30) Priority: 18.09.2001 EP 01122301; 08.04.2002 EP 02007373
(43) Date of publication of application: 16.06.2004
(60) Divisional application: 10182215.3
(73) Proprietor: CARNEGIE INSTITUTION OF WASHINGTON, Washington, D.C. 20005 (US)
(72) Inventor: FROMMER, Wolf-Bernd, San Francisco, CA 94110 (US); FEHR, Marcus, 72074 Tübingen (DE); LALONDE, Sylvie, 72127 Kusterdingen (DE)
(74) Representative: Lipscombe, Martin John
(86) International application number: PCT/EP2002/010340
(87) International publication number: WO 2003/025220

(56) References cited:
- WO-A-00/49183
- US-B1- 6 197 534
- MIYAWAKI A ET AL: "FLUORESCENT INDICATORS FOR CA2+ BASED ON GREEN FLUORESCENT PROTEINSAND CALMODULIN" NATURE, MACMILLAN JOURNALS LTD. LONDON, GB, vol. 388, no. 6645, 28 August 1997 (1997-08-28), pages 882-887, XP002058386 ISSN: 0028-0836
- MITRA R D ET AL: "Fluorescence resonance energy transfer between blue-emitting and red-shifted excitation derivatives of the green fluorescent protein" GENE, ELSEVIER BIOMEDICAL PRESS. AMSTERDAM, NL, vol. 173, no. 1, 1996, pages 13-17, XP004042847 ISSN: 0378-1119
- JENNE A ET AL: "Real-Time Characterization of Ribozymes by Fluorescence Resonance Energy Transfer (FRET)" ANGEWANDTE CHEMIE. INTERNATIONAL EDITION, VERLAG CHEMIE. WEINHEIM, DE, vol. 38, no. 9, 1999, pages 1300-1303, XP002226175 ISSN: 0570-0833
- BENSON D E ET AL: "Design of bioelectronic interfaces by exploiting hinge-bending motions in proteins" SCIENCE, 31 AUG. 2001, AMERICAN ASSOC. ADV. SCI, USA, vol. 293, no. 5535, pages 1641-1644, XP002231572 ISSN: 0036-8075 cited in the application

## Description

The present invention relates to fusion proteins comprising two detection portions and a periplasmic binding protein (PBP) portion between these two detection portions which undergoes a conformational change upon binding of a compound. The present invention also relates to nucleic acid molecules comprising a nucleotide sequence encoding such a fusion protein as well as to expression cassettes, vectors, transgenic plants and transgenic non-human animals comprising said nucleic acid molecules. In addition, the present invention relates to methods for detecting an analyte in a sample or in a cell using said fusion protein, and optionally, a control sensor allowing a calibration of analyte detection made by said fusion protein. The present invention also relates to such a control sensor and to nucleic acid molecules encoding it, as well as to diagnostic compositions, kits, and uses of the fusion protein and the control sensor for in vitro or in vivo analyte detection and for preparing a diagnostic composition.

The analysis of the composition of metabolites and solutes in different compartments of living cells of different tissues or organs is a time-consuming procedure and requires the disruption of the cells. Moreover, most techniques do not measure metabolite and solute changes in real-time and do not take into account temporal and spatial variations of local metabolite concentrations at the cellular or subcellular level. In addition, the methods known in the art have a low resolution and are prone to artifacts. For example, up to now the determination of the sugar and amino acid content in the apoplast of plant tissues is carried out by washing of the extracellular fluid (Büssis et al., Planta (1997) 202, 126-136). As one can easily imagine, no resolution in space is obtained and any information on the distribution of metabolite concentration thereby gets lost. Also to gain information on the subcellular distribution of such metabolites so far requires the analysis of respective compartments after a destructive approach (Büssis et al., Planta (1997) 202, 126-136). Thus, with respect to such measurements, there is no tool available that allows a differentiation between individual cells or subcellular compartments. The same holds true if one aims at elucidating transport processes, e.g. the sugar or amino acid import and export in vascular systems, e.g. sieve elements of leaves. Currently, it is not possible to determine the sugar or amino acid composition and concentration at the actual site of transport in the extracellular space or within the cell or the cellular compartments. Thus despite extensive knowledge about the properties of the transporters involved, their actual function remains unclear. A non-invasive technique for elucidating such transport processes, in particular regarding its compartmentation and time course, would thus be of significant advantage for understanding metabolism and transport.

The advent of fluorescent proteins has allowed non-invasive intracellular labeling, especially of peptides, which are easily detectable by optical means. The green fluorescent protein (GFP) from *Aequorea victoria* is now the most widely used reporter gene in many organisms. Multiple variants with different spectral properties have been developed. Furthermore, combinations of fluorescent proteins exhibiting energy transfer provide for differential fluorescence in response to conformational changes in the protein's immediate environment. Based on this principle, fusion constructs have been developed which allow to detect specific analytes such as calcium ions, cAMP or cGMP (e.g. WO 98/40477; Honda, Proc. Natl. Acad. Sci. USA 98 (2001), 2437-2442; Zaccolo, Nat. Cell. Biol. 2 (2000), 25-29). However, there are no reports on systems to measure, for example, primary metabolites *in vivo.* Thus, due to the limitation of the methods described in the prior art, in particular with regard to substrate specificity, there is an urgent need to provide further sensor molecules facilitating the measurement of a broad range of analytes such as organic compounds (e.g. sugars, amino acids, organic acids, vitamins), organic macromolecules and the like, but also anions like nitrate, sulfate or phosphate and metals such as Hg, Ni, Zinc and iron.

Apart from such requirements for detecting analytes *in vivo,* there is also a need for tools that allow a fast and easy to handle *in vitro* detection of analytes such as in samples taken from living organisms, nutrients or the environment. Recently, Benson (Science 293 (2001), 1641-1644) proposed a system using periplasmic binding proteins in connection with an electro-chemical detection method for in vitro detection of analyzes, however, with all of the limitations that electro-chemical measurements have.

US 6,197,534 (Lakowicz et al) is primarily concerned with sensor molecules which include a fluorophore and a quencher covalently attached to cysteine residues at certain permissive sites within a bacterial periplasmic binding protein (PBP). The document also discloses in passing a PBP molecule in which fluorescent proteins are fused at the N- and C- terminals. However, there is no disclosure or suggestion of the use of linkers to attach the fluorescent proteins to the PBP, and the disclosure is purely speculative, since the document does not contain any experimental data to suggest that such a fusion protein might constitute an operable sensor molecule.

Thus, the technical problem underlying the present invention is to provide means nd methods that allow a real time and easy to handle measurement of the concentration of compounds which are not yet accessible by prior art techniques. Preferably such means and methods should allow for *in vivo* measurements.

This technical problem is solved by the provisions of the embodiments as characterized in the claims.

Accordingly, the present disclosure relates to a fusion protein comprising:
(a) two detection portions, wherein
   (i) the first detection portion is an energy-emitting protein portion and the second detection portion is a fluorescent protein portion; or
   (ii) the two detection portions are portions of a split fluorescent protein; and
(b) a periplasmic binding protein (PBP) portion between said two detection portions, which undergoes a conformational change upon binding of a compound;
wherein said conformational change results in a change of the energy emitted by said two detection portions.

The fusion protein of the present disclosure is useful for detecting analytes in any given liquid. The term "analyte" used in connection with the present invention refers to compounds that can be bound by a PBP and generate a conformational change in the PBP. It has surprisingly been found that such a conformational change of a PBP can be utilized in that it can change the relative position, i.e. the distance, orientation and/or the spatial relationship, of two detection portions which are fused to said PBP in a way that an energy emission generated by the detection portions is detectably altered. The term "and/or" wherever used herein includes the meaning of "and", "or" and "all or any other combination of the elements connected by said term". The term "energy emission" refers to an optical signal, preferably in the visible spectrum of light, that can be detected by suitable devices known in the art. A change of energy emission may include a significant change, i.e. increase or decrease, of light intensity at a given wave length or a significant change in the ratio of the light intensities at two or more different wavelengths compared with each other (herein also referred to as "ratiometric measurements"). Preferably, a fusion protein of use in the invention shows a change of light intensity or of the ratio between light intensities at two or more different wavelengths of at least 0.1 %, preferentially at least 0.5%, more preferably at least 1%, still more preferably at least 2%, even more preferably at least 5%, most preferably at least 10% of the state where the analyte is not bound to the PBP portion compared to the bound state.

In the prior art there have been some approaches to employ a binding protein moiety fused at its N- and C-terminal flanks to green fluorescent proteins. For instance, WO 98/40477 describes such a system based on calmodulin which is useful for the measurement of calcium concentrations. However, this system requires as a second analyte binding portion a calcium-calmodulin binding target peptide moiety to which the conformationally flexible calmodulin binds. Thus, the applicability of the fusion protein of WO 98/40477 is restricted because of the requirement of two binding portions and the use of a ubiquitous and endogenous regulatory component. Thus, other cellular components might interact with the calmodulin or helper peptide moiety of this calcium indicator, making it highly sensitive to artefacts. This calcium-calmodulin based approach has been described in further detail e.g. by Miyawaki (Nature 388 (1997), 882-887; Proc. Natl. Acad. Sci. USA 96 (1999), 2135-2140; Methods Enzymol. 327 (2000), 472-501), Romoser (J. Biol. Chem. 272 (1997), 13270-13274) and Allen (Plant J. 19 (1999), 735-747). Furthermore; Zaccolo (Nat. Cell. Biol. 2 (2000), 25-29) describes the use of a cAMP binding portion of a protein kinase A and Honda (Proc. Natl. Acad. Sci. USA 98 (2001), 2437-2442) a portion of an inactivated cGMP-dependant protein kinase for constructing similar fusion proteins in order to measure cAMP or cGMP concentration *in vivo.* It is thus difficult to expand this system directly to use in detection of other analytes. Also, these proteins are subject to endogenous allosteric regulators which can affect conformation and thus the output signal. However, the use of PBP for measuring analyte concentrations by a fusion protein as provided by the present invention has so far not been described. Thus, the present invention opens up the application of the versatility of PBPs as regards their binding-specificity for a broad spectrum of compounds to sensoring fusion proteins which are easy to use and, in particular, accessible to *in vivo* detection by recombinant techniques. Furthermore, since most PBPs are bacterial proteins, interference with endogenous regulators in eukaryotes is highly unlikely. There have been some attempts to make use of a PBP for measuring analytes *in vitro,* wherein a conformational change upon binding of the analyte generates a change of fluorescence (Zhou, Biosens. Bioelectron. (1991), 445-450; Gilardi, Anal. Chem. 66 (1994), 3840-3847; Tolosa, Anal. Biochem. 267 (1999), 114-120). However, in all of these attempts, only one fluorescent moiety was attached to the PBP, thus, not allowing ratiometric measurements and, in addition, the fluorescent moieties used were fluorophores that were attached to the PBP by chemical linkage. Thus, these sensor molecules were not amenable to *in vivo* analyte detection, particularly by applying recombinant expression techniques with all of their advantages such as subcellular targeting for compartmental analyses.

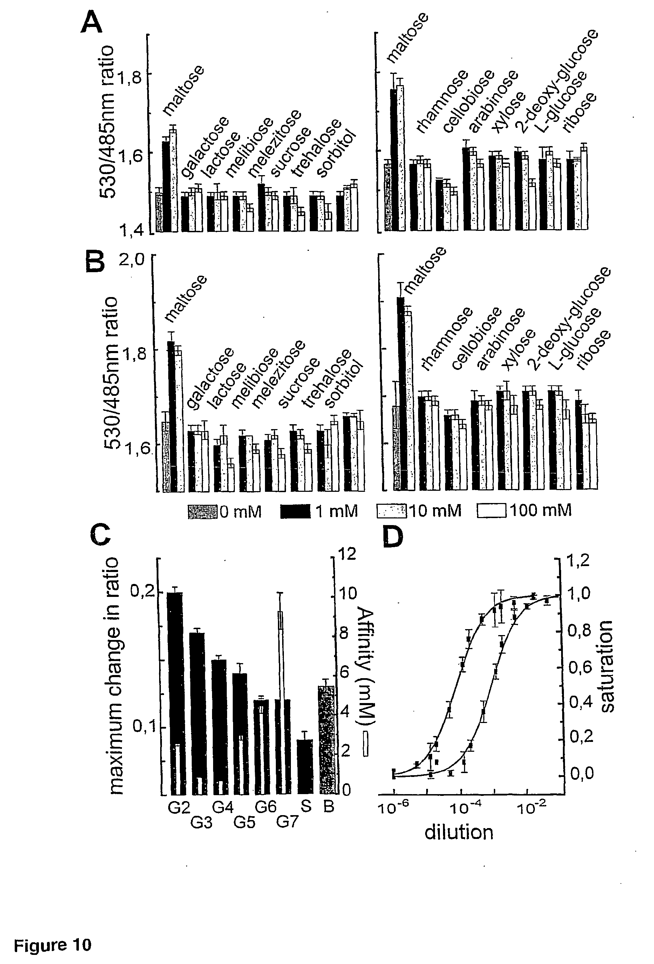

In the experiments made in connection with the present invention, it has surprisingly been shown that PBPs are particularly well suited for constructing fusion proteins for analyte detection. Significant changes in the ratio of resonance energy transfer are normally only obtained if there is a significant change in the distance between the two detection portions. However, since, for instance, the distance between the N- and the C-terminus of the maltose binding protein changes only by 1 nm upon substrate binding (Hall, J. Biol. Chem 272 (1997), 17610-17614) one had to expect that this change in distance is insufficient for obtaining a detectable change in the ratio of light intensities at two different frequencies due to resonance energy transfer (RET). Surprisingly, the present disclosure shows that nevertheless PBPs are suitable for such measurements. Despite the small overall size of PBPs and the corresponding small change in the distance of the two lobes upon binding, the torsion of the molecule brings about a change in relative position of the two detection portions sufficient for a change in RET. Thus, by suitable modifications of the PBP portion it was possible to engineer fusion proteins in a way that, despite of the small size of PBPs, detectable RET ratio changes were generated upon substrate binding. Furthermore, in view of the notoriously high binding affinity of free PBPs to their specific substrates and the observation that they release their bound substrate only upon binding to a transporter localized in the inner bacterial membrane, this could not be expected Chen et al., PNAS 98 (2001), 1525-1530). Accordingly, it has been shown that a fusion protein (FLIPmal) containing the ultrahigh affinity wild-type maltose-binding protein (MBP) as the PBP portion could not even be purified in a way that it was free of maltose bound (Example 1, infra; Silhavy et al., PNAS 72 (1975), 2120). However, by introducing specific amino acid substitutions into the PBP portion, the binding affinity to maltose could be reduced with the result that the fusion proteins containing this PBP protion were amenable to maltose concentration measurements at practically interesting concentration ranges, e.g. between 1 and 5000 µM (FLIPmal W62A and FLIPmal W230A, Example 2, Table 3). The fusion proteins retain the substrate specificity for maltose and oligomaltosides (Fig. 10A and B). Obviously an increasing chain length of the oligomaltoside leads to a reduced closure movement so that the maximum change in the FRET ratio decreases with the chain length (Fig. 10C). FRET measurements allow to directly monitor the concentration of compounds in a fluid. This has been demonstrated by applying FLIPmal fusion proteins that were successfully used to determine the presence of maltosides in beer samples (Fig. 10D).

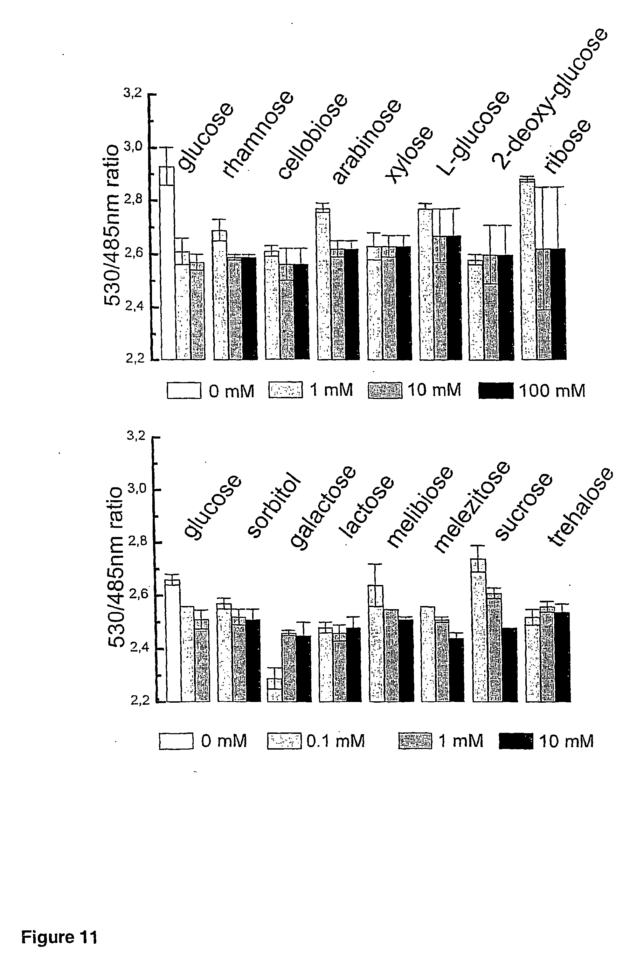

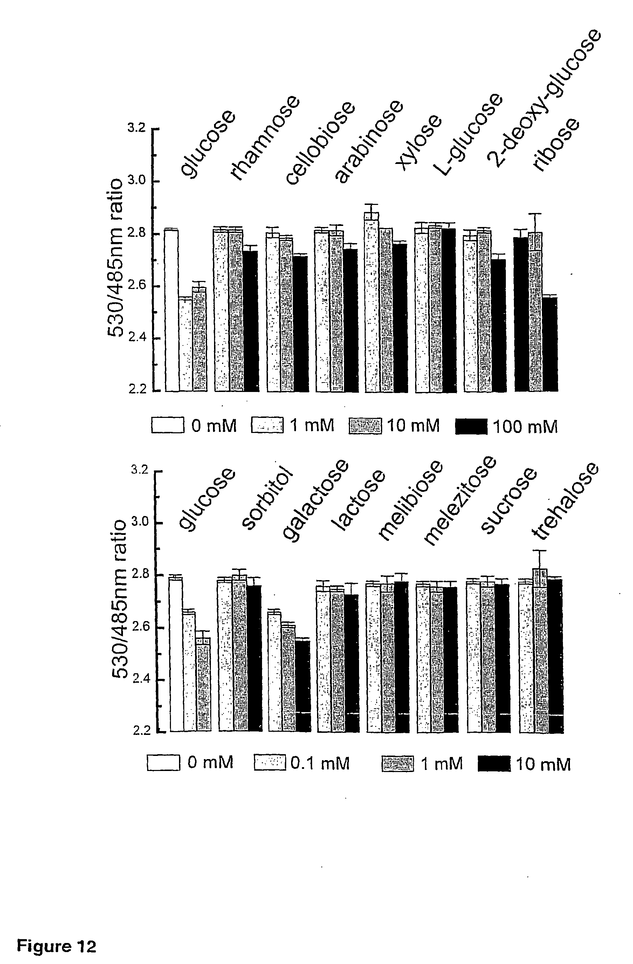

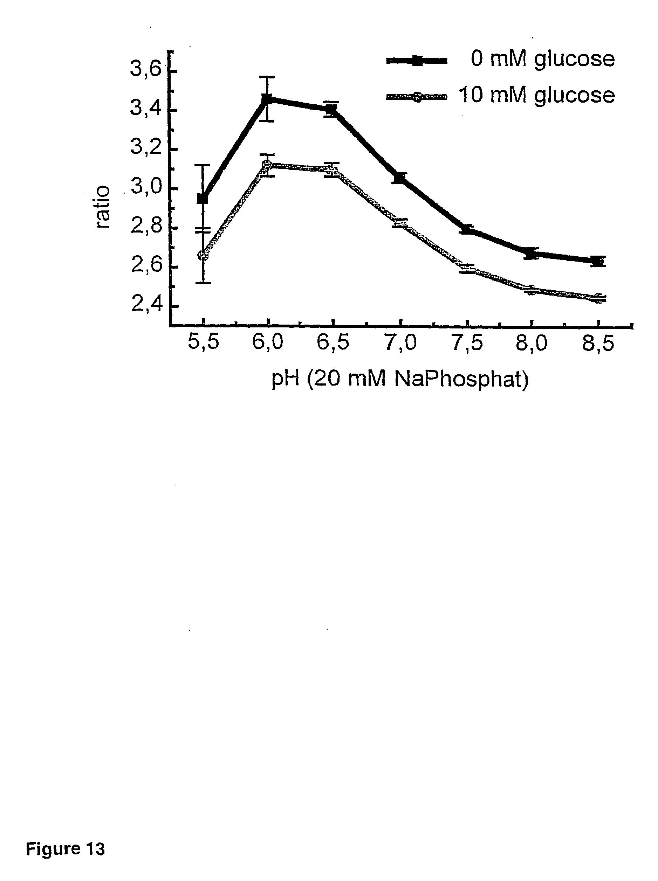

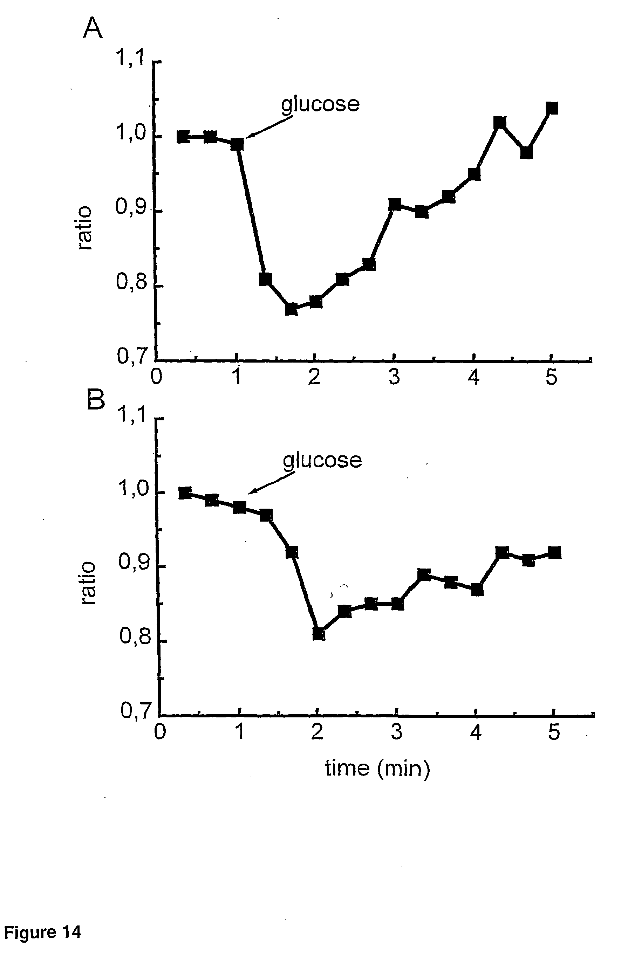

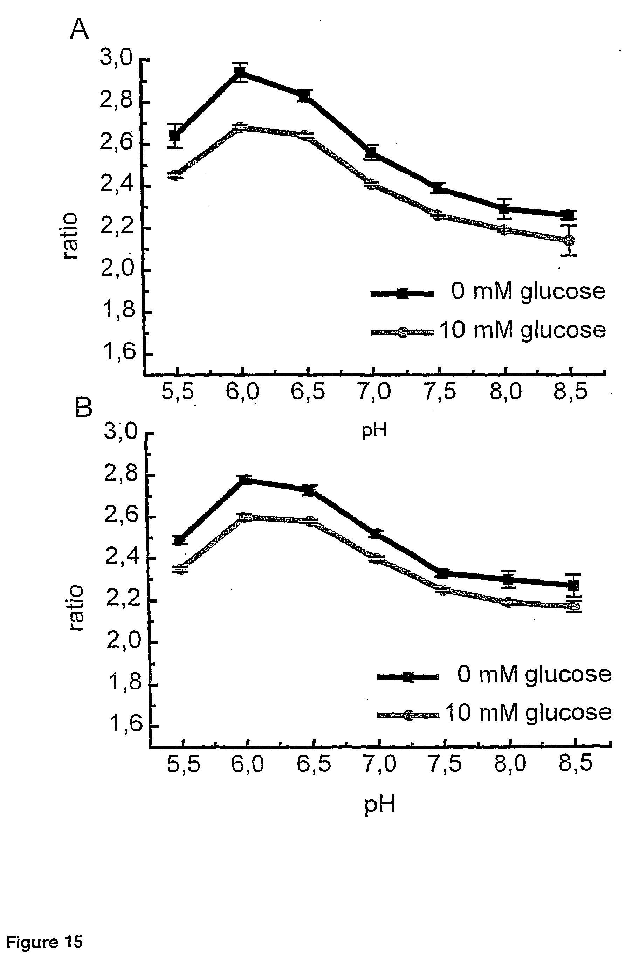

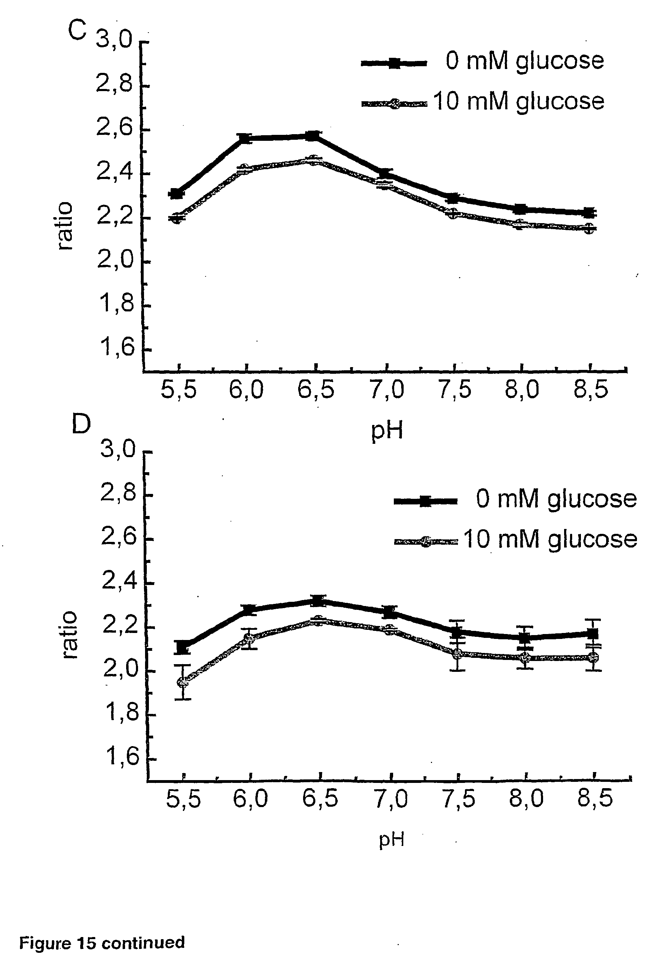

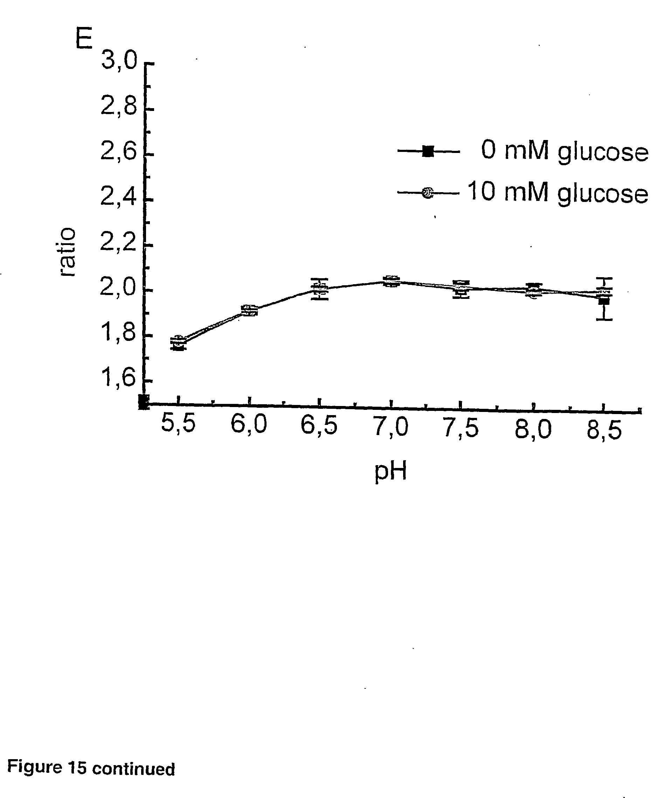

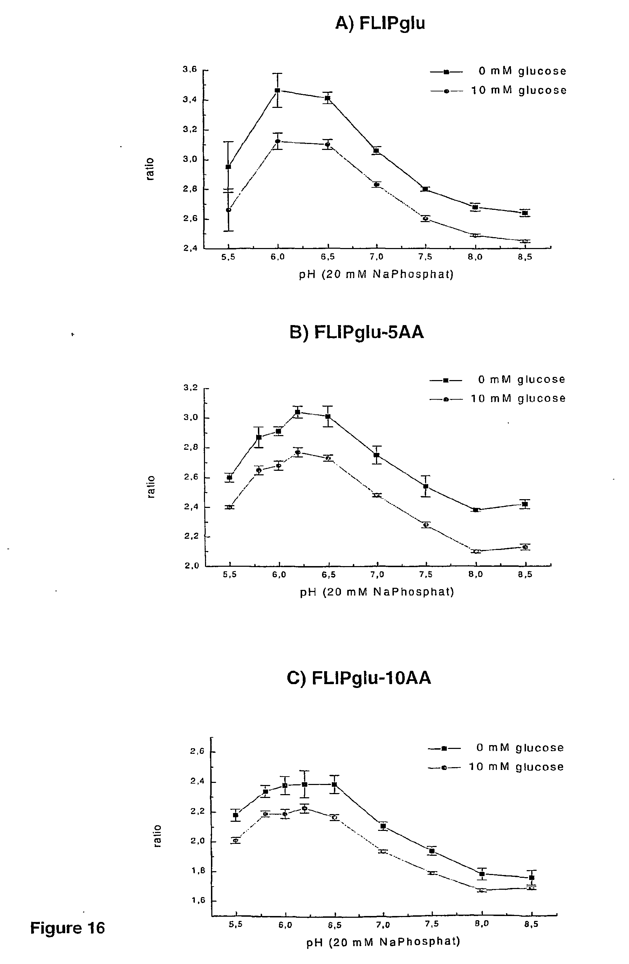

Furthermore, it could be shown that the principle developed for the maltose binding protein can be applied to other PBPs even belonging to a different PBP-family and sharing little homology at the primary sequence level (PBP-like I; see below) by constructing fusion proteins containing the glucose/galactose binding protein (GGBP) and the ribose binding protein (RBS) as the PBP portion. Here, glucose concentrations of 0.01 to 5000 µM were found to be reliably detectable using fusion proteins comprising a wild-type GGBP portion or a modified GGBP portion with a reduced binding affinity (Example 2, Table 3). Ribose could be quantified at concentrations between 0.04 and 3 µM using the wild-type RBS as the PBP portion (Example 2, Table 3). Thus, it can be expected that the principle to construct analyte-detecting fusion proteins as described herein can be extended to PBPs in general. With regard to fusion proteins comprising a GGBP portion, two aspects have to be taken into account when applying them for analyte detection. On the one hand, such fusion proteins generally have a relatively broad range of binding substrates. Therefore, multiple sugars may be measured at the same time (e.g. as shown by in vitro measurements: galactose, disaccharides, trisaccharides, pentoses are recognized; Figure 11). Thus, they may be of limited applicability for specifically determining glucose concentrations in vitro or in vivo when the presence of such other sugars can be expected. Furthermore, within the physiological range, GGBP fusion proteins are shown to be sensitive to pH changes (Fig. 13). This was also apparent from in vivo imaging experiments using the glucose binding protein control sensor FLlPglu D236A (Fig. 14). In view of these findings, mutants were generated and screened for novel properties, i.e. a higher selectivity for glucose and/or an increased insensitivity to pH changes. The mutant FLIPglu F16A turned out to be much more selective for glucose (Fig. 12). In addition, GGBP fusion proteins in which histidines were modified so that their side chain cannot be protonated were considerably more insensitive to pH (Fig. 15). FLIPglu-5AA and FLIPglu-10AA containing a GGBP that is lacking the last 5 and 10 amino acids, respectively, also showed an increase insensitivity to pH (Fig. 16). A mutant that combines these properties (i.e. improved glucose selectivity and reduced pH sensitivity) can now be used for glucose detection in which the above-mentioned restrictions connected for example with the wild type GGBP do not apply. Furthermore, according to the findings described above, glucose binding protein control sensors that are inactive in analyte binding but otherwise show the same characteristics as the corresponding GGBP fusion protein (e.g., regarding sensitivity to halide ions or pH) can be used to monitor pH changes and to calibrate glucose measurements, e.g. by in vivo imaging.

The term "periplasmic binding protein" or "PBP" refers to proteins that are characterized by a three-dimensional lobe-hinge-lobe structure and which upon binding of an analyte undergo a conformational change sufficient and suitable for analyte detection as mentioned above. The lobes are globular to ellipsoid. The hinge region preferably contains two or more amino acid strands. This structure is furthermore characterized by the presence of a cleft between the lobes, which is the site of substrate binding, i.e. binding of the compound to be analyzed. Upon binding of a compound, the PBP undergoes a conformational change, whereby the two lobes change relative positions, a movement which is also often referred to as that of a Venus flytrap or Pacman or, as it is preferred, as a hinge-twist motion. Advantageously, the PBP for use in a fusion protein of the invention has no enzymatic activity. Table 1 shows examples of PBPs from which the three-dimensional structure is already known. In addition, proteins of Table 1, which have not yet been characterized structurally, can be modelled on the basis of the existing structures according to methods known In the art. The PBP useful for the fusion protein used in the invention has the same three-dimensional structure as these examples or is similar thereto. Thus, they show the above mentioned lobe-hinge-lobe structure and the conformational change upon binding of a compound. The person of average skill in analyzing three-dimensional protein structures knows how to identify proteins having such a lobe-hinge-lobe structure. It is furthermore possible for the person skilled in the art to identify among the proteins that match these structural criteria those who show a conformational change as mentioned above. For this purpose, he/she can compare three-dimensional structure data from a protein having bound its substrate with data from one not bound. Methods for determining the three-dimensional structure of proteins are well known to the person skilled in the art and are described in the literature such as in Mancini, Structure (1997) 5, 741-750 (cryoelectron microscopy), Qian, Biochemistry (1998) 37, 9316-9322 (NMR), Shilton, J. Mol. Biol. (1996) 264, 350-363 (modeling on the basis of related structures) and Spurlino, J. Biol. Chem. (1991) 266, 5202-5219 (X-ray crystallography).

Preferably, the PBP in a fusion protein according to the present invention belongs to one of the protein superfamilies "periplasmic binding protein like I" (PBP-like I), "periplasmic binding protein like II" (PBP-like II) or "helical backbone" metal receptor superfamily according to the nomenclature of the structural classification of proteins (SCOP; Murzin, J. Mol. Biol. 247 (1995), 536-540; http:/scop.mrc-Imb.cam.ac.uk/scop/index.htm), to the families as defined by Saier et al., 1993 Microbiol Rev. 57, 320-346 or to the binding proteins classified as being "ATP-dependent" in the compilation of transport proteins shown under www.biology.ucsd.edu/~ipaulsen/transport. The structure of members of these superfamilies are characterized to consist of two similar lobe domains of parallel β-sheets and adjacent alpha helices fused by a hinge region consisting of at least two strands. The glucose/galactose binding protein (GGBP) for example belongs to the superfamily PBP-like I and the maltose binding protein (MBP) to the superfamily PBP-like II.

The PBPs to be taken for constructing a fusion protein useful in the invention may be naturally occurring PBPs. Preferably, such PBPs originate from gramnegative bacteria and especially are localized in the periplasm. Also preferred are extracellular binding proteins from gram-positive bacteria. Examples of such PBPs are given in Table 1. From these, MBP having the amino acid sequence shown in SEQ ID NO:2 and GGBP having the amino acid sequence shown in SEQ ID NO:4 are most preferred.

**Table 1: Periplasmic binding proteins suitable for the fusion protein of the invention. Where appropriate, the species identified is the one for which the 3D-structure is known. At present, the 3D-structures of PBPs are mostly known from E.coli and S. typhimurium. These structures are shown at http://scop.mrc-lmb.cam.ac.uk/scop/index.htm or at hftp://ncbi.nlm.nih.qov. The column headed "3D" indicates whether three-dimensional, open ("o") or closed ("c") structures of the respective PBP are available in the literature.**

| **Gene name** | **Substrate** | **Species** | **3D** | **Reference** |
|---|---|---|---|---|
| alsB | Allose | *E.coli* | -/c | J. Bacteriol. (1997) 179, 7631-7637 J. Mol. Biol. (1999) 286, 1519-1531 |
| araF | Arabinose | *E.coli* | -/c | J. Mol. Biol. (1987) 197,37-46 J. Biol. Chem. (1981) 256, 13213-13217 |
| AraS | arabinose/fructose/xylose | *S.solfataricus* | -/- | Mol. Microbiol. (2001) 39, 1494-1503 |
| argT | lysine/arginine/ornithine | *Salmonella typhimurium* | o/c | Proc. Natl. Acad. Sci. U S A (1981) 78, 6038-6042 J. Biol. Chem. (1993) 268, 11348-11355 |
| artI | Arginine | *E.coli* | | Mol. Microbiol. (1995) 17, 675-686 |
| artJ | Arginine | *E.coli* | | Mol. Microbiol. (1995) 17, 675-686 |
| b1310 | Unknown (putative, multiple sugar) | *E.coli* | -/- | NCBI accession A64880 |
| b1487 | Unknown (putative, oligopeptide binding) | *E.coli* | -/- | NCBI accession B64902 |
| b1516 | Unknown (sugar binding protein homolog) | *E.coli* | -/- | NCBI accession G64905 |
| butE | vitamin B12 | *E.coli* | -/- | J. Bacteriol. (1986) 167, 928-934 |
| CAC1474 | proline/glycine/betaine | *Clostridium acetobutylicum* | -/- | NCBI accession AAK79442 |
| cbt | Dicarboxylate (Succinate, malate,fumarat) | *E.coli* | -/- | J. Supramol. Struct. (1977) 7, 463-80 J. Biol. Chem. (1978) 253, 7826-7831 J. Biol.Chem. (1975) 250, 1600-1602 |
| CbtA | Cellobiose | *S.solfataricus* | -/- | Mol. Microbiol. (2001) 39, 1494-1503 |
| ChvE | Sugar | *A.tumefaciens* | -/- | J. Bacteriol. (1990) 172, 1814-1822 |
| CysP | Thiosulfate | *E.coli* | -/- | J. Bacteriol. 172:3358-3366(1990). |
| dctP | C4-dicarboxytate | *Rhodobacter capsulatus* | -/- | Mol. Microbiol. (1991) 5, 3055-3062 |
| dppA | Dipeptide | *E.coli* | o/c | Biochemistry (1995) 34, 16585-16595 |
| FbpA | Iron | *Neisseria gonorrhoeae* | -/c | J. Bacteriol. (1996) 178, 2145-2149 |
| fecB | Fe(III)-dicitrate | *E.coli* | | J. Bacteriol. (1989) 171, 2626-2633 |
| fepB | enterobactin-Fe | *E.coli* | -/- | J. Bacteriol. (1989) 171, 5443-5451 Microbiology (1995) 141, 1647-1654 |
| fhuD | Ferrichydroxamate | *E.coli* | -/c | Mol. Gen. Genet. (1987) 209, 49-55 Nat. Struct. Biol. (2000) 7, 287-291 Mol. Gen. Genet. (1987) 209, 49-55 |
| FliY | Cystine | *E.coli* | -/- | J. Bacteriol. 178 (1), 24-34 (1996) NCBI accession P39174 |
| GICS | glucose/galactose/ mannose | *S.solfataricus* | -/- | Mol. Microbiol. (2001) 39, 1494-1503 |
| glnH (protein: GLNBP) | Glutamine | *E.coli* | o/- | Mol. Gen. Genet. (1986) 205, 260-9 J. Mol. Biol. (1996) 262, 225-242 J. Mol. Biol. (1998) 278, 219-229 |
| gntX | Gluconate | *Ecoli* | -/- | J.Basic. Microbiol. (1998) 38, 395-404 |
| hemT | Haemin | *Y.enterocolitica* | -/- | Mol. Microbiol. (1994) 13, 719-732 |
| hisJ (protein: HBP) | Histidine | *E.coli* | -/C | Biochemistry (1994) 33, 4769-4779 |
| hitA | Iron | *Haemophilus influenzae* | o/c | Nat. Struct. Biol. (1997) 4, 919-924 Infect. Immun. (1994) 62, 4515-25 J. Biol. Chem. (195) 270, 25142-25149 |
| livJ | leucine/valine/ isoleucine | *E.coli* | -/c | J. Biol. Chem. (1985) 260, 8257-8261 J. Mol. Biol. (1989) 206, 171-191 |
| livK (protein: L-BP) | Leucine | *E.coli* | -/c | J. Biol. Chem. (1985) 260, 8257-8261 J. Mol. Biol. (1989) 206, 193-207 |
| malE (protein: MBP) | maltodextrine/ maltose | *E.coli* | o/c | Structure (1997) 5, 997-1015 J. Bio.I Chem. (1984) 259, 10606-13 J. Bio.I Chem. (1984) 259, 10606-13 |
| mglB (protein: GGBP) | glucose/galactose | *E.coli* | -/c | J. Mol. Biol. (1979) 133, 181-184 Mol. Gen. Genet. (1991) 229, 453-459 |
| modA | Molybdate | *E. coli* | -/c | Nat. Struct. Biol. (1997) 4, 703-707 Microbiol. Res. (1995) 150, 347-361 |
| MppA | L-alanyl-gamma-D-glutamyl-meso-diaminopimelate | *E.coli* | | J. Bacteriol. (1998) 180, 1215-1223 |
| nasF | nitrate/nitrite | *Klebsiella oxytoca* | -/- | J. Bacteriol. (1998) 180, 1311-1322 |
| nikA | Nickel | *E.coli* | -/- | Mol. Microbiol. (1993) 9, 1181-1191 |
| opBC | Choline | *B. Subtilis* | -/- | Mol. Microbiol. 32 (1), 203-216 (1999) |
| OppA | Oligopeptide | *Salmonella typhimurium* | o/c | Biochemistry (1997) 36, 9747-9758 Eur. J. Biochem. (1986) 158,561-567 |
| PhnD | Alkylphosphonate | *E.coli* | -/- | J. Biol. Chem. 265:4461-4471(1990). |
| PhoS (Psts) | Phosphate | *E.coli* | -/c | J. Bacteriol. (1984) 157, 772-778 Nat. Struct. Biol. (1997) 4, 519-522 |
| potD | putrescine/ spermidine | *E.coli* | -/c | J. Biol. Chem. (1996) 271, 9519-9525 |
| potF | Polyamines | *E.coli* | -/c | J. Biol. Chem. (1998) 273, 17604-17609 |
| proX | Betaine | *E.coli* | | J. Biol. Chem. (1987) 262, 11841-11846 |
| rbsB | Ribose | *E.coli* | o/c | J. Biol. Chem. (1983) 258, :12952-6 J .Mol. Biol. (1998) 279, 651-664 J. Mol. Biol. (1992) 225,155-175 |
| SapA | Peptides | *S. typhimurium* | -/- | EMBO J. 12 (11), 4053-4062 (1993) |
| sbp | Sulfate | *Salmonella typhimurium* | -/C | J. Biol. Chem. (1980) 255, 4614-4618 Nature (1985) 314, 257-260 |
| TauA | Taurin | *E.coli* | -/- | J. Bacteriol. 178 (18), 5438-5446 (1996) NCBI accession Q47537 |
| TbpA | Thiamin | *E.coli* | -/- | J. Biol. Chem. 273 (15), 8946-8950 (1998) NCBI accession P31550 |
| tctC | Tricarboxylate | *Salmonella typhimurium* | -/- | |
| TreS | Trehalose | *S.solfataricus* | -/- | Mol. Microbiol. (2001) 39, 1494-1503 |
| tTroA | Zinc | *Treponema pallidum* | -/C | Gene (1997) 197, 47-64 Nat. Struct. Biol. (1999) 6, 628-633 |
| UgpB | sn-glycerol-3- phosphate | *E.coli* | -/- | Mol. Microbiol. (1988) 2, 767-775 |
| XylF | Xylose | *E. coli* | -/- | Receptors Channels (1995) 3, 117-128 |
| YaeC | Unknown (putative) | *E.coli* | -/- | J Bacteriol 1992 Dec;174(24):8016-22 NCBl accession P28635 |
| YbeJ(Gltl) | glutamate/aspartate (putative, superfamily: lysine-arginine-ornithine-binding protein) | *E.coli* | -/- | NCBI accession E64800 |
| YdcS(b1440) | Unknown (putative, spermidine) | *E.coli* | -/- | NCBI accession P76108 |
| YehZ | Unknown (putative) | *E.coli* | -/- | NCBI accession AE000302 |
| YejA | Unknown (putative, homology to periplasmic oligopeptide-binding protein - *Helicobacter pylon*) | *E.coli* | -/- | NCBI accession AAA16375 |
| YgiS (b3020) | Oligopeptides (putative) | *E.coli* | -/- | NCBI accession Q46863 |
| YhbN | Unknown | *E.coli* | -/- | NCBI accession P38685 |
| YhdW | Unknown (putative, amino acids) | *E.coli* | -/- | NCBI accession AAC76300 |
| YliB (b0830) | Unknown (putative, peptides) | *E.coli* | -/- | NCBI accession P75797 |
| YphF | Unknown (putative sugars) | *E.coli* | -/- | NCBI accession P77269 |
| Ytrf | Acetoin | *B.subtilis* | -/- | J. Bacteriol. (2000) 182, 5454-5461 |

Likewise, homologues of the PBPs mentioned in Table 1 may be used for carrying out the present invention such as orthologues originating from related species of gram-negative bacteria. Such homologues may for instance be characterized in that they are encoded by a nucleotide sequence which hybridizes, preferably under stringent conditions, to a nucleotide sequence encoding a PBP as depicted in Table 1 and have the properties of a PBP as explained above.

In this context the term "hybridization" means hybridization under conventional hybridization conditions. They may be low stringent, preferably stringent (i.e. high stringent) hybridization conditions, as for instance described in Sambrook at al., Molecular Cloning, A Laboratory Manual, 2nd edition (1989) Cold Spring Harbor Laboratory Press, Cold Spring Harbor, NY. In an especially preferred embodiment the term "hybridization" means that hybridization occurs under the following conditions:

| | |
|---|---|
| Hybridization buffer: | 2 x SSC; 10 x Denhardt solution (Fikoll 400 + PEG + BSA; ratio 1:1:1); 0.1% SDS; 5 mM EDTA; 50 mM Na₂HPO₄; |
| | 250 µg/ml of herring sperm DNA; 50 µg/ml of tRNA; or |
| | 0.25 M of sodium phosphate buffer, pH 7.2; |
| | 1 mM EDTA |
| | 7% SDS |
| Hybridization temperature T | = 60°C |
| Washing buffer: | 2 x SSC; 0.1 % SDS |
| Washing temperature T | = 60°C |

Advantageously, nucleotide sequences encoding a homologous PBP are at least 60%, preferably at least 70%, more preferably at least 80% and most preferably at least 90% identical with the nucleotide sequence encoding a PBP mentioned in Table 1. Likewise, such a homologous PBP has an amino acid sequence of at least 60%, preferably of at least 70%, more preferably of at least 80%, still more preferably of at least 90% and most preferably of at least 95% identity to the amino acid sequence of a PBP mentioned in Table 1.

Furthermore, encompassed by the term "PBP" are functional analogues of the PBPs of gram-negative bacteria. Such functional analogues likewise show a lobe-hinge-lobe structure, they can bind compounds and show a conformational change upon compoundbinding as described above. For instance, such functional analogues include homologues of PBPs from gram-negative bacteria which are found in gram-positive bacteria (Quiocho, Mol. Microbiol. 20 (1996), 17-25; Gilson, EMBO J. 7 (1988), 3971-3974; Turner, J. Bacteriol (1999) 181: 2192-2198). These homologues are extracellular binding lipoproteins in most cases maintained at the outer cell surface membrane by embedding their N-terminal glyceride cysteine into the lipid bilayer (Gilson, loc. cit.). For example, MalX is a maltose-inducible membrane-bound protein of *Streptococcus pneumoniae* that is homologous to the MBP of gram-negative bacteria. Similarly, AmiA of S. pneumoniae is a homologue of the oligopeptide PBP OppA (Gilson, loc. cit.). BspA is a cystine binding protein not linked to the membrane (Turner, loc. cit.).

Further examples of functional PBP analogs comprise proteins belonging to the above-mentioned superfamilies, which are not present in the periplasm of gram-negative bacteria such as the amide receptor/negative regulator of the amidase operon (AmiC) from *Pseudomonas aeruginosa,* the lac-repressor (lacR) core C-terminal domain from *E.coli* as examples of the PBP-like I superfamily and thiaminase I from *Bacillus subtilis* or the putrescine receptor (potF) from *E.coli* as examples of the PBP-like II superfamily.

Moreover, it is contemplated that the term "PBP" includes derivatives of any one of the above-mentioned periplasmic binding proteins as long as such derivatives have the above-described lobe-hinge-lobe structure, binding capacity and suitable conformational change upon binding of a compound. The skilled person is capable of modifying and optimizing naturally occurring PBPs by suitable techniques known in the art such as *in vitro* or *in vivo* mutagenesis, PCR shuffling mutagenesis, chemical modification and the like.

Preferentially, such derivatives are encoded by a nucleotide sequence which hybridizes, preferably under stringent conditions with a nucleotide sequence encoding a PBP as described above. For such derivatives, the above mentioned preferred hybridization conditions and sequence identities likewise apply. PBP derivatives may be modified by addition, deletion, shuffling, substitution, preferably by conservative amino acid substitution and the like with respect to a naturally occurring PBP. For instance, such alterations may be introduced in order to adapt a coding sequence to a certain codon usage of a given host organism or to modify the binding properties, i.e. the specificity for the compound or the binding affinity, or to improve the three-dimensional structure, e.g. the relative position and topology of the detection portions contained in the fusion protein and/or to improve the change in resonance energy transfer ratio or to increase the insensitivity to ionic conditions, e.g. the pH. The person skilled in the art is aware of suitable techniques of determining whether a derivative has a lobe-hinge-lobe structure characteristic of PBPs such as X-ray crystallography, NMR studies, modelling etc. and whether it undergoes a conformational change upon binding of a compound. To determine a conformational change upon binding of an analyte, the relative positions of both lobes in the crystal structure of the ligand-free conformation and in those of the conformation with a bound ligand are compared. A change of conformation will affect the relative position of the lobes. If a crystal structure is not available it can be modeled on the basis of related structures or a known structure of a related protein can directly be used as a model for the missing structure of the protein of interest. The technology described here allows directly determining the suitability of a given binding protein for analyte detection, e.g. by constructing respective fusion proteins and measuring responses upon titrating analytes. This can even be used to determine the function and specificity of a candidate protein.

The term "PBP portion" refers to a PBP or a part thereof which shows a conformational change upon binding a compound. For example, such a PBP portion may be truncated, i.e. N- and/or C-terminally deleted by one or more amino acid residues.

The term "conformational change" refers to any movement of the lobes relative to one another whereby the relative position between the first and the second detection portion flanking the PBP portion is changed resulting in a change of energy emitted by said detection portions. The term "relative position" refers to any possible kind of spatial relationship the two detection portions can have to one another such as distance and orientation. For instance, the conformation may change by rotation of one or both of the lobes, by folding up the PBP at the hinge, by twisting one or both of the lobes laterally or by any combination of these movements. Useful is a conformational change where the distance between the detection portions is altered. However, other changes such as those affecting the orientation of one detection portion relative to the other are also within the scope of the invention. Preferably, the PBP portion shows a combined hinge-twist motion upon binding of an alalyte. Where resonance energy transfer is used for detecting the conformational change, a small size of the PBP portion relative to the half-maximum RET distance R₀ (see below), allowing only a small change in distance and RET efficiency, a change in relative position by tilting or twisting may be preferable. In such cases, it is advantageous that, either before binding or upon binding, the detection portions are oriented in a way that at least half-maximum energy transfer takes place.

The term "compound" (also designated "analyte" or "substrate" throughout the present description) refers to any compound that can be bound by the PBP portion present as a fusion protein used in the invention. Depending on the analyte that is to be detected, a suitable PBP can be selected to construct the fusion protein. Examples of compounds detectable by the method of the invention are given in Table 1. Notably, PBPs may display a high selectivity as regards the compound as it is for example the case for the phosphate PBP (phoS) or the sulfate PBP (sbp). Other PBPs may have rather broad substrate selectivity as it is observed for leucine-isoleucine-valine (LIV) PBP, lysine-arginine-ornithine (LAO) PBP or the peptide PBPs (Quiocho, Mol. Microbiol. 20 (1996), 17-25).

Accordingly, as a preferred embodiment, the compound that is bound by the PBP portion of the fusion protein used in the invention is selected from the group consisting of sugars, amino acids, peptides, organic acids, anions, metals (e.g. molybolate, mercury, iron, zinc or nickel) or ions, oxides, hydroxides or conjugates thereof, inorganic ions (e.g. phosphate, sulfate or thiosulfate), (poly)amines and vitamins.

The term "binding of a compound" refers to a non-covalent, preferably reversible binding of a compound to the PBP portion which triggers a conformational change of the PBP portion as described above. Preferably, such binding involves non-covalent interactions such as salt bridges, hydrogen bonds, van der Waal forces, stacking forces, complex formation or combinations thereof between the compound and the PBP portion. It also includes interactions with water molecules in the binding pocket.

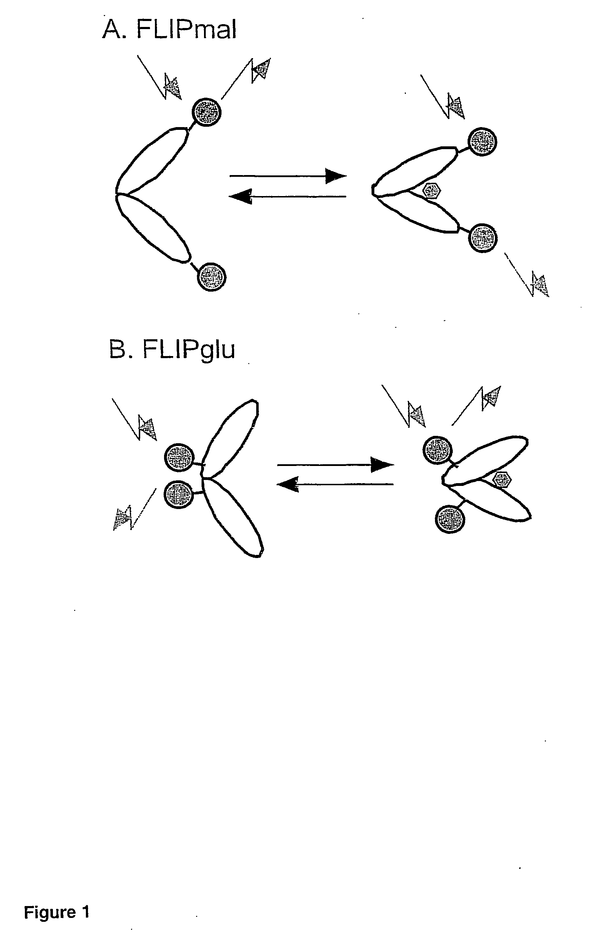

The term "detection portion" refers to the two protein portions of the fusion protein, which flank the PBP portion, wherein one detection portion is fused to the first lobe and the other to the second lobe of the PBP portion. The detection portions can be fused both to the front side (Figure 1A), both to the backside (Figure 1 B) or one detection portion to the front and the other to the backside of the different lobes. It is preferred that one detection portion is fused to the N-terminus and the other to the C-terminus of the PBP portion. If, however, the C- and N-termini of the PBP portions are located on the same lobe, the fusion protein of the invention may be realized in that, e.g., the amino acid sequence of one detection portion is inserted within that part of the amino acid sequence of the PBP portion which folds into one lobe and the amino acid sequence of the other detection portion is fused to the N- or C-terminus of the PBP portion lying in the other lobe. Methods for preparing a fusion of the protein portions comprised in the fusion protein are well known to those skilled in the art and are described in the literature. Preferably, such fusions are carried out by recombinant techniques, i.e. by combining nucleic acid molecules encoding the respective portions of the fusion protein, e.g., by classical cloning techniques, by in vitro techniques such as PCR or by chemical synthesis or by combinations of these techniques, followed by the expression of the fusion protein from the recombinant nucleic acid molecule. Other methods for producing protein fusions may be applied as well, such as the introduction of chemical linkages between polypeptides. A connection between a detection portion and the PBP portion, is indirect, i.e. via a polypeptide stretch lying between these portions such as a linker peptide. This linker may be used to bring the two detection portions into relative positions in order to allow or improve the change of energy emission such as the change of RET of the detection portions upon binding of a compound.

Thus, the fusion protein used in the invention further comprises linker peptides connecting the first and the second detection portion with the PBP portion.

Such linker peptides may in principle have any amino acid sequence which ensures that they do not interfere negatively with the analyte detection function of the fusion protein. Such peptides have a length between 3 and 35, more preferably between 5 and 15, most preferably of 6 amino acids. Also preferred is that the linker is mainly or only composed of hydrophilic amino acid residues, such as glycine, alanine, threonine and/or serine residues.

One main goal of inserting a linker peptide between a detection portion and the PBP portion may lie in an optimization of the fusion protein conformation. For instance, the insertion of a linker peptide may improve the energy emission signal, i.e. increase the difference of the energy emitted in the bound state of the fusion protein from that emitted in the unbound state or to increase energy emission in absolute terms. Such an optimization may be recommendable if the detection portions are partners of resonance energy transfer and the relative position between these partners is not optimal, preferably when the distance is too small or too big or the orientation is imperfect. Preferably, in the conformation with the highest possible resonance energy transfer for a given fusion protein, the distance between the detection portions should not be below R₀, which is the distance where resonance energy transfer for a given pair of resonance energy transfer partners is 50% efficient. Further functions of such linker peptides refer to the transmission of the conformational change motion of the PBP portion to the detection portion(s) and to facilitating proper folding of the fusion protein portions separated by the linker peptide. For testing different fusion protein constructs, e.g. varying with the linker peptide used for their energy emission behavior, titration experiments may be carried out as described for instance in Example 2.

Another preferred embodiment refers to the above-described fusion proteins, wherein the PBP portion, the first and/or the second detection portion is truncated compared to the corresponding wild-type PBP portion, first and/or second detection portion.

The term "truncated" means a deletion of one or a few amino acid residues at the N- and/or C-terminus or internal amino acid residues of one or both detection portion(s) and/or the PBP portion compared to the corresponding wild-type protein. In particular, the truncated region(s) comprise(s) at least 1, preferably at least 2, more preferably at least 5, still more preferably at least 10 and most preferably at least 20 amino acid residues. It is evident that the extent of truncation is limited to a maximum where the PBP portion still shows its functions necessary for the fusion protein to measure analyte concentration. Specifically, the truncated region(s) does not comprise more than 50 amino acid residues, preferably not more than 30, more preferably not more than 20 and still more preferably not more than 10 amino acid residues. The term "wild-type" refers to the protein that has been used as the starting point for constructing the fusion protein of the invention and includes naturally occurring proteins as well as variants or derivatives thereof as described herein for PBPs (supra) and for the detection portions (infra). Such truncations may have the same purpose as the insertion of linker peptides as mentioned above, i.e. to optimize the relative position of the detection portions to one another. Additionally, truncation of the PBP portion may have a positive effect in that it may render binding of the PBP portion to the compound reversible or at least increase its reversibility. This effect is based on the observation that the domain of a PBP that is involved in the interaction between the complex consisting of the PBP and the bound substrate and the specific receptor at the inner periplasma membrane may be located near the N- or C-terminus of the PBP (Duplay, J. Mol. Biol. 194 (1987), 663-673; Hall, J. Biol. Chem. 272 (1997), 17615-17622; Chen et al., PNAS 98 (2001), 1524-1530). There is some evidence that the release of the bound substrate is triggered via this interaction and that, in the absence of such an interaction, the substrate may virtually be irreversibly bound to the PBP (Ames, Ann. Rev. Biochem. 55 (1986), 397-425). Thus, it is contemplated that the inactivation of the domain which is involved in this interaction by truncation of the PBP portion, may result in an increased reversibility of binding to the compound for which the PBP portion is specific.

As a further aspect, it is particularly preferred that truncation of the PBP portion leads to a reduction of pH sensitivity. This applies especially to fusion proteins of the invention comprising, as the PBP portion, a GGBP. Preferably, this truncation comprises the deletion of at least one amino acid residue at the C-terminal end. As it is already explained above, the three-dimensional conformation of GGBP has surprisingly been found to depend on the pH value, in the unbound as well as in the bound state (Figure 13). As shown for fusion proteins comprising a truncated GGBP portion (Example 8 and Figure 16), these fusion proteins show an increased insensitivity to the pH value compared to the corresponding untruncated fusion protein, possibly due to optimized relative positions of the detection portions to one another. As it is shown in the experiments of Example 8, truncation of the PBP portion can lead to a decrease of a detectable change of energy emission between the bound and the unbound state. However, for the benefit of an improved pH insensitivity it may be reasonable under certain circumstances to accept a decreased energy emission change. With regard to GGBP-containing fusion proteins, this preferred embodiment in particular pertains to applications where glucose concentrations are to be measured under conditions where the pH cannot be controlled such as in vivo. The term "increased insensitivity to the pH value" means that the difference between maximum energy emission of the fusion proteins (in Fig. 16; for example at around pH 6.0 to 6.5) and the lowest energy emission observed within the physiological pH range, e.g. between pH 5.5 and pH 8.5, under otherwise substantially identical conditions is reduced by at least 20%, preferably at least 50% and more preferably at least 80%.

Furthermore, it is evident that truncating of the detection and/or PBP portion(s) is carried out so that the function of the fusion protein as regards, e.g., compound binding, conformational change and/or energy emission are substantially retained, preferably improved.

The detection portions present in the fusion protein used in the invention facilitate the detection of a conformational change, which, in turn, is indicative for binding of a compound by a change of the energy emitted by the detection portions.

The term "green fluorescent protein" or "GFP" as used throughout the present application refers to the GFP initially cloned by Prasher (Gene 111 (1992), 229-233) from *Aequorea victoria* and mutants thereof showing GFP activity. The term "GFP activity" refers to the known properties of a GFP, i.e. fluorescence emission upon excitation by a suitable light, the capacity of autocatalytic maturation involving folding into tertiary structure and the formation of the chromophore and the independence of any co-factors or metabolic energy supply for carrying out fluorescence as well as autocatalytic maturation. These properties are well known in the art and for example reviewed by Tsien (Ann. Rev. Biochem. 67 (1998), 509-544). For the purposes of the present invention, unless otherwise stated, any detectable emission wavelength of a GFP mutant can be useful for applying the fusion protein used in the invention. In the prior art, many GFP mutants are described, wherein specific amino acid residues are substituted with the effect of an improved fluorescence efficiency and/or a shifted excitation and/or emission wavelength (see, e.g., Heim, Methods Enzymol. 302 (1999), 408-423; Heikal et al., PNAS 97 (2000), 11996-12001). Particularly, mutating glutamine in position 69 to methionine can reduce the inherent pH and halide sensitivity of EYFP (Griesbeck et al., J. Biol. Chem. (2001) 276, 29188-29194). Thus, if EYFP, or a derivative thereof having substantially the same excitation and emission spectrum, is used as one detection portion of the fusion protein, it is preferred that the EYFP or derivative thereof shows this mutation. Examples for GFP mutants useful for applying the invention include enhanced yellow fluorescent protein (EYFP), enhanced cyan fluorescent protein (ECFP), enhanced blue fluorescent protein (EBFP) enhanced green fluorescent protein (EGFP), DsRED, Citrine and Sapphire. Within the scope of the present invention any GFP mutant or functional analog of GFP may be used as long as it shows GFP activity and are encoded by a nucleic acid molecule that hybridizes, preferably under stringent conditions, with the nucleotide sequence encoding the wild-type GFP such as the sequence depicted under SEQ ID NO: .5. Suitable preferred hybridization conditions and sequence identity values for preferred hybridizing nucleotide sequences' encoding a mutant GFP are mentioned above in connection with functional analogs of PBPs.

The term "fluorescent protein portion" refers to proteins that are capable of fluorescence, i.e. to absorb energy from radiation of a certain wave length, e.g. ultraviolet or visible light, and to emit this energy or a part thereof by radiation, wherein the emitted radiation has a higher wavelength than the eliciting radiation. There are many examples of fluorescent proteins described in the literature that may be useful in connection with the present invention such as GFPs as mentioned above.

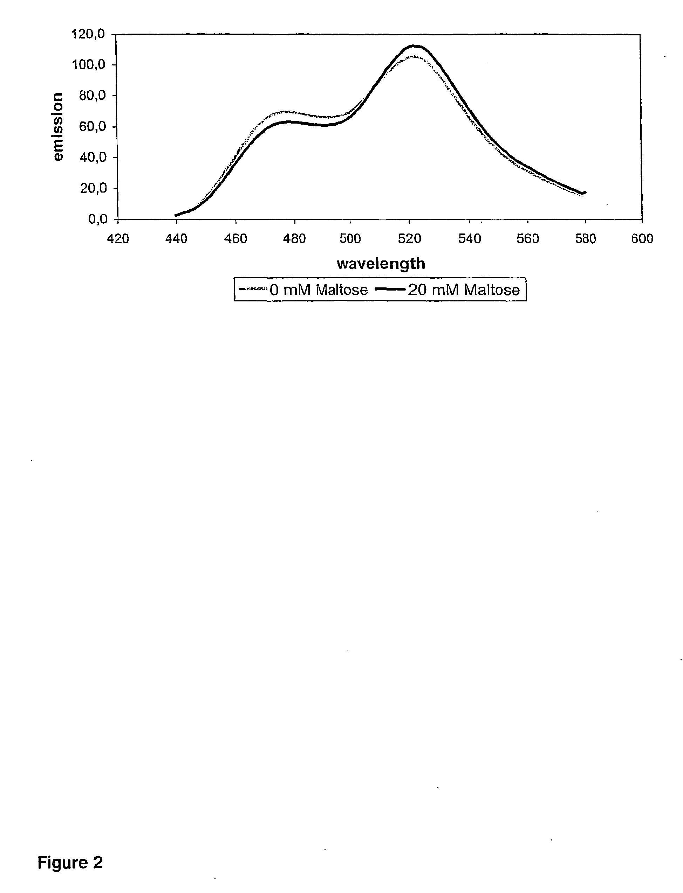

The term "resonance energy transfer" (RET) refers to a non-radiative transfer of excitation energy from a donor (first detection portion) to an acceptor molecule (second detection portion). The conformational change of the fusion protein results in a detectable change of RET between the detection portions. Such a change can for instance be taken from a comparison of the emission spectra of a fusion protein in the absence of a suitable binding compound with the same fusion protein in the presence of such a compound. If, for example, RET is increased, the emission peak of the acceptor is raised and the emission peak of the donor is diminished. An example of a corresponding change of the emission spectrum is shown in Figure 2. Thus, the ratio of the emission intensity of the acceptor to that of the donor is indicative for the degree of RET between the detection portions. As it is apparent from Figure 1, the conformational change of the fusion protein upon binding of a compound may result either in a decrease or an increase of the distance between the detection portions. However, not only the distance but also other aspects of the relative position of the detection portions to one another such as the orientation influences RET. Thus, depending on the topology of the detection portions in the fusion protein, RET may increase or decrease upon binding of a compound. The actual behaviour of the fusion protein in this regard can be predicted from the three-dimensional structure of the PBP. In addition, the RET behaviour can be determined empirically, e.g., by performing titration experiments as they are described in Example 2.

The change of the energy emitted by the detection portions is an increase or decrease of fluorescence resonance energy transfer (FRET).

In FRET, both donor and acceptor, i.e. both detection portions, are fluorescent protein portions and, for measuring FRET, the fusion protein is supplied with energy, i.e. radiation, appropriate for exciting energy emission by the first detection portion. Accordingly, in the fusion protein of use in the present invention, that the first detection portion is a fluorescent protein portion.

The efficiency of FRET is dependent on the distance between the two fluorescent partners. The mathematical formula describing FRET is the following: E = R₀⁶/(R₀⁶+r⁶), where E is the efficiency of FRET, r is the actual distance between the fluorescent partners, and R₀ is the Förster distance at which FRET is 50% of the maximal FRET value which is possible for a given pair of FRET partners. Ro, which can be determined experimentally, is dependent on the relative orientation between the fluorescent partners (κ), refractive index of the media (n), integral overlap of the emission of the donor with the excitation of the acceptor partner (J(λ)), and the quantum yield of the fluorescent donor partner (Q_{D}) (R₀⁶ = 8.79X10⁻²⁵[κ²n⁻⁴Q_{D}J(λ)] (in cm⁶)). In classical FRET based applications the orientation factor κ² is assumed to equal 2/3, which is the value for donors and acceptors that randomize by rotational diffusion prior to energy transfer (Lakovicz, Principles of Fluorescence spectroscopy, second edition, page 370). Thus, at randomized rotational diffusion, the change in ratio is assumed to be only due to a change in distance between the chromophores. For perpendicular dipoles κ² is 0.

In order to apply FRET for analyte detection, the person skilled in the art is capable of selecting suitable detection portions for the fusion protein that show a detectable FRET and a detectable change of FRET upon a conformational change in the PBP portion. Preferably, maximum FRET efficiency is at least 5%, more preferably at least 50% and most preferably 80% of the energy released by the first detection portion upon excitation. Additionally, the two detection portions need to have a spectral overlap. The greater the overlap of the emission spectrum of the donor with the absorption spectrum of the acceptor, the higher is the value of R₀. Acceptors with larger extinction coefficients lead to higher R₀ values. In contrast, the overlap in excitation spectra of both detection portions should be small enough to prevent coexcitation of the acceptor chromophore. Likewise, the spectra of both detection portions should only overlap to an extent that discrimination between the two emission signals is still possible.

In a particularly preferred embodiment, the first detection portion is enhanced cyan fluorescent protein (ECFP) and the second detection portion is enhanced yellow fluorescent protein (EYFP) or EYFP containing mutation glutamine 69 to methionine (Citrine) (Griesbeck et al., J. Biol. Chem. (2001) 276, 29188-29194).

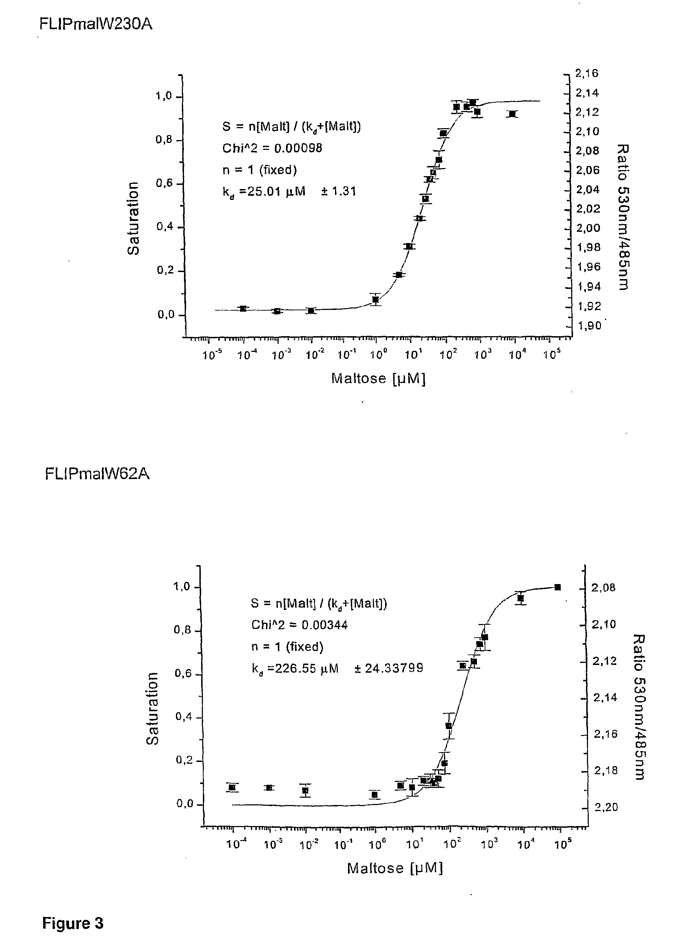

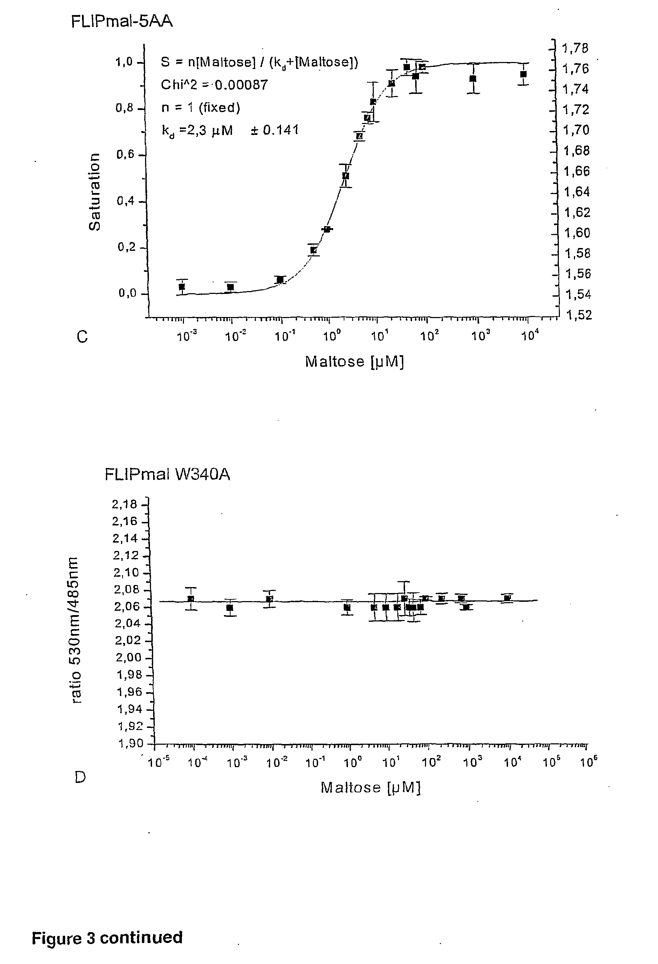

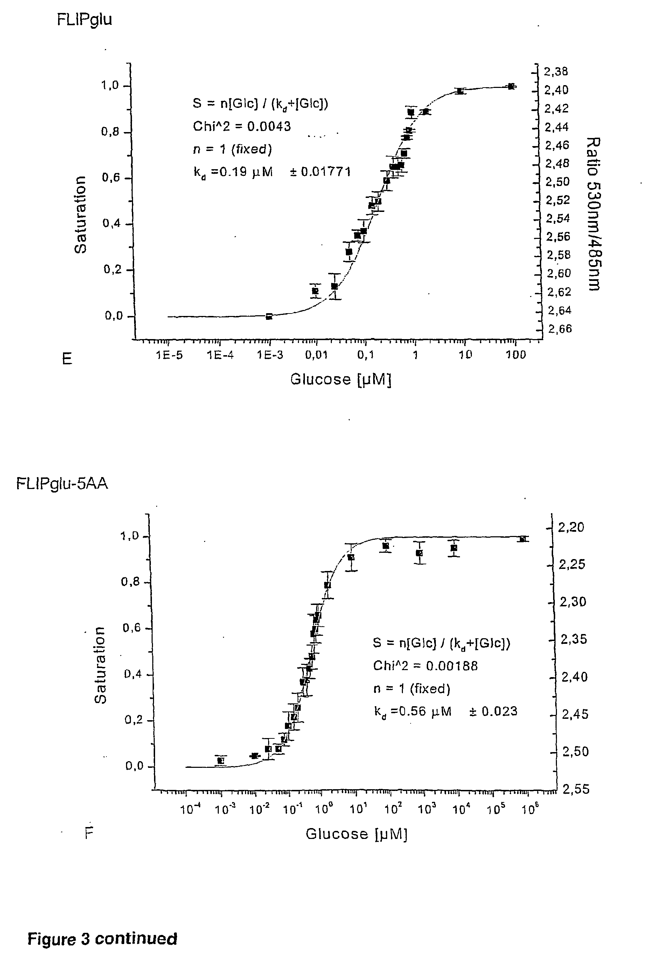

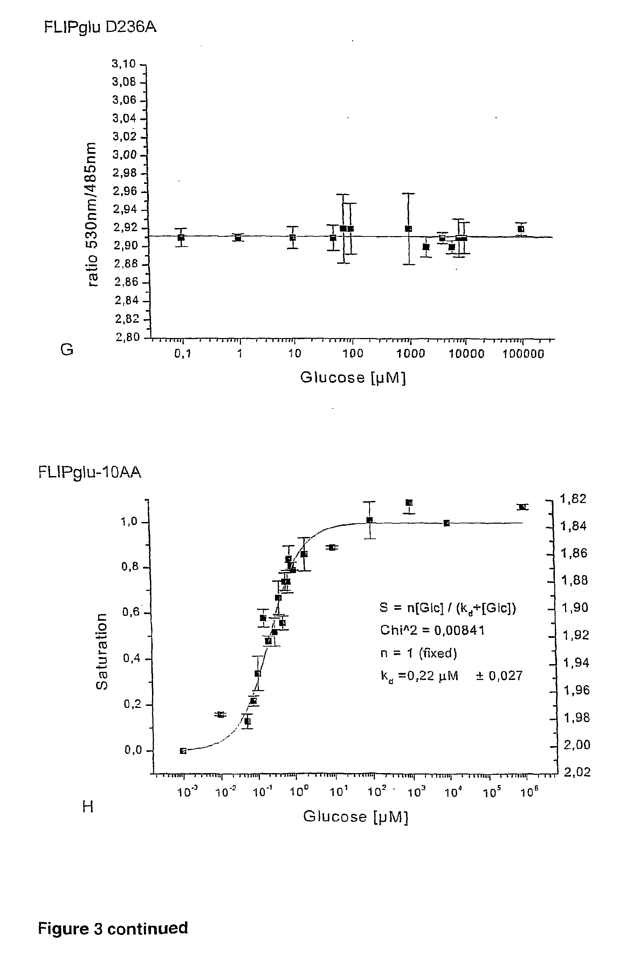

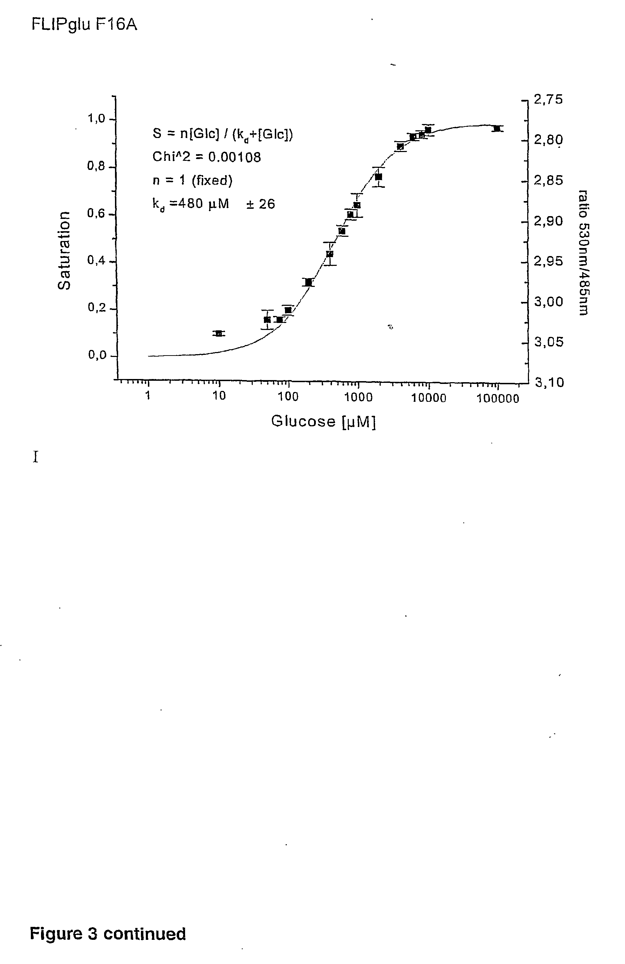

It has been shown that ECFP and EYFP are particularly well suited for the fusion protein of use in the present invention since they show an efficient change in FRET upon binding of an analyte. This is a surprising finding since the calculated R₀ for this system is approx. 5 nm (Miyawaki & Tsien, Meth. Enzymol. 327 (2000), 472) and, due to the relatively small size of PBPs (e.g. 3 x 4 x 6.5 nm; Spurlino, J. Biol. Chem. 266 (1991), 5202), it was likely that the distance between the fluorescent partners is considerably below 5 nm which would lead to very low or even non-detectable changes of FRET upon binding of an analyte. However, as it can be seen in the Examples and in Figure 3, the fusion proteins containing ECFP and EYFP produced significant and reproducible changes of FRET as exemplified by the ratio between the emission intensities of the acceptor and the donor fluorescent protein portion in dependence on analyte concentration. ECFP and EYFP are well known in the art and nucleic acid molecules containing corresponding coding sequences are commercially available e.g. from InVitrogen (The Netherlands).

Generally, the fusion protein of the invention may be produced according to techniques; which are described in the prior art. For example, these techniques involve recombinant techniques which can be carried out as described in Sambrook and Russell (2001), Molecular Cloning: A Laboratory Manual, CSH Press or in Volumes 1 and 2 of Ausubel (1994), Current Protocols in Molecular Biology, Current Protocols. Accordingly, the individual portions of the fusion protein may be provided in the form of nucleic acid molecules encoding them which are combined and, subsequently, expressed in a host organism or in vitro. Alternatively, the provision of the fusion protein or parts thereof may involve chemical synthesis or the isolation of such portions from naturally occurring sources, whereby the elements which may in part be produced by recombinant techniques may be fused on the protein level according to suitable methods, e.g. by chemical cross-linking for instance as disclosed in WO 94/04686. Furthermore, if deemed appropriate, the fusion protein may be modified post-translationally in order to improve its properties for the respective goal, e.g., to enhance solubility, to increase pH insensitivity, to be better tolerated in a host organism, to make it adherent to a certain substrate in vivo or in vitro, the latter potentially being useful for immobilizing the fusion protein to a solid phase etc. The person skilled in the art is well aware of such modifications and their usefulness. Illustrating examples include the modification of single amino acid side chains (e.g. by glycosylation, phosphorylation, carbethoxylation or amidation), coupling with polymers such as polyethylene glycol, carbohydrates, etc. or with protein moieties such as antibodies or arts thereof, enzymes etc. The fusion protein useful in the invention may comprise further elements in addition to the PBP portion, the detection portions and linker peptides as they are described above. Such further elements may include moieties that may be useful for isolating and purifying the fusion protein as for example the His-tag e.g. contained in the pQE vector, Qiagen, Hilden, Germany), the FLAG-tag (Knappik, Biotechniques 17 (1994), 754-761), the HA-tag (Wilson, Cell 37 (1984), 767) or glutathione-S-transferase (GST). Furthermore contemplated are moieties that facilitate binding of the fusion protein to a substrate such as via biotin/streptavidin, antibody/antigen or other binding interactions known in the art or that improve stability of the expressed polypeptide in the host cell such as by the presence of GST.

In another preferred embodiment of the invention, the fusion protein further comprises a targeting signal sequence.

The term "targeting signal sequence" refers to amino acid sequences, the presence of which in an expressed protein targets it to a specific subcellular localization. For example, corresponding targeting signals may lead to the secretion of the expressed fusion protein, e.g. from a bacterial host in order-to simplify its purification. Preferably, targeting of the fusion protein may be used to measure an analyte in a specific subcellular or extracellular compartment. Appropriate targeting signal sequences useful for different groups of organisms are known to the person skilled in the art and may be retrieved from the literature or sequence data bases.

If targeting to the plastids of plant cells is desired, the following targeting signal peptides can for instance be used: amino acid residues 1 to 124 of *Arabidopsis thaliana* plastidial RNA polymerase (AtRpoT 3) (Plant Journal (1999) 17, 557-561); the targeting signal peptide of the plastidic Ferredoxin:NADP+ oxidoreductase (FNR) of spinach (Jansen et al., Current Genetics 13 (1988), 517-522) in particular, the amino acid sequence encoded by the nucleotides -171 to 165 of the cDNA sequence disclosed therein; the transit peptide of the waxy protein of maize including or without the first 34 amino acid residues of the mature waxy protein (Klösgen et al., Mol. Gen. Genet. 217 (1989), 155-161); the signal peptides of the ribulose bisphosphate carboxylase small subunit (Wolter et al., Proc. Natl. Acad. Sci. USA 85 (1988), 846-850; Nawrath et al., Proc. Natl. Acad. Sci. USA 91 (1994), 12760-12764), of the NADP malat dehydrogenase (Gallardo et al., Planta 197 (1995), 324-332), of the glutathione reductase (Creissen et al., Plant J. 8 (1995), 167-175) or of the R1 protein (Lorberth et al., Nature Biotechnology 16 (1998), 473-477).

Targeting to the mitochondria of plant cells may be accomplished by using the following targeting signal peptides: amino acid residues 1 to 131 of Arabidopsis thaliana mitochondrial RNA polymerase (AtRpoT 1) (Plant Journal (1999) 17, 557-561) or the transit peptide described by Braun (EMBO J. 11 (1992), 3219-3227).

Targeting to the vacuole in plant cells may be achieved by using the following targeting signal peptides: The N-terminal sequence (146 amino acids) of the patatin protein (Sonnewald et al., Plant J. 1 (1991), 95-106) or the signal sequences described by Matsuoka and Neuhaus (Journal of Experimental Botany 50 (1999), 165-174); Chrispeels and Raikhel (Cell 68 (1992), 613-616); Matsuoka and Nakamura (Proc. Natl. Acad. Sci. USA 88 (1991), 834-838); Bednarek and Raikhel (Plant Cell 3 (1991), 1195-1206) or Nakamura and Matsuoka (Plant Phys. 101 (1993), 1-5).

Targeting to the ER in plant cells may be achieved by using, e.g., the ER targeting peptide HKTMLPLPLIPSLLLSLSSAEF (SEQ ID NO: 6) in conjunction with the C-terminal extension HDEL (SEQ ID NO: 7). (Haselhoff, Proc. Natl. Acad. Sci. USA (1997) 94, 2122-2127)

Targeting to the nucleus of plant cells may be achieved by using, e.g., the nuclear localization signal (NLS) of the tobacco C2 polypeptide QPSLKRMKIQPSSQP (SEQ ID NO: 8).

Targeting to the extracellular space may be achieved by using e.g. one of the following transit peptides: the signal sequence of the proteinase inhibitor II-gene (Keil et al., Nucleic Acid Res. 14 (1986), 5641-5650; von Schaewen et al., EMBO J. 9 (1990), 30-33), of the levansucrase gene from *Erwinia amylovora* (Geier and Geider, Phys. Mol. Plant Pathol. 42 (1993), 387-404), of a fragment of the patatin gene B33 from Solanum tuberosum, which encodes the first 33 amino acids (Rosahl et al., Mol Gen. Genet. 203 (1986), 214-220) or of the one described by Oshima et al. (Nucleic Acids Res. 18 (1990),181).

Furthermore, targeting to the membrane may be achieved by using the N-terminal signal anchor of the rabbit sucrase-isomaltase (Hegner et al., J. Biol. Chem. (1992) 276, 16928-16933).

Targeting to the membrane in mammalian cells can be accomplished by using the N-terminal myristate attachment sequence MGSSKSK (SEQ ID No:9) or C-terminal prenylation sequence CaaX (SEQ ID No:10), where a is an aliphatic amino acid (i.e. Val, Leu or Ile) and X is any amino acid (Garabet, Methods Enzymol. (2001) 332, 77-87).

Targeting to the plasma membrane of plant cells may be achieved by fusion to a transporter, preferentially to the sucrose transporter SUT1 (Riesmeier, EMBO J. (1992) 11, 4705-4713). Targeting to different intracellular membranes may be achieved by fusion to membrane proteins present in the specific compartments such as vacuolar water channels (γTIP) (Karlsson, plant J. (2000) 21, 83-90), MCF proteins in mitochondria (Kuan, Crit. Rev. Biochem. Mol. Biol. (1993) 28, 209-233), triosephosphate translocator in inner envelopes of plastids (Flügge, EMBO J. (1989) 8; 39-46) and photosystems in thylacoids.

Targeting to the golgi apparatus can be accomplished using the C-terminal recognition sequence K(X)KXX (SEQ ID No:11) where X is any amino acid (Garabet, Methods Enzymol. (2001) 332, 77-87).

Targeting to the peroxisomes can be done using the peroxisomal targeting sequence PTS I or PTS II (Garabet, Methods Enzymol. (2001) 332, 77-87).

Targeting to the nucleus in mammalian cells can be achieved using the SV-40 large T-antigen nuclear localisation sequence PKKKRKV (SEQ ID No:12) (Garabet, Methods Enzymol. (2001) 332, 77-87).

Targeting to the mitochondria in mammalian cells can be accomplished using the N-terminal targeting sequence MSVLTPLLLRGLTGSARRLPVPRAKISL (SEQ ID No:13) (Garabet, Methods Enzymol. (2001) 332, 77-87). In yet another preferred embodiment of the fusion protein used in the invention, said PBP portion is modified with respect to the corresponding wild-type PBP so that the binding affinity to the compound of said PBP portion is altered, preferably decreased compared to the wild-type PBP.

For some applications, it may be advantageous or necessary that the binding affinity of the PBP portion to the compound is reduced. Many PBPs notoriously show a high binding affinity to the compounds they are specific for so that binding of the compounds may even be virtually irreversible under physiological conditions. This is reflected by a low dissociation constant K_{d} in the range from 10⁻⁹ to 10⁻⁶ M. In its natural environment, the compound is released by the interaction of the PBP-compound complex with its specific membrane receptor. However, for many applications of the fusion protein used in the invention, reversibility of binding is desirable, for instance when changes of analyte concentration in a given compartment shall be observed, e.g. in *in vivo* assays. Likewise, it may be necessary to adjust the binding affinity of the PBP portion to the range of analyte concentration to be measured. Thus, according to the preferred embodiment, the binding affinity of the PBP portion present in the fusion protein to its specific compound, as expressed in terms of K_{d}, is significantly decreased compared to the binding affinity of the corresponding wild-type PBP. Preferably, said decrease is by a factor of least 10, more preferably by at least 10², still more preferably by at least 10³ and most preferably by at least 10⁴ when comparing the K_{d} of the PBP used as starting point with that of the PBP having a reduced binding affinity. The dissociation constant of a PBP portion present in a fusion protein may conveniently be determined in titration experiments as described in Example 2. Other methods for determining the K_{d} of a PBP are described in Duplay et al., J. Mol. Biol. (1987) 194, 663-673; Hall et al., J. Biol. Chem (1997) 272, 17615-17622. It has been shown in the experiments performed in connection with the present invention that the dissociation constants of a PBP alone and the same PBP contained in a fusion protein are more or less equal and can therefore be directly compared.

A decrease of binding affinity may be achieved by introducing amino acid substitutions in the PBP portion, in particular by exchanging amino acid residues which are involved in binding to the compound by other amino acid residues of which it is expected that they are not involved in binding, such as Ala or Gly residues. The effect of such substitutions on binding affinity of the maltose binding protein (MBP) has been reported by Martineau (J. Mol. Biol. 214 (1990), 337-352). As it is discussed therein, stacking interactions between the aromatic residues of the binding sites of the PBP and its bound compound such as maltose may contribute to the binding interaction (Nand et al. Science (1998) 242, 1290-1295; Spurlino et al. Journal of Biological Chemistry (1991) 226, 5202-5219; Spurlino et al. J. Mol. Biol. (1992) 226, 15-22). Thus, the elimination of the aromatic side chains of tryptophane residues may lead to a reduction of stacking interactions. It has been found in connection with the present invention that another PBP, the glucose/galactose binding protein (GGBP) may correspondingly be specifically modified in order to achieve a decrease of binding affinity (see Example 2, infra). In order to modify other PBPs accordingly, the person skilled in the art may select amino acid residues which are involved in binding, preferably aromatic amino acid residues, in particular, tryptophane or phenylalanine residues, lying in the substrate binding site of the PBP or residues in the vicinity of the binding site which, if modified, interfere with binding. For determining the binding site, one may rely on amino acid sequence alignments with the known PBP sequences such as the MPB or GGBP sequence and determine the respective amino acid positions as being conserved with the amino acid residues of MBP or GGBP. Alternatively, for instance in case the amino acid sequence of a given PBP is too distant for a significant alignment with the MBP or GGBP sequence, one may determine the relevant amino acid residues from a three-dimensional protein structure model based on crystallographic data. Suitable techniques for introducing amino acid substitutions such as PCR-based in vitro mutagenesis are described in the literature and may be applied herein accordingly.

Yet another embodiment refers to fusion proteins, wherein said PBP has approximately the same binding affinity to the compound as the corresponding wild-type PBP.

This means that the K_{d} of the PBP portion to its substrate is approximately identical with that of the corresponding wild-type PBP, preferably the PBP portion is not modified, i.e. does not contain any amino acid substitution and/or is not truncated as compared to the corresponding wild-type PBP. "Approximately" means in this context that the K_{d} values may deviate by the statistical error, preferably by less than 50%, more preferably by less than 20% and most preferably by less than 10% from the K_{d} of the corresponding wild-type PBP, The present embodiment thus refers to a fusion protein, wherein the binding affinity of the PBP portion is relatively high, including the possibility of having virtually irreversible binding.

Such fusion proteins may be of use for in vitro analyte detection. For example, for flow-through high throughput *in vitro* analyte detection, different fusion proteins with K_{d} values ranging from the nanomolar to the high millimolar range and specificities to different analytes can be immobilized on a solid support. To perform online quantification of different analytes over a broad concentration range, the solution that is analyzed flows over the solid support. Fluorescence is read out for each area of the solid support using a one-wavelength excitation/dual wavelength emission analyzer. This system can for instance serve as an online quality control for the production of food, e.g. juice.

In another possible application, the fusion proteins may be immobilized on test sticks or in microtiter plates. To perform easy to handle analyte quantification, these sticks can then be dipped into a solution to be analyzed and fluorescence can be subsequently read out using a mobile one-wavelength excitation/dual wavelength emission analyzer. For example, this mobile system can be used to perform ex vivo metabolite quantification of blood or urine or for quantification of soil contaminants and other environmentally interesting compounds.

Furthermore, these fusion proteins can be added to the medium of fermentation processes to monitor the process of fermentation by analyte quantification.

In another possible application, the fusion proteins can be added to cell culture media thereby allowing monitoring substrate consumption and product formation. Detection can be performed by a simple device consisting of a CCD camera equipped with two filters or by a more sophisticated fluorescence ratio detection device.

In a further preferred embodiment, the fusion protein used in the invention is characterized by a PBP portion which is a glucose/galactose binding protein (GGBP) in which the phenylalanine residue at a position corresponding to position 16 of a wild type GGBP polypeptide as represented by SEQ ID NO:4 is replaced by an Ala residue.

It has surprisingly been found that the fusion protein FLIPglu F16A shows an improved selectivity for glucose as compared to other fusion proteins comprising GGBP as PBP portion. Consequently, it is contemplated that fusion proteins with a GGBP portion bearing a mutation corresponding to this F16A substitution may be especially suited for analyte detection in which a discrimination between glucose and other sugars is important.

In connection with this preferred embodiment, the PBP portion comprises the GGBP portion with the amino acid sequence of SEQ ID NO:4 having the phenylalanine residue at position 16 replaced by an Ala residue and to derivatives of this GGBP portion showing a conformational change suitable for analyte detection and an increased selectivity for glucose compared to the corresponding wild type GGBP. The term "increased selectivity" means that the conformational change of the PBP portion, as for instance detectable by a decrease of FRET between the two detection portions of the fusion protein, is significantly reduced for at least one compound other than glucose. Such other compounds may for example be sugars, i.e. mono-, di- or oligosaccharides, sugar alcohols, other carbohydrates like for instance starch or conjugates between a carbohydrate and an aglycone moiety. It is preferred that this reduction of the conformational change, e.g. by comparing the change of energy emitted by the two detection portions at a concentration of 0.1 mM and 10 mM of the compound in question (see Example 6), is by at least 20%, preferably at least 50% and more preferably at least 80%. Most preferably, the PBP portion of a fusion protein of the present preferred embodiment does not show any significant conformational change for at least one compound for which the corresponding wild type GGBP shows a significant conformational change. For FLIPglu comprising wild type GGBP and FLIPglu F16A, the difference in selectivity for various compounds is shown in Figures 11 and 12. It is however contemplated that the PBP portion may still retain sensitivity for specific compounds other than glucose, as for instance ribose or galactose. A person skilled in the art knows how to determine whether a given PBP portion (or fusion protein) has an increased selectivity for glucose, e.g. by applying the method described in Example 6.

The term "derivative of the GGBP portion comprising the amino acid sequence of SEQ ID NO:4 with the phenylalanine residue at position 16 being replaced by an Ala residue" refers to any possible variants of GGBPs according to what is described hereinabove for fusion proteins useful in the invention as long as the variants show an increased selectivity for glucose. Specifically, such derivatives may be truncated versions or homologous sequences, for instance originating from other organisms than E. coli. The derivatives may be characterized as being encoded by a nucleotide sequence hybridizing to SEQ ID NO:3 under low stringent or, as it is preferred, under high stringent hybridization conditions (see for reference, e.g., Sambrook and Russell (2001), Molecular Cloning, CSH Press, Cold Spring Harbor, NY, USA). The derivatives may display at least one further mutation (e.g. substitution, deletion, insertion, transversion, etc.) as compared to the corresponding wild type sequence such as SEQ ID NO:4 comprising the F16A substitution. Such further mutations may be introduced by human intervention applying appropriate techniques known in the art, for instance with the aim to further improve the fusion protein's properties.

In addition, it is evident that a person skilled in the art is capable of determining in a given PBP portion sequence the phenylalanine residue that corresponds to the phenylalanine residue at position 16 of SEQ ID NO:4. This can for example be done by sequence comparisons wherein conserved amino acid positions are aligned.

As it is evident to a person skilled in the art, the GGBP-based fusion protein may be used as a basis for randomly mutagenizing and selecting for other mutants than the one bearing a mutation corresponding to the F16A substitution described above that show an increased selectivity for glucose. For this purpose, selection can readily be conducted by using FRET as it is for instance described in Example 6.

Furthermore, in another embodiment, the invention relates to a method using fusion proteins wherein the PBP portion is a GGBP in which at least one histidine residue is modified so that the side chain of said residue cannot be protonated. Preferably, this modification leads to an increased insensitivity of the PBP portion to the pH value.

This preferred embodiment in particular pertains to applications where glucose concentrations are to be measured under conditions where the pH cannot be controlled such as in vivo.

Dependence of the three-dimensional conformation of GGBP on the pH value has surprisingly been found for the unbound as well as for the bound state (Figure 13). This also holds true for the control sensor FLIPglu D236A which has been deprived of its analyte-binding activity (Figure 14). In view of this, fusion proteins comprising a PBP portion, wherein one or more histidine residues are modified according to the present embodiment may constitute a particular improvement with respect to their applicability. Modifications of histidine residues resulting in the inability of the side chain to be protonated under appropriate pH conditions may be carried out according to methods known in the art.

This present preferred embodiment is based on the finding that FLIPglu that was treated with diethyl pyrocarbonate (DEPC) shows a reduced pH sensitivity as compared to untreated FLIPglu (Example 7 and Figure 15). Since it is known that DEPC modifies histidine residues so that their side chain can no longer be protonated, the observed reduction of pH sensitivity was interpreted to rely on this effect. It is therefore conceivable that fusion proteins containing, as the PBP portion, a GGBP having an increased pH insensitivity can be prepared by modifying the histidine residues in the PBP portion so that their side chains cannot be protonated. For instance, such a modification may be performed chemically, e.g. by treatment with diethyl pyrocarbonate (DEPC; carbethoxylation) after expression and purification of the fusion protein (see Example 7 and Figure 15 for optimal DEPC treatment conditions, i,e. reducing pH sensitivity to the maximum, while retaining a significant difference of energy emission between the bound and unbound state of the fusion protein). Thus, in a preferred embodiment, the present invention also relates to a method comprising use of fusion proteins containing a GGBP portion as the PBP portion which are treated in that way by DEPC. Furthermore, a person skilled in the art is capable of carrying out such chemical modifications by means equivalent to DEPC treatment.

Likewise, modifying histidine residues so that the side chain cannot be protonated may likewise include substituting the histidine residues recombinantly by other amino acid residues for instance by site-directed mutagenesis as, e.g., described in Sambrook and Russell (2001; loc. cit.). When carrying out this embodiment, one has however to take into account that the mutation of single histidine residues by substitution was shown not to lead to a significant reduction of pH sensitivity. Thus, it may be likely that the substitution of at least two, preferably of all three histidine residues will lead to a significantly reduced pH sensitivity.

The term "increased insensitivity to pH value" means that the difference between maximum energy emission of the fusion proteins (in Figure 13 at around pH 6.0 to 6.5) and the lowest energy emission observed within the physiological pH range, e.g. between pH 5.5 and pH 8.5, under otherwise substantially identical conditions is reduced by at least 20%, preferably at least 50% and more preferably at least 80%.For certain applications, an increased selectivity for glucose may be required together with a reduced sensitivity to pH. Thus, in a particularly preferred embodiment of the present invention, the fusion protein comprises a PBP portion in which the phenylalanine residue at a position corresponding to position 16 of a wild type GGBP polypeptide as represented by SEQ ID NO:4 is replaced by an alanine residue, at least one histidine residue is modified as it is explained hereinabove and/or a truncation leads to a reduction of pH sensitivity (supra).

Another embodiment of the present disclosure relates to nucleic acid molecules comprising a nucleotide sequence encoding the fusion protein of the present invention.

The term "nucleic acid molecule" means DNA or RNA or both in combination or any modification thereof that is known in the state of the art (see, e.g., US 5525711, US 4711955, US 5792608 or EP 302175 for examples of modifications). Such nucleic acid molecule(s) are single- or double-stranded, linear or circular and without any size limitation. The nucleic acid molecules can be obtained for instance from natural sources or may be produced synthetically or by recombinant techniques, such as PCR. In a preferred embodiment, the nucleic acid molecules of the invention are DNA molecules, in particular genomic DNA or cDNA, or RNA molecules. Preferably, the nucleic acid molecule is double-stranded DNA.

In view of the fact that the invention refers to fusion proteins, the nucleic acid molecule comprising a nucleotide sequence encoding it preferably is a recombinant nucleic acid molecule, i.e. a nucleic acid molecule that has been produced by a technique useful for artificially combining nucleic acid molecules or parts thereof that were beforehand not connected as in the resulting recombinant nucleic acid molecule. Suitable techniques are for example available from the prior art, as represented by Sambrook and Russell (2001), Molecular Cloning: A Laboratory Manual, CSH Press and Ausubel, Current Protocols in Molecular Biology, Green Publishing Associates and Wiley Interscience, N.Y. (1989).

Furthermore, the present disclosure relates to expression cassettes comprising the above-described nucleic acid molecule of the invention and operably linked thereto control sequences allowing expression in prokaryotic or eukaryotic cells.

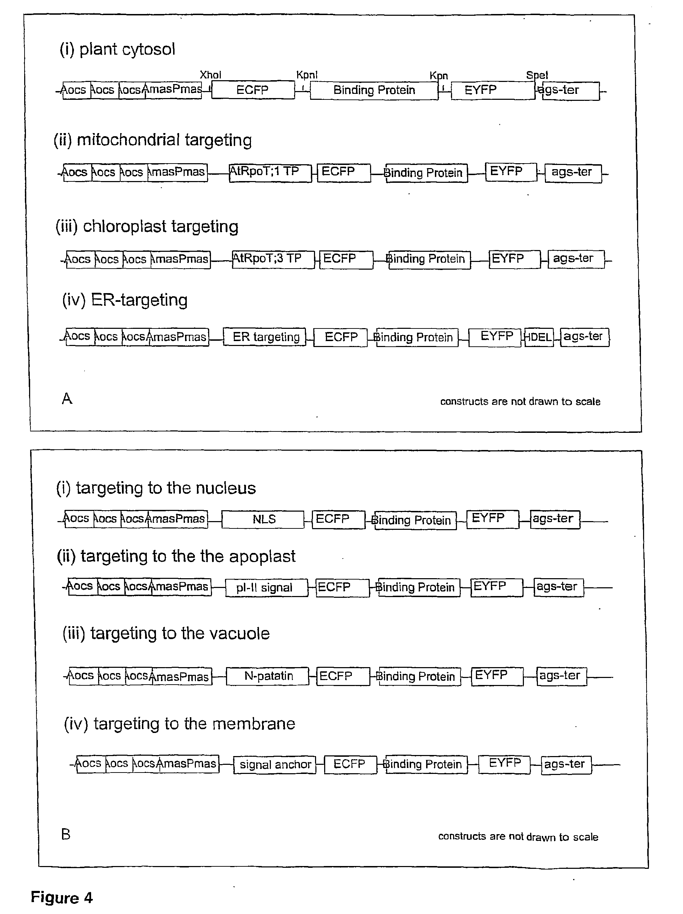

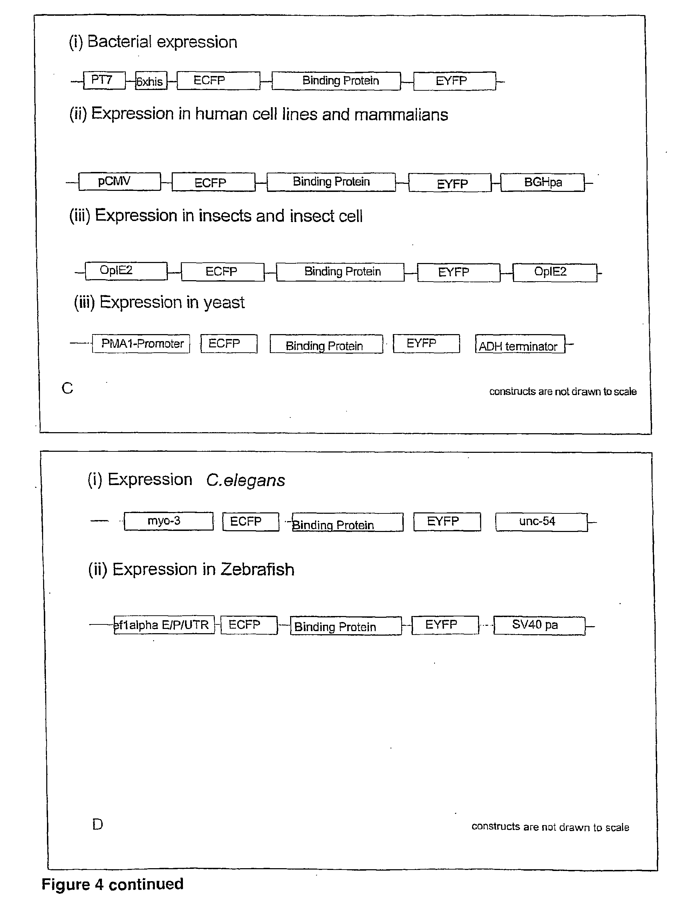

Suitable expression control sequences include promoters that are applicable in the target host organism. Such promoters are well known to the person skilled in the art for diverse hosts from prokaryotic and eukaryotic organisms and are described in the literature. For example, such promoters can be isolated from naturally occurring genes or can be synthetic or chimeric promoters. Likewise, the promoter can already be present in the target genome and will be linked to the nucleic acid molecule by a suitable technique known in the art, such as for example homologous recombination. Specific examples of expression control sequences and sources from where they can be derived are given further below and in Figure 4.

Expression cassettes of use in the invention are particularly meant for an easy to use insertion into target nucleic acid molecules such as vectors or genomic DNA. For this purpose, the expression cassette is preferably provided with nucleotide sequences at its 5'- and 3'-flanks facilitating its removal from and insertion into specific sequence positions like, for instance, restriction enzyme recognition sites or target sequences for homologous recombination as, e.g. catalyzed by recombinases.

The present disclosure also relates to vectors, particularly plasmids, cosmids, viruses and bacteriophages used conventionally in genetic engineering, that comprise a nucleic acid molecule or an expression cassette.

In a preferred embodiment, the vectors useful in the method of the invention are suitable for the transformation of fungal cells, plant cells, cells of microorganisms (i.e. bacteria, protists, yeasts, algae etc.) or animal cells, in particular mammalian cells. Preferably, such vectors are suitable for the transformation of human cells. Methods which are well known to those skilled in the art can be used to construct recombinant vectors; see, for example, the techniques described in Sambrook and Russell (2001), Molecular Cloning: A Laboratory Manual, CSH Press and Ausubel, Current Protocols in Molecular Biology, Green Publishing Associates and Wiley Interscience, N.Y. (1989). Alternatively, the vectors may be liposomes into which the nucleic acid molecules or expression cassettes can be reconstituted for delivery to target cells. Likewise, the term "vector" refers to complexes containing such nucleic acid molecules or expression cassettes which furthermore comprise compounds that are known to facilitate gene transfer into cells such as polycations, cationic peptides and the like.

In addition to the nucleic acid molecule or expression cassette used in performing the method of the invention, the vector may contain further genes such as marker genes which allow for the selection of said vector in a suitable host cell and under suitable conditions. Generally, the vector also contains one or more origins of replication,

Advantageously, the nucleic acid molecules contained in the vectors are operably linked to expression control sequences allowing expression, i.e. ensuring transcription and synthesis of a translatable RNA, in prokaryotic or eukaryotic cells.

In one aspect, the expression of the nucleic acid molecules in prokaryotic or eukaryotic cells is interesting because it permits a more precise characterization of the function of the fusion protein encoded by these molecules. In addition, it is possible to insert different additional mutations into the nucleic acid molecules by methods usual in molecular biology (see for instance Sambrook and Russell (2001), Molecular Cloning: A Laboratory Manual, CSH Press), leading to the synthesis of proteins possibly having modified properties, e.g. as concerns binding affinity or energy emission (e.g. RET) efficiency. In this regard, it is possible to mutate the nucleic acid molecules present in the vector by inserting or deleting coding sequences or to introduce amino acid substitutions by replacing the corresponding codon tripletts.

For genetic engineering, e.g. in prokaryotic cells, the nucleic acid molecules or parts of these molecules can be introduced into plasmids which permit mutagenesis or sequence modification by recombination of DNA sequences. Standard methods (see Sambrook and Russell (2001), Molecular Cloning: A Laboratory Manual, CSH Press) allow base exchanges to be performed or natural or synthetic sequences to be added. DNA fragments can be connected to each other by applying adapters and linkers to the fragments. Moreover, engineering measures which provide suitable restriction sites or remove surplus DNA or restriction sites can be used. In those cases, in which insertions, deletions or substitutions are possible, in vitro mutagenesis, "primer repair", restriction or ligation can be used. In general, sequence analysis, restriction analysis and other methods of biochemistry and molecular biology are carried out as analysis methods.

In a further embodiment, the disclosure relates to a method for producing cells capable of expressing the fusion protein used in the invention comprising genetically engineering cells with an above-described nucleic acid molecule, expression cassette or vector.

Another embodiment of the disclosure relates to host cells, in particular prokaryotic or eukaryotic cells, genetically engineered with an above-described nucleic acid molecule, expression cassette or vector, and to cells descended from such transformed cells and containing a nucleic acid molecule, expression cassette or vector and to cells obtainable by the above-mentioned method for producing the same.

Preferably, these host cells are bacterial, fungal, insect, plant or animal host cells. In a preferred embodiment, the host cell is genetically engineered in such a way that it contains the introduced nucleic acid molecule stably integrated into the genome. More preferably the nucleic acid molecule can be expressed so as to lead to the production of the fusion protein of the invention.

An overview of different expression systems is for instance contained in Methods in Enzymology 153 (1987), 385-516, in Bitter et al. (Methods in Enzymology 153 (1987), 516-544) and in Sawers et al. (Applied Microbiology and Biotechnology 46 (1996), 1-9), Billman-Jacobe (Current Opinion in Biotechnology 7 (1996), 500-4), Hockney (Trends in Biotechnology 12 (1994), 456-463), Griffiths et al., (Methods in Molecular Biology 75 (1997), 427-440). An overview of yeast expression systems is for instance given by Hensing et al. (Antoine von Leuwenhoek 67 (1995), 261-279), Bussineau (Developments in Biological Standardization 83 (1994), 13-19), Gellissen et al. (Antoine van Leuwenhoek 62 (1992), 79-93, Fleer (Current Opinion in Biotechnology 3 (1992), 486-496), Vedvick (Current Opinion in Biotechnology 2 (1991), 742-745) and Buckholz (Bio/Technology 9 (1991), 1067-1072).

Expression vectors have been widely described in the literature. As a rule, they contain not only a selection marker gene and a replication origin ensuring replication in the host selected, but also a bacterial or viral promoter and, in most cases, a termination signal for transcription. Between the promoter and the termination signal, there is in general at least one restriction site or a polylinker which enables the insertion of a coding nucleotide sequence. It is possible to use promoters ensuring constitutive expression of the gene and inducible promoters which permit a deliberate control of the expression of the gene. Bacterial and viral promoter sequences possessing these properties are described in detail in the literature. Regulatory sequences for the expression in microorganisms (for instance *E*. *coli, S*. *cerevisiae)* are sufficiently described in the literature. Promoters permitting a particularly high expression of a downstream sequence are for instance the T7 promoter (Studier et al., Methods in Enzymology 185 (1990), 60-89), lacUV5, trp, trp-lacUV5 (DeBoer et al., in Rodriguez and Chamberlin (Eds), Promoters, Structure and Function; Praeger, New York, (1982), 462-481; DeBoer et al., Proc. Natl. Acad. Sci. USA (1983), 21-25), Ip1, rac (Boros et al., Gene 42 (1986), 97-100). Inducible promoters are preferably used for the synthesis of proteins. These promoters often lead to higher protein yields than do constitutive promoters. In order to obtain an optimum amount of protein, a two-stage process is often used. First, the host cells are cultured under optimum conditions up to a relatively high cell density. In the second step, transcription is induced depending on the type of promoter used. In this regard, a tac promoter is particularly suitable which can be induced by lactose or IPTG (isopropyl-ß-D-thiogalactopyranoside) (deBoer et al., Proc. Natl. Acad. Sci. USA 80 (1983), 21-25). Termination signals for transcription such as the SV40-poly-A site or the tk-poly-A site useful for applications in mammalian cells are also described in the literature. Suitable expression vectors are known in the art such as Okayama-Berg cDNA expression vector pcDV1 (Pharmacia), pCDM8, pRc/CMV, pcDNA1, pcDNA3 (Invitrogene), pSPORT1 (GIBCO BRL)) or pCl (Promega).

The transformation of the host cell with a nucleic acid molecule or vector according to the invention can be carried out by standard methods, as for instance described in Sambrook and Russell (2001), Molecular Cloning: A Laboratory Manual, CSH Press; Methods in Yeast Genetics, A Laboratory Course Manual, Cold Spring Harbor Laboratory Press (1990). For example, calcium chloride transfection is commonly utilized for prokaryotic cells, whereas, e.g., calcium phosphate or DEAE-Dextran mediated transfection or electroporation may be used for other cellular hosts. The host cell is cultured in nutrient media meeting the requirements of the particular host cell used, in particular in respect of the pH value, temperature, salt concentration, aeration, antibiotics, vitamins, trace elements etc. The fusion protein of use in the present invention can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromography and lectin chromatography. In view of the possession of a PBP portion, the fusion protein may be purified applying an affinity chromatography with a substrate to which the PBP portion binds. Protein refolding steps can be used, as necessary, in completing the configuration of the protein. Finally, high performance liquid chromatography (HPLC) can be employed for final purification steps.

Accordingly, a further embodiment of the disclosure relates to a method for producing the fusion protein useful in the invention comprising culturing the above-described host cells under conditions allowing the expression of said fusion protein and recovering said fusion protein from the culture. Depending on whether the expressed protein is localized in the host cells or is secreted from the cell, the protein can be recovered from the cultured cells and/or from the supernatant of the medium.

Moreover, the disclosure relates to fusion proteins which are obtainable by a method for their production as described above.

The fusion protein may, e.g., be a product of chemical synthetic procedures or produced by recombinant techniques from a prokaryotic or eukaryotic host (for example, by bacterial, yeast, higher plant, insect or mammalian cells in culture). Depending upon the host employed in a recombinant production procedure, the expressed fusion protein may be glycosylated or may be non-glycosylated. The fusion protein may also include an initial methionine amino acid residue. The protein used in performing the method of the invention may be further modified to contain additional chemical moieties not normally part of the protein. Those derivatized moieties may, e.g., improve the stability, solubility, the biological half life or absorption of the protein. The moieties may also reduce or eliminate any undesirable side effects of the protein and the like. An overview for these moieties can be found, e.g., in Remington's Pharmaceutical Sciences (18th edition, Mack Publishing Co., Easton, PA (1990)).

The present disclosure furthermore relates to non-human transgenic organisms, i.e. multicellular organisms comprising a nucleic acid molecule encoding a fusion protein or an expression cassette or vector as described above, preferably stably integrated into its genome, at least in a subset of the cells of that organism, or to parts thereof such as tissues or organs.

In a preferred embodiment the present disclosure relates to transgenic plants or plant tissue comprising transgenic plant cells as described above, i.e. comprising, preferably stably integrated into their genome, an above-described nucleic acid molecule, expression cassette or vector or to transgenic plants, plant cells or plant tissue obtainable by a method for their production as outlined below. The present disclosure also relates to a method for producing transgenic plants, plant tissue or plant cells comprising the introduction of a nucleic acid molecule, expression cassette or vector into a plant cell and, optionally, regenerating a transgenic plant or plant tissue therefrom. In particular, transgenic plants expressing a fusion protein of use in the invention can be of use for investigating metabolic or transport processes of, e.g., organic compounds with a timely and spatial resolution that was not achievable in the prior art. Such transgenic plants may furthermore be useful for screening herbicides.

Methods for the introduction of foreign nucleic acid molecules into plants are well-known in the art. For example, plant transformation may be carried out using Agrobacterium-mediated gene transfer, microinjection, electroporation or biolistic methods as it is, e.g., described in Potrykus and Spangenberg (Eds.), Gene Transfer to Plants. Springer Verlag, Berlin, New York (1995). Therein and in numerous other prior art references useful plant transformation vectors, selection methods for transformed cells and tissue as well as regeneration techniques are described which are known to the person skilled in the art and may be applied for the purposes of the present embodiment.

In yet another aspect, the disclosure relates to harvestable parts and to propagation material of the transgenic plants according which contain transgenic plant cells as described above. Harvestable parts can be in principle any useful part of a plant, for example, leaves, stems, fruit, seeds, roots etc. Propagation material includes, for example, seeds, fruits, cuttings, seedlings, tubers, rootstocks etc.

The disclosure also relates to a transgenic non-human animal comprising at least one nucleic acid molecule, expression cassette or vector of the invention as described above, preferably stably integrated into their genome.

The present disclosure also encompasses a method for the production of a transgenic non-human animal comprising introducing a nucleic acid molecule, expression cassette or vector into a germ cell, an embryonic cell, stem cell or an egg or a cell derived therefrom. It is preferred that such transgenic animals expressing the fusion protein of the invention or any developmental stage thereof starting from the zygote may be used as model organisms where it is possible to determine the distribution of a certain compound (depending on the PBP present in the fusion protein) in real time without disrupting tissue integrity. These model organisms may be particularly useful for nutritional or pharmacological studies or drug screening. Production of transgenic embryos and screening of them can be performed, e.g., as described by A. L. Joyner Ed., Gene Targeting, A Practical Approach (1993), Oxford University Press. The DNA of the embryos can be analyzed using, e.g., Southern blots with an appropriate probe or based on PCR techniques.

A transgenic non-human animal may, e.g., be a transgenic mouse, rat, hamster, dog, monkey, rabbit, pig, frog, nematode such as Caenorhabditis elegans, fruitfly such as Drosophilia melanogaster or fish such as torpedo fish or zebrafish comprising a nucleic acid molecule, expression cassette or vector, preferably stably integrated into its genome, or obtained by the method mentioned above. Such a transgenic non-human animal may comprise one or several copies of the same or different nucleic acid molecules. Preferably the presence of a nucleic acid molecule, expression cassette or vector in such a transgenic non-human animal leads to the expression of the fusion protein useful in the invention. The transgenic non-human animal has numerous utilities, including as a research model. Accordingly, in this instance, the mammal is preferably a laboratory animal such as a mouse or rat.

Thus, in a preferred embodiment, the transgenic non-human animal is a mouse, a rat or a zebrafish. Numerous reports revealed that said animals are particularly well suited as model organisms for the investigation of the drug metabolism and its deficiencies or cancer. Advantageously, transgenic animals can be easily created using said model organisms, due to the availability of various suitable techniques well known in the art.

The present invention relates to an in vitro method for detecting an analyte in a cell as defined in claim 1 of the claims appended hereto.

With this method, it is possible to measure the concentration of a given analyte solved in the sample over a molar range of several orders of magnitude. The term "sample" refers to any volume of a liquid or suspension in which an analyte to be measured can be present in solution.

The meaning of the phrase "energy suitable for exciting energy emission by said first and/or second detection portion" depends on which detection portions are present in the fusion protein used.

If the first detection portion is an energy-emitting protein and the second detection portion is a fluorescent protein portion, said energy should be suitable to excite the energy-emitting portion as it is described further above. Thus, if for example this energy-emitting protein portion is a fluorescent protein portion, the energy should be in the form of light radiation comprising a wavelength that excites this fluorescent protein portion.

The measurement of energy emission in step (c) may be carried out as described above. As with energy absorption, the signal of the measurable energy emitted depends on what kind of the detection portions is present in the fluorescent protein used. A resonance energy transfer (RET) signal is the signal indicative for the analyte concentration. Then, the term "energy emission" in step (c) refers to the light emission of both detection portions, whereby the ratio between the emission of the second detection portion to that of the first detection portion is indicative for the efficiency of RET allowing to draw a conclusion on the presence and concentration of analyte in the sample. The analysis of the energy emission values measured is preferably carried out by comparing these values with a standard curve showing the relationship of analyte concentration and energy emission for a given fluorescent protein of the invention. Such standard curves may be made by titration experiments for instance as described in Example 2, infra. For establishing such curves, preferably the same experimental conditions are applied as present during the effective measurement. Furthermore, titrations with different analyte dilutions can be performed to determine the analyte concentration at half-saturation. Using the equation: ([Analyte]=k_{d}/dilution at half-saturation), the analyte concentration in the sample can be calculated.

In a preferred embodiment of this method for detecting an analyte in a sample, the fusion protein is fixed to a solid support. Preferably, the binding affinity of the PBP portion of said fusion protein is in a range that allows reversible analyte binding. It is conceivable to use such fixed fusion proteins to construct measuring devices where said support containing the fusion protein is assembled with means for excitation and detection of energy emission. Such devices may be useful as sensors for detecting compounds in fluid samples, for example, for measuring blood sugar.

It is furthermore conceivable that a multitude of different fusion proteins being specific for different analytes and/or having different measuring ranges are assembled on one array. Upon contacting a sample to such an array, the array may for example be scanned with a combined excitation/detection means. Such a system may be of particular utility in automated or semi-automated high throughput screening of samples. Corresponding techniques for fixing proteins on a solid support as well as excitation and detection means necessary for such applications are known to the skilled person and are described in the literature such as in (Shriver-Lake, Biosens. Bioelectron (1997) 12, 1101-1106; Lin, Biotechniques (1999) 26, 318-22, 324-326; Blattner, Anal. Biochem. (2001) 295, 220-226; Liu, Bioconjug. Chem. (2000) 11; 755-761; Schuler, Analyst (1999) 124, 1181-1184; Vidal, Biomaterials (1999) 20, 757-763)

In a further preferred embodiment, the above-identified analyte detection method additionally comprises
(iv) calibrating the energy emission measurement of step (c) with the measurement of step (iii).

Thus, in addition to the above-mentioned steps (a) to (c), this embodiment provides steps (i) to (iii) which correspond to steps (a) to (c), whereby instead of a fusion protein capable of analyte detection a control sensor is used which allows a calibration of the measurements of step (c) in step (iv).

The term "control sensor" refers to a protein which corresponds to the fusion protein used in step (a) except for a modification of the PBP portion which renders it incapable of binding the respective analyte. Thus, apart from this, the control sensor has the same functional properties as the corresponding fusion protein with respect to the excitation and emission spectrum. Preferably, other characteristics are also identical such as solubility, isoelectric point, molecular weight, pH sensitivity, sensitivity to other factors such as ionic environment etc.

Most preferably, the control sensor is identical with the corresponding fusion protein except for said modified PBP portion. The modification of the PBP portion may be any conceivable structural change of the PBP portion that results in its incapability of analyte binding and may preferentially refer to mutations, i.e. deletions, insertions, inversions or substitutions of the amino acid sequence of the PBP portion. For example, in GGBP the substitution of the Asp residue at position 236 by an Ala residue and, in MBP, the substitution of the tryptophane residue at position 340 by an Ala residue was shown to render these PBP portions non-binding. The term "non-binding" refers to PBP portions which do not show a detectable conformational change at a physiological concentration, i.e. at concentrations of at least 100 nM, preferably at least 500 nM, of the corresponding substrate. Preferably, the overall three-dimensional structure of the control sensor is not significantly altered by such a structural change in order to have the same light-spectroscopic properties as the corresponding fusion protein.

It is contemplated that parallel measurements using a control sensor may greatly refine the measurements conducted with the fusion protein of the invention because it excludes influences apart from analyte concentration that may affect energy emission of the fusion protein. For instance, it has been shown that EYFP emission is reduced at increasing halide concentrations and decreasing pH (Miyawaki, Proc. Natl. Acat. Sci. USA 96 (1999), 2135-2140; Wachter, J. Mol. Biol. 301 (2000), 157-171; Jayaraman et al., J. Biol. Chem. (2000) 275, 6047-6050) which may interfere with the analyte measurement. Using control sensor measurements to calibrate analyte detection may eliminate such interferences from measurements made with a fusion protein containing EYFP as a detection portion.

Moreover, the overall protein structure of the fusion protein may be affected by other factors than analyte concentration such as ionic conditions. As an example, fusion proteins comprising a GGBP portion were shown to be sensitive to pH value. This applies to FLIPglu (comprising the wild type GGBP sequence) as well as to the control sensor FLIPglu D236A (see Figures 13 and 14). Thus, expectedly, the use of a control sensor, in particular for in vivo measurements, may significantly improve analyte detection since parameters other than analyte concentration, in particular ionic conditions such as pH that may affect overall protein structure, can be excluded to a great extent.

According to the explanations given above, it is possible to express the fusion protein in any cell which is accessible to molecular biological techniques, at any desired subcellular compartment for which target signal sequences are provided. Thereby the term "subcellular compartment" encompasses, in addition to intracellular compartments, also the extracellular space surrounding said cells such as the apoplast in plant tissue. With regard to supply of excitation energy and emission measurements, the same provisions apply as explained above in connection with the method for detecting an analyte in a sample.

The quality of measurement may be decisively improved by undertaking parallel measurements in cells expressing a control sensor. Such control experiments are particularly useful for detecting analytes in a cell or tissue where potentially existing parameters that may disturb measurements of energy emission such as halide concentration, osmotic conditions or pH value cannot be controlled as in in vitro experiments. Accordingly, the provisions for this method likewise apply to the present method.

The present invention relates in a further preferred embodiment to a method for detecting an analyte in a cell comprising:
(a) introducing into a cell by means of microinjection the fusion protein of the invention, wherein the PBP portion of said fusion protein is capable of binding said analyte, or RNA which encodes and is capable of expressing a suitable fusion protein in said cell, as afore said.

For intracellular analyte measurements, the fusion protein of the invention may be transferred into a cell by microinjection of RNA encoding the fusion protein and capable of expressing it in the cell. Apart from the way of introducing the fusion protein into the cell, this embodiment corresponds to the method for detecting an analyte in a cell described above, wherein the cells are genetically engineered with an nucleic acid molecule, expression construct or vector encoding and capable of expressing the fusion protein in the cell.

Suitable techniques for introducing protein-expressing RNA into cells by way of microinjection are known to the person skilled in the art and are described in the literature. For instance, the present preferred embodiment may be carried out by applying techniques as described in Celis J.E., Graessmann A., Loyter A. (1986), Microinjection and Organelle Transplantation Techniques, Academic Press, London; Celis J.E. (1994), Cell Biology: A Laboratory Handbook, Vol. 3, Academic Press, New York; and Cid-Arregui A. and Garcia-Carranca A. (eds) (1997), Microinjection and Transgenesis: Strategies and Protocols, Springer-Verlag, Berlin-Heidelberg-New York.

The use of microinjection for introducing the fusion protein of the invention, indirectly in the form of an expressable mRNA, brings about the advantage of a reduced time required until results are obtained, as compared to transformation approaches. In particular, it usually takes only a few days until the introduced mRNA expresses the encoded fusion protein and subsequently fluorescence can be measured. Another advantage of the present preferred embodiment is that the level of expression of the introduced mRNA, can easily be modulated by the amount of the microinjected material. Since fluorescence is known to be a sensitive method, usually relatively small amounts of material may be required in order to achieve significant measurements of intracellular analyte concentration. On the other hand, the application of microinjection may be less favorable than transformation approaches if it is intended to observe analyte concentration in a given cell for a longer time period than for instance a few days. Also, if analyte concentration in cellular compartments other than the cytoplasm or the nucleus is intended to be measured, microinjection may not be feasible since direct microinjection into such other compartments is, at least at present, not possible. Evidently, in the present embodiment, it may also be under certain circumstances useful to calibrate analyte measurements by parallel measurements using a control sensor as it is described above in connection with intracellular analyte measurements carried out at cells being genetically engineered so as to express the fusion protein.

Thus, in a particularly preferred embodiment, said method additionally comprises:
(i) introducing into a cell corresponding to the cell used in step (a) by means of microinjection a control sensor or RNA which encodes and is capable of expressing said control sensor, whereby said control sensor corresponds to the fusion protein used in step (a) with the exception that the PBP portion is modified and therefore incapable of binding said analyte;
(ii) supplying the cell of step (i) with the same energy as mentioned in step (a);
(iii) measuring the energy emission; and
(iv) calibrating the energy emission measurement of step (b) with the measurement of step (iii).

In a further preferred embodiment, the present invention relates to a method for the identification of a compound that affects the concentration and/or distribution of an analyte in a cell comprising:
(a) contacting a candidate compound with a cell that expresses a fusion protein according to the invention which is suitable for detecting said analyte; and
(b) determining whether said contacting leads to a change in the energy emission of said fusion protein.

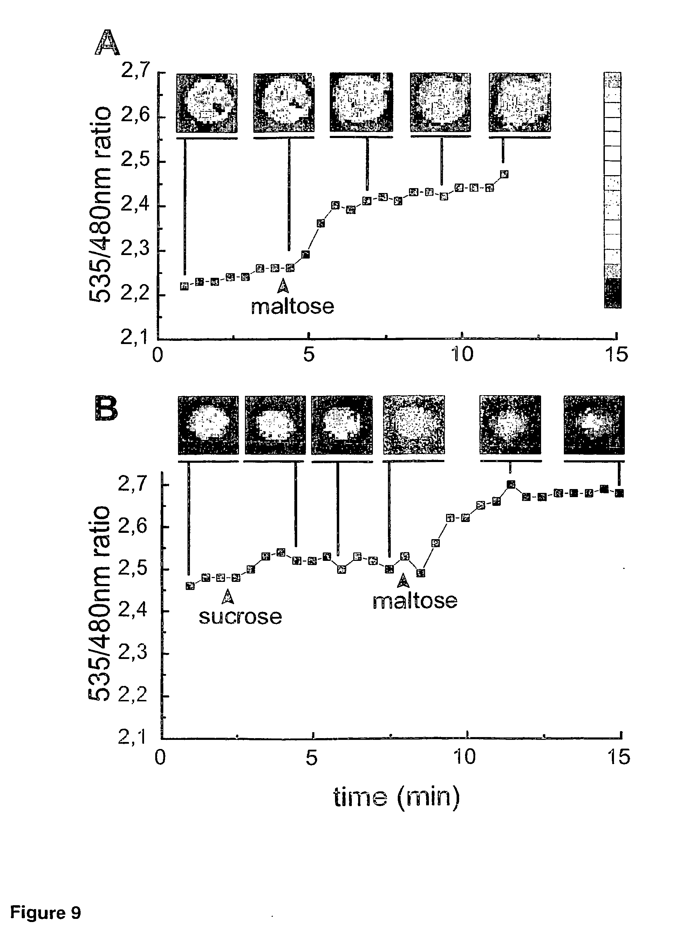

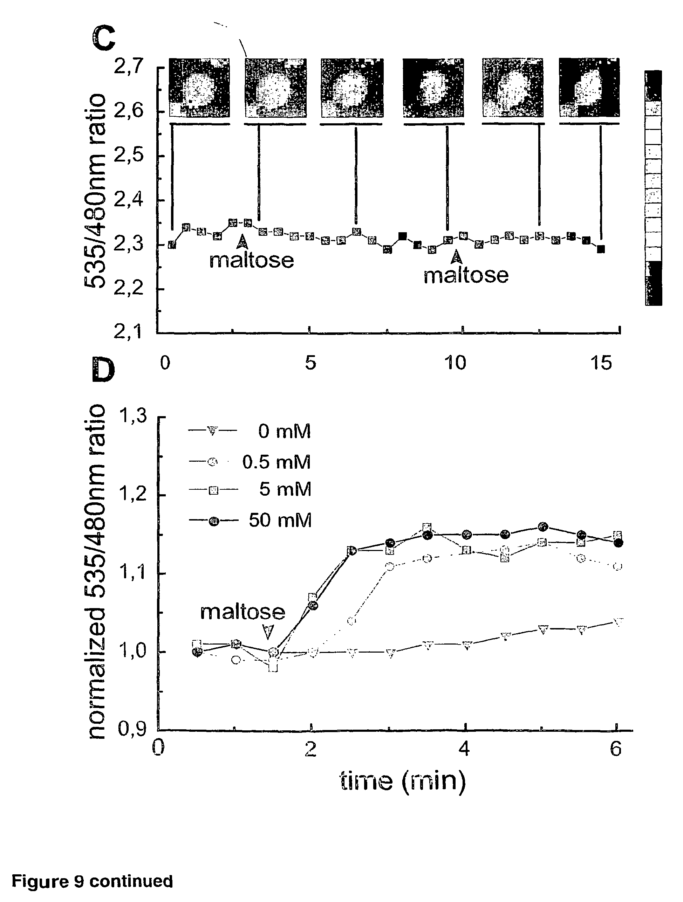

With the provision of the fusion protein disclosed herein, it has become possible to directly observe the distribution and concentration of a certain analyte (e.g. a metabolite) in a single living cell (see for instance Figure 9). This allows the observation whether contacting a single cell expressing an appropriate fusion protein with a candidate compound leads to a change in the concentration and/or distribution of the analyte in the cell, such a change being apparent from a change in the energy emission pattern that is emitted by the cell that expresses the fusion protein. In addition, the technology provided herein not only allows detecting whether there is a change in analyte concentration and/or distribution but it also allows quantifying such a change. A person skilled in the art will immediately appreciate that this technology may present an important contribution to pharmacological research, in particular in the field of drug screening. Thus, corresponding techniques for drug screening described in the literature are incorporated herein by reference. This includes for instance Kyranos (Curr. Opin. Drug. Discov. Devel. 4 (2001), 719-728), Pochapsky (Curr. Top. Med. Chem. 1 (2001), 427-441) and Bohets (Curr. Top. Med. Chem. 1 (2001), 367-383).

According to the present embodiment, in principle any kind of cell may be used for the present method that is amenable to optical detection and that can be transformed so as to express a heterologous protein. Thus, the cells may be single cells such as bacteria, yeasts, protozoa or cultured cells, e.g., of vertebrate, preferably mammalian, more preferably human origin or plant cells. For certain applications, it may be useful to take pathogenetically affected cells such as tumor cells or cells infected by an infectious agent, e.g. a virus, wherein preferentially measurements are conducted in comparison with corresponding healthy cells. Likewise, the cells may be part of a tissue, organ or organism. Preferably, the cells are immobilized which facilitates their observation. Immobilization may be put into practice, e.g., as described in Example 3.

The candidate compounds can in principle be taken from any source. They may be naturally occurring substances, modified naturally occurring substance, chemically synthesized substances or substances produced by a transgenic organism and optionally purified to a certain degree and/or further modified. Practically, the candidate compound may be taken from a compound library as they are routinely applied for screening processes.

The term "contacting" refers to the addition of a candidate compound to the analyzed cell in a way that the compound may become effective to the cell at the cell surface or upon cellular uptake. Typically, the candidate compound or a solution containing it may be added to the assay mixture. Step (a) may likewise be accomplished by adding a sample containing said candidate compound or a plurality of candidate compounds to the assay mixture. If such a sample or plurality of compounds is identified by the present method to contain a compound of interest, then it is either possible to isolate the compound from the original sample or to further subdivide the original sample, for example, if it consists of a plurality of different compounds, so as to reduce the number of different substances per sample and repeat the method with the subdivisions of the original sample. Depending on the complexity of the sample, the steps described herein can be performed several times, preferably until the sample identified according to the method of the invention only comprises a limited number of or only one substance(s). Preferably said sample comprises substances of similar chemical and/or physical properties, and most preferably said substances are identical.

Step (b) may be carried out in accordance with the explanations regarding measuring a change in energy emission of the fusion proteins as given hereinabove. Particularly preferred are optical measurement techniques that allow a resolution of fluorescence on the level of single cells, preferably at the subcellular level. Suitable imaging techniques are described in the literature such as in Periasamy A., Methods in Cellular Imaging, 2001, Oxford University Press or in Fluorescence Imaging Spectroscopy and Microscopy, 1996, edited by: X.F. Wang; Brian Herman. John Wiley and Sons. They may involve fluorescence, preferably confocal, microscopy, digital image recording, e.g. by way of a CCD camera, and suitable picture analysis software. In Example 3, a useful setting is described that gave rise to Figures 9A to C showing the concentration and distribution of maltose within a yeast cell expressing FLIPmal W230A. Preferentially, step (b) is carried out by running parallel control experiments. For instance, a corresponding cell expressing the same fusion protein may be observed under corresponding conditions as in steps (a) and (b), however, without contacting a candidate compound, In the alternative, a cell corresponding to that of steps (a) and (b) is contacted with the same candidate compound, however, this cell expresses a control sensor corresponding to the fusion protein used therein. Such an approach would be suited to exclude influences on the measurement that are not caused by the concentration and/or distribution of the analyte in the cell.

In yet a further embodiment, the invention relates to a method for the identification of a gene product involved in one or more enzymatic, transport or regulatory functions in a cell, wherein said functions affect the concentration and/or distribution of an analyte in the cell, said method comprising:
(a) providing a cell that expresses a fusion protein which is suitable for detecting said analyte, wherein the activity of a candidate gene product is altered in said cell compared to a corresponding wild type cell;
(b) culturing the cell of (a) under conditions that the fusion protein and, if appropriate, the candidate gene product is expressed and active; and
(c) determining whether, in the cell of (b), the energy emission of said fusion protein differs from that of the same fusion protein present in a cell corresponding to the cell of (b) in which the activity of said gene product is not altered, e.g. as in the corresponding wild-type cell.

This method allows the identification of genes the gene products of which affect the concentration and/or distribution of an analyte in a cell. For this purpose, the activity of a candidate gene product in a cell is altered compared to the corresponding wild type cell and the effect of this alteration on concentration and/or distribution of the analyte is observed in comparison to a corresponding cell in which the activity of said gene product is not altered.

Thereby, the term "alteration of gene product activity" may refer to a significant increase or decrease of the activity, depending on which question is to be answered. Thus, in one preferred embodiment of the present method for the identification of a gene product, the activity of a candidate gene product is increased as compared to the corresponding wild type cell, for instance, by at least 20%, preferably by at least 50%, more preferably by at least 100%. Most preferably, the increase starts from zero since the respective candidate gene product is not at all present in the corresponding wild type cell, for instance because the candidate gene product is heterologous for the cell with regard to its origin. If the biological activity of the gene product, e.g. the enzymatic activity, is not available, the increase in gene product activity may likewise be inferred from the abundance of the protein or the corresponding transcript in the cell under investigation.

Methods for increasing the activity of a certain gene product in a cell are known to a person skilled in the art and are widely described in the literature. For instance, the methods for expressing a fusion protein of use in the invention described in detail above may be applied correspondingly for over-expressing a candidate gene product in a given cell. In this context, step (b) of the method thus involves culturing the cell of (a) under conditions that both the fusion protein and the candidate gene product is expressed and active.

Advantageously, the method for identifying a gene product may be carried out by transforming a population of cells that may already carry a gene for the fusion protein to be used with a library of expression clones comprising open reading frames for potential candidate gene products. The population of transformed cells may then be screened by performing steps (b) and (c) for cells in which the concentration and/or distribution of a certain analyte deviates from that of a corresponding cell which only expresses the fusion protein and which is cultured under the same conditions as the cell of step (b).

In another preferred embodiment of the method, for identifying a gene product, the activity of a candidate gene product is reduced as compared to the corresponding wild type cell, for instance by at least 20%, preferably by at least 50% and more preferably by at least 80%. Most preferably, the activity is reduced by 100%, i.e. the gene product is not at all active in the cell, for instance because the gene encoding the candidate gene product is completely knocked out. Preferably, the activity of the gene product is defined by way of its biological activity, e.g. the enzymatic activity. If for any reason the biological activity is not available, the reduction of gene product activity may likewise be inferred from the abundance of the protein or the corresponding transcript in the cell under investigation.

Methods for reducing the activity of a candidate gene product in a cell are known to a person skilled in the art and are widely described in the literature. For instance, the activity of endogenous gene products may be reduced in a randomized manner such as by chemical mutagenesis or by randomly inserting genetic elements such as by gene or transposon tagging. Mutants obtained in this way may be identified in step (c) of the present method.

On the other hand, the activity of a given gene product may also be reduced specifically. For such an approach, a variety of techniques is provided in the state of the art. On the microbiological scale and, preferably, for genes that are not essential for the survival of the cell, genes may be knocked out by in vivo mutagenesis involving homologous recombination with the target gene. Suitable heterologous DNA sequences that can be taken for such an approach are described in the literature and include, for instance vector sequences capable of self-integration into the host genome or mobile genetic elements.

Generally, techniques for specifically reducing the activity of a gene product in a cell include but are not limited to antisense, ribozyme, co-suppression, RNA interference, expression of dominant negative mutants, antibody expression and in vivo mutagenesis approaches. These methods are further explained in the following.

Accordingly, reduction of candidate gene product activity may be achieved by transforming the cell with a nucleic acid molecule encoding an antisense RNA which is complementary to the transcripts of a gene encoding the candidate gene product. Thereby, complementarity does not signify that the encoded RNA has to be 100% complementary. A low degree of complementarity may be sufficient as long as it is high enough to inhibit the expression of the candidate gene product upon expression of said RNA in the cells used in the present method of the invention. The transcribed RNA is preferably at least 90% and most preferably at least 95% complementary to the transcript of the candidate gene product. In order to cause an antisense effect during the transcription in cells such RNA molecules have a length of at least 15 bp, preferably a length of more than 100 bp and most preferably a length of more than 500 bp, however, usually less than 2000 bp, preferably shorter than 1500 bp. Exemplary methods for achieving an antisense effect are for instance described by Müller-Rober (EMBO J. 11 (1992), 1229-1238), Landschütze (EMBO J. 14 (1995), 660-666), D'Aoust (Plant Cell 11 (1999), 2407-2418) and Keller (Plant J. 19 (1999), 131-141) and are herewith incorporated in the description of the present invention. Likewise, an antisense effect may also be achieved by applying a triple-helix approach, whereby a nucleic acid molecule complementary to a region of the gene encoding the relevant candidate gene product, designed according to the principles for instance laid down in Lee (Nucl. Acids Res. 6 (1979), 3073); Cooney (Science 241 (1998), 456) or Dervan (Science 251 (1991), 1360) may inhibit its transcription.

A similar effect as with antisense techniques can be achieved by constructs that mediate RNA interference (RNAi). Thereby, the formation of double-stranded RNA leads to an inhibition of gene expression in a sequence-specific fashion. More specifically, in RNAi constructs, a sense portion comprising the coding region of the gene to be inactivated (or a part thereof, with or without non-translated region) is followed by a corresponding antisense sequence portion. Between both portions, an intron not necessarily originating from the same gene may be inserted. After transcription, RNAi constructs form typical hairpin structures. In accordance with the teachings of the present invention, the RNAi technique may be carried out as described by Smith (Nature 407 (2000), 319-320) or Marx (Science 288 (2000), 1370-1372).

Also DNA molecules can be employed which, during expression in cells, lead to the synthesis of an RNA which reduces the expression of the gene encoding the candidate gene product in cells due to a co-suppression effect. The principle of co-suppression as well as the production of corresponding DNA sequences is precisely described, for example, in WO 90/12084. Such DNA molecules preferably encode an RNA having a high degree of homology to transcripts of the target gene. It is, however, not absolutely necessary that the coding RNA is translatable into a protein. The principle of the co-suppression effect is known to the person skilled in the art and is, for example, described in Jorgensen, Trends Biotechnol. 8 (1990), 340-344; Niebel, Curr. Top. Microbiol. Immunol. 197 (1995), 91-103; Flavell, Curr. Top. Microbiol. Immunol. 197 (1995), 43-36; Palaqui and Vaucheret, Plant. Mol. Biol. 29 (1995), 149-159; Vaucheret, Mol. Gen. Genet. 248 (1995), 311-317; de Borne, Mol. Gen. Genet. 243 (1994), 613-621 and in other sources.

Likewise, DNA molecules encoding an RNA molecule with ribozyme activity which specifically cleaves transcripts of a gene encoding the candidate gene product can be used. Ribozymes are catalytically active RNA molecules capable of cleaving RNA molecules and specific target sequences. By means of recombinant DNA techniques, it is possible to alter the specificity of ribozymes. There are various classes of ribozymes. For practical applications aiming at the specific cleavage of the transcript of a certain gene, use is preferably made of representatives of the group of ribozymes belonging to the group I intron ribozyme type or of those ribozymes exhibiting the so-called "hammerhead" motif as a characteristic feature. The specific recognition of the target RNA molecule may be modified by altering the sequences flanking this motif. By base pairing with sequences in the target molecule, these sequences determine the position at which the catalytic reaction and therefore the cleavage of the target molecule takes place. Since the sequence requirements for an efficient cleavage are low, it is in principle possible to develop specific ribozymes for practically each desired RNA molecule. In order to produce DNA molecules encoding a ribozyme which specifically cleaves transcripts of a gene encoding a candidate gene product, for example a DNA sequence encoding a catalytic domain of a ribozyme is bilaterally linked with DNA sequences which are complementary to sequences encoding the target protein. Sequences encoding the catalytic domain may for example be the catalytic domain of the satellite DNA of the SCMo virus (Davies, Virology 177 (1990), 216-224 and Steinecke, EMBO J. 11 (1992), 1525-1530) or that of the satellite DNA of the TobR virus (Haseloff and Gerlach, Nature 334 (1988), 585-591). The expression of ribozymes in order to decrease the activity of certain proteins in cells is known to the person skilled in the art and is, for example, described in EP-B1 0 321 201. The expression of ribozymes in plant cells is for example described in Feyter (Mol. Gen. Genet. 250 (1996), 329-338).

Furthermore, nucleic acid molecules encoding antibodies specifically recognizing the candidate gene product in the cell, i.e. specific fragments or epitopes thereof, can be used for inhibiting the activity of this protein. These antibodies can be monoclonal antibodies, polyclonal antibodies or synthetic antibodies as well as fragments of antibodies, such as Fab, Fv or scFv fragments etc. Monoclonal antibodies can be prepared, for example, by the techniques as originally described in Köhler and Milstein (Nature 256 (1975), 495) and Galfré (Meth. Enzymol. 73 (1981) 3), which comprise the fusion of mouse myeloma cells to spleen cells derived from immunized mammals. Furthermore, antibodies or fragments thereof to a candidate gene product can be obtained by using methods which are described, e.g., in Harlow and Lane "Antibodies, A Laboratory Manual", CSH Press, Cold Spring Harbor, 1988.

Moreover, nucleic acid molecules encoding peptides or polypeptides other than antibodies and that are capable of reducing the activity of a candidate gene product can be used in the present context. Examples of suitable peptides or polypeptides can be taken from the prior art and include, for instance, binding proteins such as lectins.

In addition, nucleic acid molecules encoding a mutant form of the candidate gene product can be used to interfere with the activity of the wild-type protein. Such a mutant form preferably has lost its biological activity, and may be derived from the corresponding wild-type protein by way of amino acid deletion(s), substitution(s), and/or additions in the amino acid sequence of the protein. Mutant forms of such proteins may show, in addition to the loss of, e.g., the enzymatic activity, an increased substrate affinity and/or an elevated stability in the cell, for instance, due to the incorporation of amino acids that stabilize proteins in the cellular environment. These mutant forms may be naturally occurring or, as preferred, genetically engineered mutants.

Alternatively, a reduction of candidate gene product activity may likewise be achieved by using nucleic acid molecules, the presence of which in the genome of a cell does not require its expression to exert its reducing effect on the activity of the candidate gene product. Correspondingly, preferred examples relate to methods wherein said reduced activity is achieved by in vivo mutagenesis or by the insertion of a heterologous DNA sequence into the corresponding gene.

The term "in vivo mutagenesis" relates to methods where the sequence of the gene encoding the relevant candidate gene product is modified at its natural chromosomal location such as for instance by techniques applying homologous recombination. This may be achieved by using a hybrid RNA-DNA oligonucleotide ("chimeroplast") which is introduced into cells by transformation (TIBTECH 15 (1997), 441-447; WO95/15972; Kren, Hepatology 25 (1997), 1462-1468; Cole-Strauss, Science 273 (1996), 1386-1389). Part of the DNA component of the RNA-DNA oligonucleotide is homologous to the target gene sequence, however, displays in comparison to this sequence a mutation or a heterologous region which is surrounded by the homologous regions. The term "heterologous region" refers to any sequence that can be introduced and which differs from the target sequences of the candidate gene product to be mutagenized. By means of base pairing of the homologous regions with the target sequence followed by a homologous recombination, the mutation or the heterologous region contained in the DNA component of the RNA-DNA oligonucleotide can be transferred to the corresponding gene of the cell. By means of in vivo mutagenesis, any part of the gene encoding the candidate gene product can be modified as long as it results in a decrease of its activity. Thus, in vivo mutagenesis can for instance concern, the promoter, e.g. the RNA polymerase binding site, as well as the coding region, in particular those parts encoding the substrate binding site or the catalytically active site or a signal sequence directing the protein to the appropriate cellular compartment.

Another example of insertional mutations that may result in gene silencing includes the duplication of promoter sequences which may lead to a methylation and thereby an inactivation of the promoter (Morel, Current Biology 10 (2000), 1591-1594). Furthermore, it is immediately evident to the person skilled in the art that the above-described approaches, such as antisense, ribozyme, co-suppression, in-vivo mutagenesis, RNAi, expression of antibodies, other suitable peptides or polypeptides or dominant-negative mutants and the insertion of heterologous DNA sequences, can also be used for the reduction of the expression of genes that encode a regulatory protein such as a transcription factor, that controls the expression of a candidate gene product or, e.g., proteins that are necessary for the candidate gene product to become active.

The above described methods for specifically reducing the activity of a gene product may for instance be of special utility for organisms where the sequence of the genome has been determined and individual genes are to be characterized functionally.

Step (b) of the method for identifying gene products may be carried out in accordance with suitable prior art methods such as those described in Sambrook and Russell (2001; loc. cit.). In cases where the measured analyte is imported from the exterior and the candidate gene product is involved in the import of the analyte, it may be that step (b) furthermore, involves the step of adding an appropriate amount of analyte or analyte precursor to the cell.

Step (c) may be carried out similar to step (b) of the aforementioned method for identifying a compound that affects the concentration and/or distribution of an analyte whereby, here, cells that have the activity a candidate gene product altered are compared with corresponding cells being in the wild type state with respect to the candidate gene product and, in the latter, cells being contacted with a candidate compound are compared with corresponding cells not being contacted with said candidate compound.

The present disclosure furthermore relates to a control sensor comprising:
(a) two detection portions, whereby
   (i) the first detection portion is an energy-emitting protein portion and the second detection portion is a fluorescent protein potion; or
   (ii) the two detection portions are portions of a split fluorescent protein; and
(b) a PBP portion fused to said first and second detection portion which is modified with respect to the corresponding wild-type PBP portion and therefore is incapable of binding the compound which said wild-type PBP portion binds;
wherein said first and/or second detection portion emits energy.

For putting this embodiment into practice, the same provisions apply as those outlined in connection with the fusion protein of the invention (apart from the provisions referring to the binding properties of the PBP). Preferably, the control sensor is applied in a method for detecting an analyte in a sample or in vivo as described above. For this purpose, the control sensor is to be devised so as to correspond to the fusion protein used in this method.

In a preferred embodiment, the PBP portion of the control sensor is a glucose/galactose binding protein (GGBP) which shows the amino acid residue Ala at a position corresponding to the Asp residue at position 236 of a mature wild-type (without leader peptide) GGBP as shown in SEQ ID NO. 4 or it is a maltose binding protein (MBP) which shows the amino acid residue Ala at a position corresponding to the Trp residue at position 340 of a mature wild-type MBP (without leader peptide) as shown in SEQ ID NO: 2.

Preferably, the PBP portion of this control sensor has the sequence shown in SEQ ID NO: 4, wherein the aspartate residue at position 236 is substituted by an alanine residue, or the sequence shown in SEQ ID NO:2 wherein the tryptophane residue at position 340 is substituted by an alanine residue.

Moreover, the disclosure relates in another embodiment to nucleic acid molecules comprising a nucleotide sequence encoding the control sensor of the invention as described above.

Consequently, the present disclosure also relates to expression cassettes, vectors and host cells comprising such a nucleic acid molecule whereby all the explanations made above in connection with the nucleic acid molecule, expression cassette, vector and host cells comprising a nucleotide sequence encoding the fusion protein of use in the invention herewith also apply.

In yet another embodiment, the disclosure relates to diagnostic compositions comprising the fusion protein of use in the invention or the nucleic acid molecule, the expression cassette, the vector, the host cell or the control sensor as described above.

Such a diagnostic composition may for instance be useful for diagnosing pathologically increased or decreased concentrations of a given compound in a sample taken from an individual.

In addition, another embodiment of the disclosure relates to a kit comprising the fusion protein of use in the invention or the nucleic acid molecule, the expression cassette, the vector, the host cell or the control sensor, as described above.

Such a kit may advantageously be used for carrying out the methods of the invention and could be, inter alia, employed in a variety of applications referred to herein as for examples for the uses mentioned infra. It is contemplated that the kit includes further ingredients such as standard equipment for applying molecular biological or other appropriate techniques. The parts of the kit can be packaged individually in vials or in combination in containers or multicontainer units. Manufacture of the kit follows preferably standard procedures which are known to the person skilled in the art. The kit may include instructions which are written in a way so as to enable a person skilled in the art to carry out any of the methods described therein.

Yet another embodiment of the present disclosure relates to the use of the fusion protein or the nucleic acid molecule, the expression cassette, the vector, the host cell or the control sensor, as described above, for detecting analytes in vitro.

Furthermore, the present disclosure also relates to the use of the fusion protein or the nucleic acid molecule, the expression cassette, the vector, the host cell or the control sensor, as described above, for preparing a diagnostic composition for diagnosing a condition which is correlated with a concentration of an analyte in cells, tissues or parts of a body, preferably said condition is a pathological condition correlated with an abnormal concentration of an analyte.

In yet another aspect, the present disclosure relates to the use of the fusion protein or the nucleic acid molecule, the expression cassette, the vector, the host cell or the control sensor as described above, for the identification of a compound that affects the concentration and/or distribution of an analyte in a cell.

This use may be carried out according to the teachings given above in connection with the method for the identification of a compound that affects the concentration and/or distribution of an analyte in a cell. However, the fusion protein and the other above-mentioned compounds may likewise be subjected to this use by applying any method which the person skilled in the art considers appropriate in order to achieve the intended result.

Another embodiment of the disclosure relates to the use of the fusion protein or the nucleic acid molecule, the expression cassette, the vector, the host cell or the control sensor as described above, for the identification of a gene product involved in one or more enzymatic, transport or regulatory functions, wherein said functions affect the concentration and/or distribution of an analyte in the cell.

This use may be carried out according to the teachings given above in connection with the method for the identification of a gene product involved in enzymatic, transport or regulatory functions. However, the fusion protein and the other above-mentioned compounds may likewise be subjected to this use by applying any method which a person skilled in the art considers appropriate in order to achieve the intended result.

Moreover, the present invention relates to the use of a control sensor , the PBP portion of which is derived from a GGBP, for measuring the pH.

This embodiment makes use of the surprising property of control sensors containing a PBP portion derived from a GGBP to show pH-dependent energy emission. Figure 14 shows that FRET of the control sensor FLIPglu D236A expressed in yeast is considerably decreased upon the addition of glucose. Since glucose uptake by yeast is known to decrease the cytosolic pH, it is contemplated that GGBP-derived control sensors show a pH sensitivity corresponding to that of GGBP-derived fusion proteins which are capable of analyte binding (see for instance Figure 13). In view of this finding, it is immediately evident to the person skilled in the art that control sensors the PBP portion of which is derived from a GGBP are convenient tools for measuring the pH value. In particular, this applies to in vivo applications. Specifically, by combining the coding sequence for the control sensor with suitable targeting sequences, in principle the pH of any subcellular compartment within a cell or extracellularly can be measured using such a control sensor. In a preferred embodiment, the present use of a GGBP-derived control sensor is combined with the use of a corresponding fusion protein, preferably a corresponding control sensor that has been made more insensitive to pH changes by modifying at least one histidine and/or by suitable truncation of the PBP portion, as it is described further above. Such a combination may improve pH measurements by comparing energy emission measurements of the pH-sensitive control sensor with those of the pH-insensitive protein thereby making it possible to exclude other factors than pH from the measurements. Furthermore, a GGBP-derived control sensor being sensitive to pH can be used to determine pH changes that occur in parallel to changes in glucose concentrations and to calibrate glucose measurements performed by pH-sensitive glucose sensors. The term "derived from a GGBP" refers to a PBP portion within the control sensor which is a GGBP sequence, i.e. SEQ ID NO:4 or a derivative thereof, whereby said GGBP sequence is modified so as to be incapable of analyte binding. In this context, the term "derivative" refers to any GGBP variant which is in accordance with the aforementioned description for PBP portion variants for use in the fusion protein i.e. for example homologues from a different species, truncated versions, GGBPs encoded by nucleotide sequences hybridizing to SEQ ID NO:3 or other mutant or modified forms thereof, whereby these variants undergo a conformational change upon analyte, e.g. glucose, binding and are susceptible to modification resulting in a PBP portion that is incapable of analyte binding. Clearly, such variants do not include those that show a decreased pH sensitivity that makes them useless for pH measurements such as the GGBP variants with modified histidine residues described above and/or being correspondingly truncated.

The person skilled in the art knows how to carry out the modification of the GGBP sequence so as to achieve the incapability of analyte binding. For instance, he/she may adapt the D236A substitution described herein with respect to the E. coli GGBP sequence shown under SEQ ID NO:4 to the respective GGBP sequence under investigation. Likewise, other sites involved in analyte binding and/or the conformational change may be chosen according to the knowledge on 3-dimensional structure and function of individual amino acid residues in GGBP's as described in the prior art literature

These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the Internet, for example under http://www.ncbi.nlm.nih.gov/PubMed/medline.html. Further databases and addresses, such as http://www.ncbi.nlm.nih.gov/, http://www.infobiogen.fr/,http://www.fmi.ch/biology/research_tools.html, http://www.tigr.org/, are known to the person skilled in the art and can also be obtained using, e.g., hftp://www.lycos.com. An overview of patent information in biotechnology and a survey of relevant sources of patent information useful for retrospective searching and for current awareness is given in Berks, TIBTECH 12 (1994), 352-364.

The present invention is further described by reference to the following non-limiting figures and examples.

The Figures show:
- **Figure 1:**: shows the two principle possibilities how a conformational change in a fusion protein may lead to a change of fluorescent resonance energy transfer (FRET) A. FLIPmal is an example for a fusion protein of use in the invention where binding of a substrate (depicted as a hexagon) leads to an increase of FRET. In the unbound state the two lobes of the PBP portion (ellipses) are open so that most of the light (flash) received by the first detection portion (upper circle) is directly emitted. Upon binding of a substrate, the two lobes of the PBP portion approach to one another resulting in an increase of FRET which can be detected in the form of light emission by the second detection portion (lower circle). B. FLIPglu represents fusion proteins of use in the invention where substrate binding leads to a decrease of FRET. The detection portions are attached to the PBP portion at sites where closing of the lobes upon substrate binding results in a decrease of FRET, e.g. because of an increased distance between them.
- **Figure 2:**: shows the emission spectra for FLIPmal-5AA purified after overexpression in E.coli cells. The grey curve depicts emission in the absence of maltose and the black curve in the presence of 20 mM maltose. As it is apparent, the ratio between fluorescence emission at 485 nm and that at 530 nm decreases which indicates an increase of FRET.
- **Figure 3:**: shows eight titration curves for FLIPmal W230A(A), FLIPmal W62A(B), FLIPmal-5AA(C), FLIPmal W340A(D), FLIPglu(E), FLIPglu-5AA(F), FLIPgluD236A(G), FLIPglu-10AA(H) and FLIPglu F16A (I). The diagrams show the degree of FRET indicated as the ratio 530 nm/485 nm (right y-axis) in dependency on maltose and glucose concentration, respectively.
- **Figure 4:**: depicts various expression constructs for use in different host organisms and for targeting to various subcellular compartments. In each of these constructs, a coding region of a PBP portion ("Binding Protein") is fused to nucleotide sequences encoding ECFP and EYFP. Specifically, the plant expression cassettes shown in A and B contain octopine synthase promoter/enhancer elements (Aocs), a manopine synthase promoter (AmasPmas) and an agropine synthase terminator (ags-ter) each from A. tumefaciens. For subcellular targeting, the constructs shown contain following additional elements: A(ii): N-terminal transit peptide of *A*. *thaliana* RNA polymerase AtRpoT;1 for mitochondrial targeting (Hedtke, Plant J. (1999) 17, 557-561); A(iii): N- terminal transit peptide of *A*. *thaliana* RNA polymerase AtRpoT;3 for chloroplast targeting (Hedtke, Plant J. (1999) 17, 557-561); A(iv): synthetic N-terminal ER targeting sequence (SEQ ID NO:6) together with C-terminal extension HDEL (see SEQ ID NO:7) for targeting to the ER (Haselhoff, Proc. Natl. Acad. Sci. 94 (1997), 2122-2127; B(i): *N*. *tabacum* c2 polypeptide nuclear localisation sequence (NLS) shown in SEQ ID NO:8 allowing nuclear import (Robert, Plant J. (1997) 11, 573- 586); B(ii): *S*. *tuberosum* vacuolar proteinase inhibitor II N-terminal sequence (pl-II signal) allowing secretion (von Schaeven et al., EMBO J. 9 (1990), 3033-3044); B(iii): *S*. *tuberosum* patatin N-terminal portion for vacuolar targeting comprising the 5'-untranslated leader, the 23 amino acid signal peptide and the 123 N-terminal amino acids from the mature patatin protein (Sonnewald et al., Plant J. 1 (1991), 95-106); B(iv): rabbit sucrase-isomaltase N-terminal signal anchor anchoring the fusion protein in the membrane (Hegner, J. Biol. Chem. 267 (1992), 16928-16933). Further expression cassettes include following elements: C(i): Bacterial expression by the pRSET vector (InVitrogen) containing T7-polymerase promoter (PT7) and His-tag (6xhis); C(ii): pcDNA3.1/Hygro-vector for mammalian expression (InVitrogen) comprising the immediate early promoter-enhancer of human cytomegalovirus (pCMV) and bovine growth hormone termination and polyadenylation sequence (BGHpa); C(iii): pIB/V5-His vector for expression in insect and insect cell lines (InVitrogen) comprising the OplE2-promoter for constitutive expression; C(iv): PDR195 vector for expression in yeast plasma membrane comprising the ATPase promoter (PMA-1) (S. cerevisiae) and the alcohol dehydrogenase (ADH) terminator (S. cerevisiae); D(i): Expression in C. elegans muscles using the C. elegans myosin heavy chain myo-3 promoter (myo-3) and the C. elegans myosin heavy chain unc-54 3'-end (Miller, BioTechniques (1999) 26, 941-921 ); D(ii): Expression in zebrafish using the 175 bp enhancer/278 bp promoter/5'-UTR of Xenopus ef1alpha gene (Ef1alphaE/P/UTR) and the simian virus 40 polyadenylation signal (SV40) (Amsterdam, Dev. Biol. (1995) 171, 123-129)

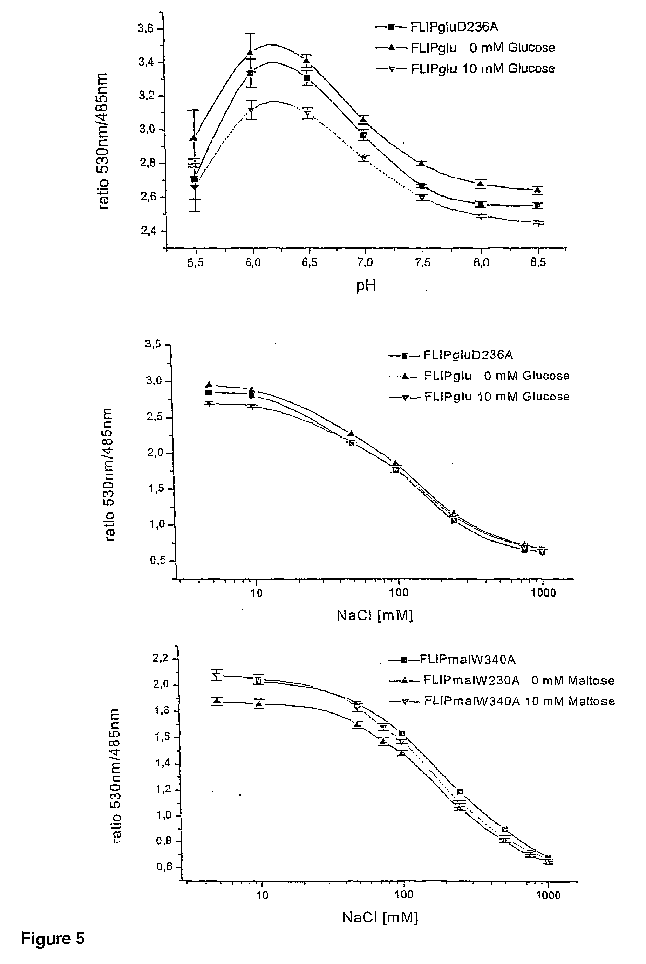

- **Figure 5:**: illustrates the function of FLIPglu D236A and FLIPmal W340A as control sensors; FLIPglu D236A and FLIPglu display a similar change in ratio with changing chloride concentrations and pH. FLIPmal W340A and FLIPmal show a similar chloride dependency.

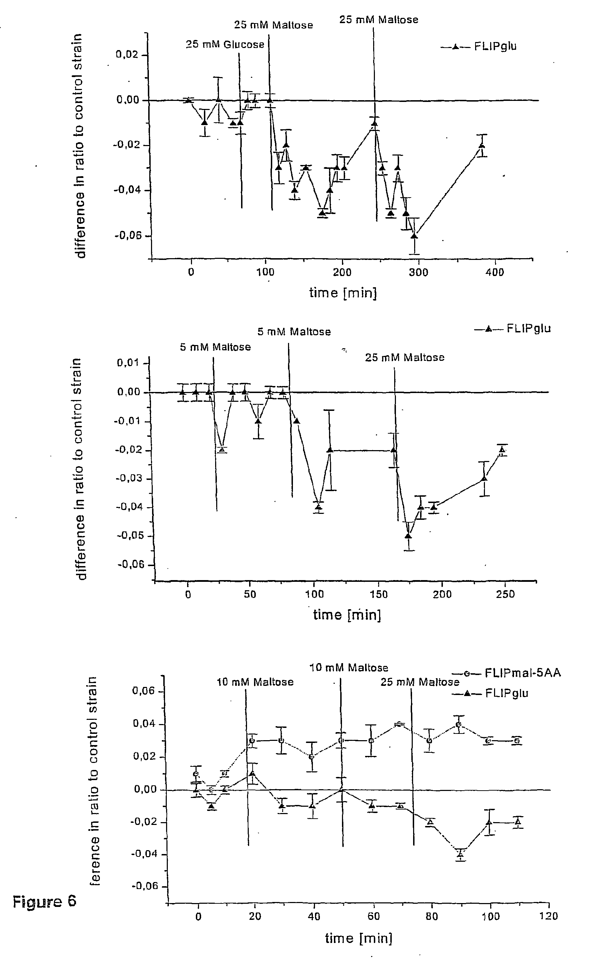

- **Figure 6:**: shows the difference in ratio observed in FLIPglu and FLIPmal-5AA expressing yeast upon incubation with analyte as compared to a control without analyte using a microtiterplate fluorometer. For FLIPglu expressing yeast, the EYFP-ECFP emission intensity ratio decreased upon addition of maltose whereas for the FLIPmal-5AA expressing yeast the emission intensity ratio increased as compared to the untreated control. Since glucose can not be taken up by this yeast strain, addition of glucose to the FLIPglu expressing yeast did not change the ratio, whereas maltose after being taken up by the yeast was metabolized into cytosolic glucose that then was detected by FLIPglu.

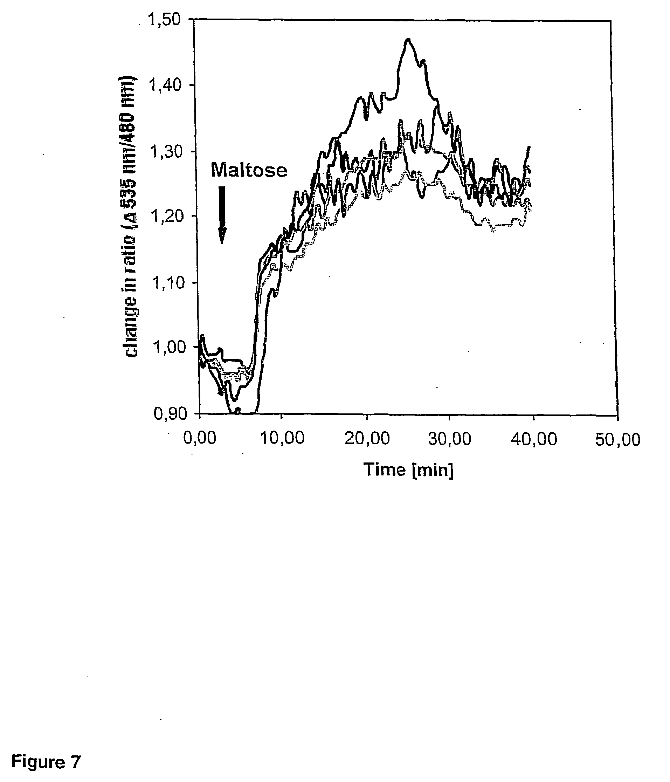

- **Figure 7:**: shows the change in ratio observed for FLIPmal-W230A expressing yeast upon incubation with maltose using single cell imaging (microscope equipped with a CCD camera). Upon addition of 47 mM maltose after 4.13 min the ratio is increasing rapidly after a short lag phase caused by diffusion through the embedded media.

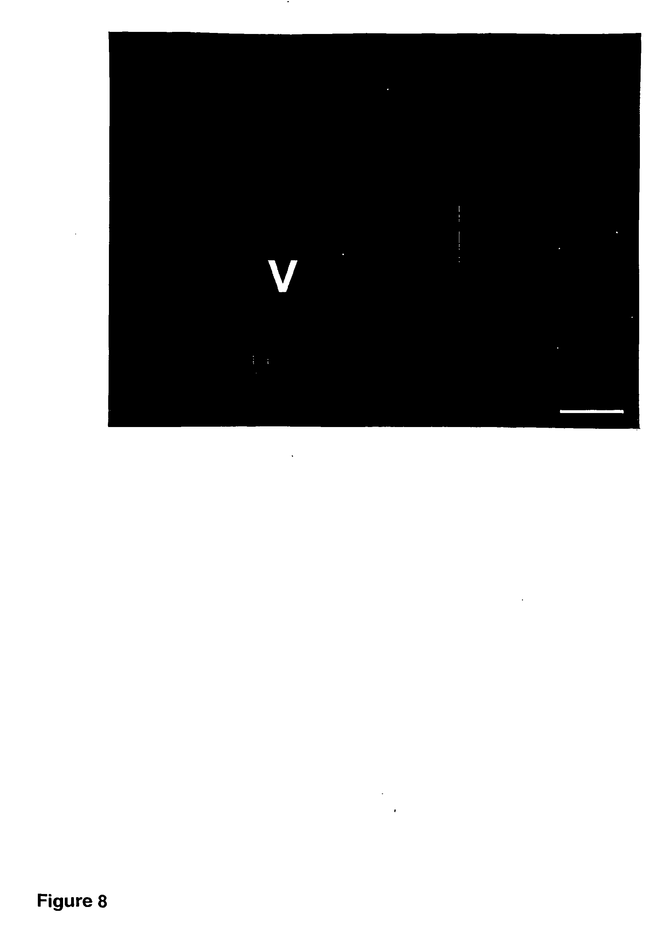

- **Figure 8:**: displays confocal imaging of FLIPmal W230A expressed in yeast. FLIPmal W230A is detected in the cytosol whereas no signal was found in the vacuole (V). Bar = 1 µm.
- **Figure 9:**: represents a visualization of dynamic maltose concentration changes in the cytosol. (A) and (B) Yeast expressing StSUT1 for maltose uptake into the cytosol and FLIPmal W230A. Each graph indicates the emission intensity ratio (535/480nm ratio) for a single yeast cell. Addition of maltose increased the ratio by 0.15 to 0.2, whereas addition of sucrose had no effect on the emission intensity ratio. Notably, Figure 9A is a more detailed view of Figure 7. (C) Yeast expressing StSUT1 and FLIPmal W340A. Addition of extracellular maltose or sucrose did not increase the ratio. Yeast images are pseudocolored to demonstrate the ratio change. Extracellular sugar solutions were added at the indicated time points at a final concentration of 50 mM (arrow head). (D) EBY4000 strain: Each graph indicates the average emission intensity ratio of four to seven cells. Addition of low levels of maltose (0.5mM ●, olive) lead to a retarded change in ratio as compared to higher levels (5mM ■; 50mM ●, blue), no change was observed with addition of water (▼).
- **Figure 10:**: gives a comparison of the substrate specificity of two FLIPmal mutants. The ratio change of purified mutants FLIPmal-5AA (A) and FLIPmal W230A (B) was tested in the presence of various pentoses, hexoses, sugar alcohols and di- and trisaccharides at three different concentrations. A significant increase in ratio was only observed in the presence of maltose. (C) The maximum change in ratio (left axis; black bars) and the affinity constant (K_{d}; right axis; white bars) of FLIPmal- 5AA were analysed in the presence of different maltosides (G2 to G7), soluble starch (S) and beer (B). The maximum change in ratio decreases with increasing chain length, whereas the K_{d} remains at a similar range. (D) Purified FLIPmaI-5AA (left curve) and FLIPmal W230A (right curve) were titrated with different dilutions of beer. The dilution at half-saturation can be used to determine the maltose concentration.
- **Figure 11:**: illustrates the substrate specificity of purified FLIPglu in the presence of various pentoses, hexoses, sugar alcohols and di- and trisaccharides at three different concentrations. A significant decrease in ratio was observed in the presence of most tested sugars.
- **Figure 12:**: illustrates the substrate specificity of purified FLIPglu F16A in the presence of various pentoses, hexoses, sugar alcohols and di- and trisaccharides at three different concentrations. Only galactose and ribose at 100 mM lead to a decrease in ratio.
- **Figure 13:**: shows the *in vitro* pH sensitivity of FLIPglu in the physiological range. The ratio shows a peak with high slopes at approximately pH 6.
- **Figure 14:**: indicates the consequence of adding external glucose to yeast expressing (A) the control sensor FLIPglu D236A or (B) the fusion protein FLIPglu. The graphs indicate the average emission intensity ratio of seven cells. Addition of 50 mM external glucose leads to a decrease in ratio followed by a subsequent increase. The decrease in FRET upon glucose addition to the yeast expressing FLIPglu D236A or FLIPglu may be explained by the fact that glucose uptake by yeast leads to a decrease of cytosolic pH.
- **Figure 15:**: depicts the effect of histidine modification in FLIPglu on its pH sensitivity. The generation of pH insensitive FLIPglu was performed by the modification of the histidine side chain with different DEPC treatments. (A) No DEPC treatment. (B) 0.25 mM DEPC for 8 minutes. (C) 0.25 mM DEPC for 20 minutes (D) 0.25 mM DEPC for 40 minutes (B) 2.5 mM DEPC for 4 minutes. All treatments were done in absence of glucose.
- **Figure 16:**: shows the pH sensitivity of FLIPglu (A), FLIPglu-5AA (B) and FLIPglu- 10AA (C), which are lacking the last five (B) and ten amino acids (C) of GGBP, respectively, compared to wild-type GGBP (A), in the absence (■) and in the presence of 10 mM glucose (•). The difference of the ratio at, e.g., pH 5.5 and that at, e.g., pH 6.5 decreases with increasing C-terminal deletions.

The following Examples illustrate the invention:

### Experimental setup

### Recombinant DNA techniques

Unless stated otherwise in the examples, all recombinant DNA techniques are performed according to protocols as described in Sambrook and Russell (2001), Molecular Cloning: A Laboratory Manual, CSH Press or in Volumes 1 and 2 of Ausubel (1994), Current Protocols in Molecular Biology, Current Protocols. Standard materials and methods for plant molecular work are described in Plant Molecular Biology Labfase (1993) by R.D.D. Croy, jointly published by BIOS Scientific Publications Ltd. (UK) and Blackwell Scientific Publications (UK).

### Constructs and plasmids.

Genomic DNA from *Escherichia coli* K12 was extracted by the alkaline lysis method and used as template for malE and mglB in PCR amplification. The PCR was performed for 30 cycles (94°C for 30 sec, 55°C for 30 sec, 72°C for 2 min) using Taq polymerase and specific primers fitted with restriction sites (Table 2). The primers were designed in such a way that the leader peptide for protein secretion was removed. A final elongation of 30 min was performed and the PCR fragment was subsequently cloned into the pCRII TOPO vector (TA cloning kit, InVitrogen, The Netherlands). EYFP and ECFP (InVitrogen, The Netherlands) were used as template for PCR amplification using the Pfu polymerase (94°C for 30 sec, 45°C for 30 sec, 72°C for 2 min and 30 cycles) and a final elongation with Taq polymerase for subsequent cloning in pCRII TOPO vector. The primers for both templates were the same (Table 2) and were fitted with specific restrictions sites. The Notl-Spel fragment of ECFP was cloned into pBC (Stratagene, The Netherlands) (without Kpnl site), then the Spel-HindIII fragment of EYFP was added. The Kpnl-Kpnl malE or mglB fragment was cloned between ECFP and EYFP at the Kpnl restriction site. The orientation of the sequences encoding the binding proteins was determined by asymmetric restriction. The whole cassette was ligated into the pRSET B vector (Invitrogen, The Netherlands) by first digesting the vector with Sacl, blunting the overhangs, digesting with Hindlll and ligating into it the insert which had a filled Notl site and HindIII, thereby creating FLIPmal (coding sequence and amino acid sequence shown in SEQ ID NO: 14 and 15), FLIPglu (coding sequence and amino acid sequence shown in SEQ ID NO: 16 and 17) and FLIPrbs (coding sequence and amino acid sequence shown in SEQ ID NO: 46 and 47). FLIPmal (SEQ ID NO.15) comprise an ECFP portion (amino acid residues 1 to 239), a linker peptide (amino acid residues 240 to 245), an MBP portion (amino acid residues 246 to 615), a linker peptide (amino acid residues 616 to 621) and an EYFP portion (amino acid residues 622 to 860). FLIPglu (SEQ ID NO:17) comprises an ECFP portion (amino acid residues 1 to 239), a linker peptide (amino acid residues 240 to 245), a GGBP portion (amino acid residues 246 to 554), a linker peptide (amino acid residues 555 to 560) and a EYFP portion (amino acid residues 561 to 799). FLIPrbs (SEQ ID NO: 47) comprises an ECFP portion (amino acid residues 1 to 239), a linker peptide (amino acid residues 240 to 245), an RBS portion (amino acid residues 246 to 518; corresponding to the E. coli rbs gene shown under SEQ ID NO: 44), a linker peptide (amino acid residues 519 to 524) and an EYFP portion (amino acid residues 525 to 763). The final constructs were transferred into the BL21(DE3) gold E. coli strain. For expression in yeast, the yeast expression vector pDR195 (Rentsch, FEBS Letters 370, 264-268) and Saccharomyces cerevisiae SuSy7/ura3 expressing StSUT1 (Barker, Plant Cell 12 (2000), 1153-1164) and EBY4000 (Wieczorke, FEBS Letters 464 (1999), 123-128) have been used. After the PCR, all inserts were sequenced in both directions to verify the integrity of the sequence (Applied Biosystems).

In vitro mutagenesis was performed to modify single amino acids in the periplasmic binding protein. Self-annealing primers were designed that contain the modified codon. PCR was performed using FLIPmal or FLIPglu as template (95°C for 1 min, 68°C for 12 min and 14 cycles). The methylated plasmid was digested with Dpnl and BL21(DE3)gold E.coli strain was transformed. All FLIP constructs were resequenced to confirm both the mutated amino acid and the integrity of the rest of the sequences. With respect to the FLIPmal amino acid sequence of SEQ ID NO:15, the corresponding mutants show the respective substitution at following positions: FLIPmal W230A at position 475, FLIPmal W62A at position 307 and FLIPmal W340A at position 585. With respect to the FLIPglu amino acid sequence of SEQ ID NO:17, the corresponding mutants show the respective substitution at following positions: FLIPglu F16A at position 261 and FLIPglu D236A at position 481.

Furthermore, deletion constructs have also been prepared. FLIPmaI-5AA corresponds to FLIPmal with the exception that the first 5 N-terminal amino acid residues of the PBP portion (i.e. positions 246 to 250 in SEQ ID NO:15) are lacking. In FLIPglu-5AA and FLIPglu-10AA, the last 5 and 10 C-terminal amino acid residues of the PBP portion, respectively, are lacking compared to FLIPglu (i.e. positions 550 to 554 and 545 to 554, respectively, in SEQ ID NO:17).

**Table 2. Primers used for PCR. Restriction sites are underlined.**

| | |
|---|---|
| forward malE | (SEQ ID NO: 18) |
| CCGGTGGTACCGGAGGCGCCAAAATCGAAGAAGGTAAACTGGTAATCTGG | |
| reverse malE | (SEQ ID NO:19) |
| CATCCACCGGTACCGGCGCCCTTGGTGATACGAGTCTGCGCG | |
| forward mglB | (SEQ ID NO: 20) |
| CTGGTGGTACCGGAGGCGCCGCTGATACTCGCATTGGTGTAACA | |
| reverse mglB | (SEQ ID NO: 21) |
| TCTCCACCGGTACCGGCGCCTTTCTTGCTGAATTCAGCCAGGT | |
| forward ECFP | (SEQ ID NO: 22) |
| CACCGCGGCCGCATGGTGAGCAAGGGCGAGGAGC | |
| forward EYFP | (SEQ ID NO: 23) |
| ACCAACTAGTGGCGCCGGTACCGGTGGAATGGTGAGCAAGGGCGAGGAGC | |
| reverse ECFP | (SEQ ID NO: 24) |
| CGCTACTAGTGGCGCCTCCGGTACCACCCTTGTACAGCTCGTCCATGCCG | |
| reverse EYFP | (SEQ ID NO: 25) |
| CGCTAAGCTTTTACTTGTACAGCTCGTCCATGCCG | |
| forward malE W62A | (SEQ ID NO: 26) |
| CCTGACATTATCTTCGCGGCACACGACCGCTTTGGTGGCTACG | |
| reverse malE W62A | (SEQ ID NO:27) |
| GCGGTCGTGTGCCGCGAAGATAATGTCAGGGCCATCGC | |
| forward malE W230A | (SEQ ID NO: 28) |
| CATCAACGGCCCGGCGGCATGGTCCAACATCGACAC | |
| reverse malE W230A | (SEQ ID NO: 29) |
| GATGTTGGACCATGCCGCCGGGCCGTTGATGGTCATCG | |
| forward malE W340A | (SEQ ID NO: 30) |
| CAGATGTCCGCTTTCGCGTATGCCGTGCGTACTGCGGTGATC | |
| reverse malE W340A | (SEQ ID NO: 31) |
| GTACGCACGGCATACGCGAAAGCGGACATCTGCGGGATGTTC | |
| forward mglB F16A | (SEQ ID NO: 32) |
| TACGACGATAACGCGATGTCTGTAGTGCGCAAGGCTAT | |
| reverse mglB F16A | (SEQ ID NO: 33) |
| GCGCACTACAGACATCGCGTTATCGTCGTACTTATAGATTG | |
| forward mglB W183A | (SEQ ID NO: 34) |
| GATACCGCAATGGCGGACACCGCTCAGGCGAAAGATAA | |
| reverse mglB W183A | (SEQ ID NO: 35) |
| CTGAGCGGTGTCCGCCATTGCGGTATCTAACTGTAACTG | |
| forward mglB D236A | (SEQ.ID NO: 36) |
| CGGTGTTTGGCGTCGCGGCGCTGCCAGAAGCGCTGGCG | |
| reverse mglB D236A | (SEQ ID NO: 37) |
| GCGCTTCTGGCAGCGCCGCGACGCCAAACACCGGAATGCTGG | |
| forward ECFP/EYFPalone | (SEQ ID NO: 38) |
| GTGGATCCGGGCCGCATGGTGAGCAAGGGCGAGGAGCTG | |
| reverse ECFP/EYFPalone | (SEQ ID NO: 39) |
| CGCTAAGCTTTTACTTGTACAGCTCGTCCATGCCG | |
| forward ECFP2A fusion | (SEQ ID NO: 40) |
| TCCTCGAGATGGTGAGCAAGGGCGAGGA | |
| forward EYFP2Afusion | (SEQ ID NO: 41) |
| TTAAGCTAGCTGGCGATGTTGAGTCTAACCCTGGTCATATGGTGAGCAAGGGCGAGGA | |
| reverse ECFP2A fusion | (SEQ ID NO: 42) |
| GCCAGCTAGCTTAAGGAGATCGAAGTTGAGGAGCTGCTTGTA | |
| reverse EYFP2A fusion | (SEQ ID NO: 43) |
| CGGGATCCTTACTTGTACAGCTCGTCCATGC | |
| rbsforward | (SEQ ID NO: 48) |
| CGGCATGGACGAGCTGTACAAGGGTGGTACCGGAGGCGCCATGGCAAAAGACACCATCGCGCT | |
| rbsforward | (SEQ ID NO: 49) |
| GCTCCTCGCCCTTGCTCACCATTCCACCGGTACCGGCGCCCTGCTTAACAACCAGTTTCAGATCA | |

### FLIP protein purification

Bacterial cultures were inoculated from single colonies and grown for two to three days at 21°C in the dark. The cells were harvested by centrifugation, resuspended in 20 mM Tris-Cl, pH 7.9 and disrupted by six rounds of ultrasonication, 15 sec each. The FLIPs were purified by His-Bind affinity chromatography (HisBind Resin, Novagen, Wisconsin, USA). Binding to the resin was performed in batch at 4°C for 4h, and washed in column with 20 mM Tris-Cl and 20 mM Tris-HCl containing 20 mM Imidazol. 200 mM imidazole in Tris-Cl was used for elution.

### Determination of dissociation constant

Measurements were performed on three independent protein purifications from three independent bacterial transformants. Proteins were quantified using the Bradford method with BSA as standard. To avoid intermolecular FRET, binding assays were performed with 0.5 to 2 µM of the purified FLIPs.

The initial behaviour of the FLIPs was analyzed using spectrofluorometer SFM25 (Kontron) under saturating and unsaturating conditions by exciting ECFP at 433 nm and recording the emission between 450 nm and 650 nm. Substrate titration curves were obtained on microtiter fluorometer FL600 (BioTek). ECFP was excited using a 440/20 nm filter, ECFP and EYFP emissions were detected using a 530/20 nm and 485/30 nm filter.

### Beer analysis

Titrations were performed as the substrate titrations described above using different dilutions of beer in 20 mM sodium phosphate buffer at pH 7. Dilutions at half-saturation were determined by non-linear regression and transformed into concentrations using the sensors K_{d}s and equation: [C]=K_{d}/f (where [C], concentration; f, dilution at half-saturation). HPLC analysis was performed using Aminex 87-H column (Bio Rad, Hercules, CA, USA) and Rezek 10µ column (Phenomenex, Torrance, CA, USA) and a differential refractometer (LKB, Sweden) for detection.

### Substrate specificity

Substrate specificity was analysed using a microtiter fluorometer (FL600, BioTek, Highland Park, VT, USA) with the same settings as mentioned in section "Determination of dissociation constant". The purified sensors were incubated with different substrates in 20 mM sodium phosphate buffer at pH 7.

### Imaging

SuSy7/ura3 expressing StSUT1 and EBY4000 transformed with FLIPmal W230A and FLIPmal W340A in pDR195 were grown for three to five days in SD medium with 2% ethanol as the sole carbon source. For EBY4000 20 mg/l histidine, 20 mg/l tryptophan and 30 mg/l leucine were added to the medium. Confocal images were taken on a Leica DMRE microscope equipped with a confocal head TCS SP (Leica, Wetzlar, Germany). For imaging, yeast cells were transferred to a poly-lysine coated cover slide and immobilized using 2% alginate and Ca²⁺ in a total volume of 100 µl. Sugar solutions were added as volumes of 5 µl on top of the alginate embedded yeast. Imaging was performed on a fluorescence microscope (DMIRB, Leica, Wetzlar, Germany) with a cooled CCD camera (Sensys Photometrics, Tucson, AZ, USA). Dual emission intensity ratio was recorded using Metafluor 4.5 software (Universal Imaging, Media, PA, USA) with 436/20 excitation and two emission filters (480/40 for ECFP and 535/30 for EYFP) and a neutral density filter (1% transmission) on the excitation port.

### pH sensitivity measurements

Purified FLIPglu-variants were incubated with 20 mM sodium phosphate buffer at different pHs. For each pH, the mission intensity ratio was analyzed in the absence and presence of 10 mM substrate (e.g. glucose) using a microtiter fluorometer (FL600, BioTek, Highland Park, VT, USA).

### Example 1: Characterization of the fusion proteins (FLIP constructs)

The maltose binding protein (MBP), the glucose/galactose binding protein (GGBP) and the ribose binding protein (RBS) were used to construct fusion proteins, wherein the binding protein portions are flanked with green fluorescent protein (GFP) variants. These fusion proteins that are also called fluorescence indicator proteins (FLIPS) were tested for their utility as sensors to measure in vivo and in vitro concentrations of different compounds. The principle underlying such measurements is a conformational change of the binding protein portion upon binding of a compound. For instance, upon maltose binding, MBP undergoes a conformational shift that brings the C- and N-termini, which are located on different lobes and to which the GFPs are fused, by 10 A towards each other (Hall, J. Biol. Chem. 272 (1997), 17610-17614; calculated with Swiss PdpViewer v. 3.7b2 from the crystal structures of the ligand free [1OMBP] and ligand binding form [4MBP] of MBP). This reduction in distance alone is not sufficient for a detectable change in fluorescence resonance energy transfer (FRET) between the detection portions. Additionally, one of the lobes rotates by 35° and twists laterally by further 8° (Scharff, Biochem. 31 (1992), 10657-10663). Thus, in addition to the altered distance, the angular orientation of the chromophores changes, providing the basis for a detectable FRET change. Depending on the location of the C- and N-termini on the different lobes, observations of the 3D-crystal structures of ligand-free and ligand-bound periplasmic binding proteins (PBPs) indicate two possible mechanisms of FRET ratiometric measurements (Figure 1). Upon binding maltose, the N- and the C-termini of MBP change their angular orientation and move closer together thereby increasing the energy transfer (FRET between a pair of fluorescent donor and acceptor molecules attached to their extremities. On the other hand, according to the RBS crystal structures with and without bound substrate, upon binding of ribose the C- and N-termini in addition to a different angular orientation move further apart, hence decreasing FRET efficiency. The same situation applies for GGBP, which is structurally highly related to RBS. Here, the RBS crystal structure without ligand can be used as a model for the missing GGBP structure (Fukami-Kobayashi J. Mol. Biol. 286 (1999), 279-290). For all these fusion proteins the hinge-twist motion of the PBP changes both the distance and the angular orientation of the attached GFPs, providing a detectable change in FRET, which could not be observed due to the small change in distance alone.

Despite the pH sensitivity (Miyawaki, Nature 388 (1997), 882-887) and the chloride quenching effect on EYFP (Wachter, J. Mol. Biol. 301 (2000), 157-171), ECFP and EYFP are the best FRET partners currently available (Heim, Methods Enzymol. 302 (1999), 408-423). Using Citrine (EYFP with methionine instead of glutamine in position 69) the sensitivities of EYFP to pH and chloride ions can be reduced.

The two fluorescent proteins were fused to the PBP using rigid peptide linkers, which ensure proper folding of each moiety, i.e. the fluorescent proteins and the binding moiety. Furthermore, these linkers transmit the torsion and/or hinge-twist motion of the binding protein moiety to the detection portions.

Fusion proteins purified by Ni-NTA affinity chromatography were first characterized in *vitro.* MBP, RBS and GGBP display strong affinities to their substrates ranging from the higher nanomolar (GGBP: K_{d}=0,21 µM) to the low micromolar range (MBP: K_{d}=3 µM). In vitro characterization of fusion proteins require ligand-free binding sites. Classic size exclusion chromatography, Ni-NTA affinity chromatography and extended dialysis were not sufficient to remove residually bound substrate from the fusion protein containing wild-type MBP. Using Ni-NTA affinity chromatography, glucose and ribose, however, could be removed from the sensors based on GGBP (FLIPglu) and RBS (FLIPrbs), respectively.

### Example 2: FRET measurement of FLIPs in vitro

In order to increase the range of concentration for measurement, the fusion proteins (FLIPS) were mutagenised to decrease their substrate affinities. Additional to hydrogen bonding interactions, binding of maltose or glucose is achieved by stacking interactions between the aromatic residues in the binding site of the PBPs and their substrates (Nand, Science 241 (1988), 1290-1295; Spurlino et al. J Biol. Chem. 266 (1991), 5202-5219; Spurlino et al. J. Mol. Biol. 226 (1992), 15-22)). For MBP, three tryptophane residues are involved in maltose binding. Replacement of these residues with alanine reduces the affinity for maltose (Martineau, J. Mol. Biol. 214 (1990), 337-352). At position 230, a change of the tryptophane residue to alanine (W230A) causes an increase of 12 times of the K_{d}, a corresponding mutation at position 62 (W62A) increases the K_{d} by 67 times, and a corresponding mutation at position 340 (W340A) renders the K_{d} even more than 300 times higher (Martineau, J. Mol. Biol. 214 (1990), 337-352). In GGBP, phenylalanine in position 16 binds glucose by stacking interactions and aspartate in position 236 is involved in glucose binding by hydrogen bonding interactions (Nand, Science 241 (1988), 1290-1295). In order to obtain FLIPs that show decreased affinities to their substrates, the above residues were mutated into alanine by PCR based in vitro mutagenesis. The dissociation constant of the resulting FLIPs was determined by FRET.

FRET was determined as the emission intensity ratio between the donor ECFP (485 nm) and the acceptor EYFP (530 nm) after ECFP excitation at 430 nm. Using the ratio change upon ligand binding, dissociation constants (K_{d}) were calculated by fitting the substrate titration curves to the equation that describes the general binding of a ligand to a protein: S=[S]_{bound}/[P]ₜₒₜₐₗ=n[S]/(k_{d}+[S]), where [S] is the substrate concentration, [S]_{bound} the concentration of substrate that is bound to the binding protein, n the number of equal binding sites and [P]ₜₒₜₐₗ the total concentration of the binding protein (Bisswanger, Enzymkinetik, 3. Auflage, Wiley-VCH page 16). For increasing ratios with increasing substrate concentrations S=(R-Rₘᵢₙ)/(Rₘₐₓ-Rₘᵢₙ), for decreasing ratios with increasing substrate concentrations S=1-(R-Rₘᵢₙ)/(Rₘₐₓ-Rₘᵢₙ).

In all cases, the K_{d} of the FLIPs was similar to that of the binding protein alone as measured by Martineau (J. Mol. Biol. 214 (1990), 337-352) and Zukin, (Biochemistry 16 (1977), 381-386) indicating that the fluorescent proteins do not interfere with the binding capacities of the binding moieties. For FLIPmal W230A, a K_{d} of 25 µM and for FLIPmal W62A a K_{d} of 226 µM were obtained (Table 3). FLIPmal W340A displayed no binding activity towards maltose. As expected by the positions of the C- and N-termini in the MBP crystal structures with and without bound substrate, for FLIPmal W230A an increase in ratio with increasing maltose concentrations was observed with a possible range for maltose quantification from 2.8 µM to 225 µM (Figure 3). Surprisingly, for FLIPmal W62A, the ratio decreased with increasing maltose concentrations. Nevertheless, FLIPmaIW62A allowed maltose quantification from 25 µM to 2000 µM, indicating that in contrast to the W230A mutation changing tryptophane in position 62 to alanine not only affected affinity but also the three dimensional structure and/or the conformational change upon analyte binding.

**Table 3** FLIPs constructs tested so far. For constructs being useful for determining analyte concentrations, the column headed "properties" gives concentration ranges that can be measured with the respective FLIP construct and ΔRmax=Rₘₐₓ-Rₘᵢₙ , which is the maximum change in ratio between absence and saturation with analyte. FLIPmaI-5AA is lacking the first 5 N-terminal amino acids, FLIPglu-5AA the last 5 C-terminal amino acids and FLIPglu-10AA the last 10 C-terminal amino acids. GGBP binds both glucose and galactose. Those FLIPglu for which two k_{d} values are given were also titrated with galactose. The range for measurement was defined as being between 10% and 90% saturation.

**Table 3**

| Constructs (each NINTA-purified) | K_{d}⁺ FLIPs (in vitro) | K_{d} Binding protein | Properties⁺ | |
|---|---|---|---|---|
| FLIPmal | - | 3 µM¹ | Irreversible binding of maltose | |
| FLIPmalW230A | 25 µM | 37 µM¹ | 2.781-225.13 µM (1-200 µM) | ΔRmax = 0.2 |
| FLIPmalW340A | >100000 µM | > 1000 µM¹ | No binding of maltose | control sensor |
| FLIPmal-5AA | 2.3 µM | - | 0.26-21.12 µM (0.1-50 µM) | ΔRmax=0.2 |
| FLIPmalW62A | 226 µM | 200 µM¹ | 25.17-2038.98 µM (10-5000 µM) | ΔRmax = 0.1 |
| | | | | |
| FLIPglu | glucose 0.17 µM (0.19 µM) galactose n.d. | glucose 0.21 µM² galactose 0.48 µM² | 0.019-1.53 µM (0.01-5 µM)) | ΔRmax = 0.23 (0.26 |
| FLIPgluF16A | glucose 589 µM (480 µM) galactose n.d. | - - | 65.4-5301 µM (10-5000 µM) | ΔRmax = 0.29 (0.26) |
| FLIPgluD236A | - | - | No binding of glucose | control sensor |
| FLIPglu-5AA | glucose 0.54 µM (0.56 µM) galactose 0.66 µM (0.7 µM) | - - | 0.06-4.68 µM (0.05-10 µM) | ΔRmax= 0.30 (0.29) |
| FLIPglu-10AA | glucose 0.66 µM (0.22 µM) galactose 0.37 µM | - - | 0.07-5.94 µM 0.01-5 µM) | ΔRmax = 0.17 (0.16) |
| FLIPgluF16A-5AA | glucose 983 µM galactose nd | - - | 109.2-8847 µM | ΔRmax = 0.23 |
| FLIPrbs | ribose 0.33 µM | | 0.036 - 2.97 µM | ΔRmax = 0.1 |

| | | | | |
|---|---|---|---|---|
| 1 Martineau et al. (J. Mol. Biol., 214 (1990) 337-352) 2 Zukin et al. (Biochemistry, 8 (1977) 381-386) ⁺ Values in brackets represent the results of first, preliminary measurements | | | | |

Since, in contrast to FLIPmal, FLIPglu could be purified, substrate titrations with the fusion protein comprising wild-type GGBP were possible. A dissociation constant of 0.17 µM for glucose was determined, which is comparable to the K_{d} of GGBP alone that was previously measured as 0.21 µM (Zukin, Biochemistry 8 (1977), 381-386). The K_{d} of FLIPglu F16A was determined as 589 µM allowing glucose concentration quantification from 65 µM to 5300 µM. For FLIPrbs, a K_{d} of 0.33 µM was determined allowing ribose quantification from 0.04 µM to 3 µM.

In contrast to FLIPglu, FLIPmal could not be purified without residual bound maltose Thus, the binding of maltose to FLIPmal appears to be virtually irreversible. Since there is some evidence that the release of substrate is triggered by the interaction of the binding protein with the transmembrane components of the bacterial transport system, the N-terminus that might be involved in this interaction was truncated. The resulting FLIPmaI-5AA could be purified by Ni-NTA affinity chromatography. The purified protein binds maltose reversibly with a similar K_{d} as MBP alone (Table 3). In summary, it could be shown that, by mutagenesis of the FLIPs, it was possible to perform in vitro measurements of maltose concentrations ranging from 0.3 µM to 200 µM and glucose concentrations ranging from 0.02 µM to 8800 µM (Table 3).

### Example 3: FRET measurement of FLIPs in vivo

To determine the feasibility of the FLIPs for in vivo analyte quantification, a yeast strain without hexose uptake activity (Wieczorke, FEBS 464 (1999) 123-128) was transformed with FLIP expressing constructs. To allow the yeast to grow on maltose, a sucrose transporter (StSUT1) that also transports maltose was integrated (Riesmeier, Plant Cell (1993) 5, 1591-1598). The yeast transformed with the yeast expression vector pDR195 (Rentsch et al., FEBS Lett (1995) 370, 264-268) containing the FLIPglu coding sequence (SEQ ID NO:16) was grown for two days on maltose or for four to six days on ethanol, whereas the yeast expressing FLIPmal-5AA was grown for four to six days on ethanol. The cells were harvested by centrifugation and washed three times with 20 mM Na-phosphate buffer at pH 7. Ratiometric measurements were performed using a Biotek FL600 microtiterplate fluorimeter. Six wells were analyzed in parallel. Half of them were treated with analyte; the second half was used as a control. To determine the ratio change upon addition of analyte the emission intensity ratio of the untreated controls was substracted from the wells containing the yeast to which an analyte was added. As one could expect from the in vitro analysis data, for the FLIPglu expressing yeast, the EYFP-ECFP emission intensity ratio decreased upon addition of maltose whereas, for the FLIPmaI-5AA expressing yeast, the emission intensity ratio increased as compared to the untreated control. Since glucose can not be taken up by this yeast strain, addition of glucose to the FLIPglu expressing yeast did not change the ratio, whereas maltose after being taken up by the yeast was metabolized into cytosolic glucose that then was detected by FLIPglu (Figure 6).

To further determine the feasibility of using these FLIPs for *in vivo* analyte quantification, the above-mentioned yeast strain without hexose uptake activity was transformed with a construct expressing FLIPmal W230A. Confocal imaging of FLIPmal W230A expressing yeast cells showed that the fluorescent fusion protein was expressed in the cytosol, whereas no signal was detected in the vacuole (Fig. 9). Thus, FLIPmal W230A should allow direct monitoring of maltose uptake into the cytosol with a subcellular resolution. To keep initial cytosolic maltose concentration low and to monitor the highest possible change in FRET, yeast was grown on ethanol as sole carbon source. The yeast transformed with FLIPmaIW230A in yeast expression vector pDR195 (Rentsch et al., FEBS Lett (1995) 370, 264-268) was grown for three days on ethanol. 70 µl of the culture were pipetted into a well whose bottom was formed by a polylysine coated cover slide. The cells were fixed using alginate and Ca²⁺ in a total volume of 200 µl. Imaging was done on a Leica DMIRB inverted microscope with a cooled CCD camera (Sensys Photomectrics, Tucson AZ). Dual emission ratio was recorded using Metafluor 4.0 software (Universal Imaging, Media PA) and a 436/20 excitation filter, two emission filters (480/40 for ECFP and 535/30 for EYFP) and a neutral density filter (1% transmission) on the excitation port. Upon addition of 47 mM maltose after 4.13 min the 535nm/480nm emission intensity ratio increased rapidly after a short lag phase caused by diffusion through the embedded media (Figure 7). The maximum ratio change appeared to be more extensive than for the purified protein. Within the next minutes a subsequent decrease of the 535nm/480nm emission intensity ratio was observed indicating a decrease in the cytosolic maltose concentration due to metabolization. This data clearly shows the feasibility of using the FLIPs for *in vivo* analyte quantification.

The measurement depicted in Figure 7 was repeated (Figure 9A). According to the scale applied in Figure 9A, the 535/480nm emission intensity ratio increased upon addition of 50 mM extracellular maltose by 0.15 to 0.2, indicating that maltose was transported into the yeast cytosol, where it was recognized by FLIPmal W230A (n=69; Fig. 9A). The response was specific, since addition of sucrose had no effect (n=38; Fig. 9B). The increase was rapid in the first two minutes after addition of maltose followed by a sustained accumulation in the cytosol increasing over the next several minutes, and in most cases leaving a central section unstained, probably representing the vacuole. Since SuSy7/ura3 lacks maltase activity, the emission intensity ratio remains constant after reaching its maximum. When FLIPmal W230A was expressed in EBY4000, a yeast strain with constitutive maltase activity, the full change in ratio was detected, indicating that the enzyme, which has a K_{M} of 16.6 mM (Vanoni, Progress Nucl. Acid Res. Mol. Biol. 37 (1989), 281-322) for maltose is unable to reduce cytosolic maltose levels below saturation of FLIPmal W230A (Fig. 9D). Due to lower uptake rates, a reduction of the external maltose concentration to 0.5 mM lead to a delayed accumulation but not to a reduction in the maximal ratio, indicating that also in this case cytosolic maltose concentrations increased to a level, where all fusion proteins were saturated (Fig. 9D).

### Example 4: Using control sensors to detect changes in chloride concentration and pH in vitro

Analyte titrations of FLIPglu D236A and FLIPmal W340A showed that both FLIPs were incapable of analyte binding (see Example 2, supra). Thus, since for both FLIPs the ratio is not a function of the analyte concentration, they can be used as control sensors to detect in vivo and in vitro changes of chloride concentration and pH that might superimpose on changes of analyte concentrations. Since it has been shown that EYFP emission is reduced at increasing halide concentrations and decreasing pH (Miyawaki, Proc. Natl. Acat. Sci. USA 96 (1999), 2135-2140; Wachter, J. Mol. Biol. 301 (2000), 157-171; Jayaraman J. Biol. Chem. (2000), 6047-6050), changes of these parameters will interfere with the analyte measurements by decreasing the EYFP-ECFP emission intensity ratio. Thus, without a control sensor a decrease in ratio that is due to a change of analyte concentration cannot be distinguished from a decrease in ratio due to an increase of chloride concentration or a decrease of pH. To test whether FLIPmal W340A and FLIPglu D236A can be used as control sensors, titrations with increasing chloride concentrations and different pH values were performed. The resulting chloride and pH titration curves were then compared to those of FLIPmal W230A and FLIPglu.

While the titration curves of FLIPglu D236A followed those of FLIPglu, the titration curve of FLIPmal W340A followed those of FLIPmal W230A, indicating that the overall three-dimensional structure and the light-spectroscopic properties of the functional FLIP and its control sensor are almost identical (Figure 5). Thus, FLIPglu D236A can be used as a calibration tool for FLIPglu and FLIPmal W340A as a calibration tool for FLIPmal W230A to rule out changes in pH and chloride concentrations that might interfere with analyte detection.

The suitability of FLIPmal W340A as a control sensor has also been proven for in vivo applications. When expressed in SuSy7/ura3 yeast expressing StSUT1, FLIPmal W340A did not show any significant ratio change upon addition of maltose. Furthermore, since energy emission of FLIPmal W340A is similarly affected by pH and chloride concentration changes as FLIPmal W230A (Figure 5), the constance of FLIPmal W340A energy emission depicted in Figure 9C gives good evidence that the maltose measurements performed with FLIPmal W230A (Figure 9A and B) were not influenced by pH or halide concentration changes.

### Example 5: Detailed analysis of FLIPmal's capacity to measure maltose in vitro

In addition to maltose MBP can bind maltotriose, maltotetraose and other maltooligosaccharides (MOS) with K_{d}s as low as 0.2 µM in the case of maltotriose (Ferenci, Biochim. Biophys. Acta 860 (1986), 44-55). Nonetheless, mutants may possess different specificities. The FRET-based detection of conformational changes allows rapid determination of the substrate spectrum. Fourteen sugars were analysed using a microtiter plate assay (Fig. 10A, B). As compared to published data, FLIPmal-5AA and FLIPmal W230A were unaltered regarding their specificity. Both specifically recognize maltose but none of the tested pentoses, hexoses, sugar alcohols, disaccharides or trisaccharides, which lack the α-1,4-glucosidic link present in maltose (Fig. 10A, B). Both fusion proteins also did not bind D-glucose (data not shown). As expected, the FLIPmal nanosensors recognize maltotriose and longer chain MOS (Fig. 10C). In agreement with the reduced closing movement in the presence of longer α-1,4-oligomaltoside chains observed in crystal structures, the maximum change in ratio (at saturation) decreased with the length of the maltose chain, soluble starch giving the lowest ratio, while the affinity remained at a similar range (Fig. 10C).

To test whether the system can be used for rapid analysis of complex solutes, the maltose concentration in a local beer was measured. Beer was diluted and maltose was quantified in a microtiter plate assay. Titration allowed determination of maltose concentrations of 29.7 ± 1.8 mM using two different FLIP mutants (Fig. 10D). To confirm the data, HPLC analysis was performed on the same sample and maltose concentration was measured as 3.5 mM (data not shown). As compared to the measurements with pure maltose, the maximum change in ratio determined for beer was much smaller (ΔRₘₐₓ = 0.13), indicating that beer mainly contains MOS, which are not detected by HPLC analysis. This may explain the difference between the concentrations measured with both methods. After fermentation, beer contains maltose but also significant amounts of MOS and even soluble starch (Thomas, J. Am. Soc. Brewing Chem. 58 (2000), 124-127). The measured values are consistent with enzymatic measurements of MOS and with determinations using MBP-based bioelectronic nanosensors (Benson, Science 293 (2001), 1641-1644).

### Example 6: Determination of substrate specificity of different FLIPglu fusion proteins

In order to determine the substrate specificity of purified fusion protein containing wild-type GGBP (FLIPglu) in vitro, energy emission at 530 mm and 485 mm was measured during incubation with various pentoses, hexoses, sugar alcohols and di- and trisaccharides at three different concentrations (glucose: 0 mM, 1 mM and 10 mM; other compounds: 1 mM, 10 mM and 100 mM or 0.1 mM, 1 mM and 10 mM). Apart from glucose, FLIPglu bound diverse compounds including for instance rhamnose, L-glucose and sucrose as indicated by the decrease in the energy emission ratio upon incubation with these compounds (Figure 11).

In contrast, FLIPglu F16A surprisingly turned out to be clearly more glucose-specific than FLIPglu (Figure 12). Beside glucose, this fusion protein only showed a significant decrease in the energy emission ratio for galactose and ribose.

### Example 7: Investigations on the pH dependence of FLIPglu fusion proteins

The GGBP-derived control sensor FLIPglu D236A was expressed in the yeast cytosol. The ratio change upon addition of 50 mM external glucose (final concentration) was studied by FRET ratio measurement as described in Example 3 (supra). FLIPglu D236A exhibited a strong decrease in ratio shortly upon the addition of glucose (Figure 14A). A corresponding, albeit less pronounced reaction could be observed for yeast cells expressing FLIPglu (Figure 14B). Since glucose uptake in yeast is known to decrease cytosolic pH (Ramos, Gen. Microbiol. 135 (1989), 2413-2422), it was suspected that the control sensor responds to a pH change which may lie around pH 7.

To test this assumption, the pH dependence of FRET was measured *in vitro.* In contrast to the FLIPmal, FLIPglu shows a local maximum of FRET around pH 6.0-6.5 (Figure 13), indicating that protonation of a histidine amino acid side chain leads to conformational changes in FLIPglu. Effects on the GFPs were excluded since FLIPmal is insensitive to pH changes in this pH range. Modification of the histidine residues by carbethoxylation of the purified fusion protein using DEPC increased its pH insensitivity (Figure 15).

The results reported above gave rise to the conclusion that the control sensor FLIPglu D236A can be used to determine cytosolic pH. Furthermore, this decrease in ratio can also be used to calibrate glucose measurements performed with the functional glucose sensors that display the same pH sensitivity as the control sensor and where ratio changes due to glucose detection are superimposed by the effect of pH changes.

### Example 8: Influence of truncation of the GGBP portion in FLIPglu fusion proteins on pH sensitivity

Purified FLIPglu and the truncation products FLIPglu-5AA and FLIPglu-10AA which lack the C-terminal five and ten amino acids of GGBP, respectively, were titrated with different pH values in the absence and presence of 10 mM glucose. The energy emission ratio was recorded for each pH. In contrast to FLIPmal, the FLIPglu variants showed a maximum energy emission between pH 6.0 and 6.5, indicating that the overall GGBP conformation is pH sensitive (see Example 7). As compared to FLIPglu, truncation of the GGBP portion leads to a reduced difference between maximum and minimum energy emission within the physiological range, e.g. between pH 5.5 and 8.0, indicating that C-terminal truncations decrease the pH sensitivity of GGBP (Figure 16). As compared to FLIPglu and FLIPglu-5AA, FLIPglu-10AA showed a reduced change in energy emission between the bound and unbound state, which, however, is still sufficient for significant glucose detection.

## Claims

1. An *in vitro* method for detecting a compound in a cell comprising:
(a) supplying a cell which is genetically engineered with a nucleic acid molecule to express a fusion protein, said fusion protein comprising:
(i) two fluorescent protein detection portions, said detection portions comprising green fluorescent protein, or a mutant thereof encoded by a nucleic acid molecule which hybridises under stringent hybridisation conditions with the sequence SEQ ID NO: 5;
(ii) a periplasmic binding protein (PBP) portion between said two detection portions, the PBP portion having a first lobe-hinge-second lobe structure; and
(iii) respective linker peptides connecting the first and second detection portions with the PBP portion, each linker peptide comprising between 3 and 35 amino acid residues, said linker peptides providing a conformation of the fusion protein which has improved energy emission; wherein the first detection portion is fused to the first lobe of the PBP portion, and the second detection portion is fused to the second lobe of the PBP portion;
with energy suitable for exciting energy emission by said first and/or second detection portion;
(b) contacting the cell with the compound, wherein the PBP portion undergoes a conformational change upon binding of the compound; and
(c) measuring an increase or decrease of fluorescence resonance energy transfer between said two detection portions and thereby detecting the compound.

2. The method of claim 1, wherein the compound is selected from the group consisting of sugars, amino acids, peptides, organic acids, metals or ions, oxides, hydroxides or conjugates thereof, inorganic ions, (poly) amines and vitamins.

3. The method of claim 1 or 2, wherein the PBP portion, the first detection portion, and/or the second detection portion is truncated compared to the corresponding wild-type PBP portion, first detection portion, and/or second detection portion.

4. The method of any one of claims 1-3, wherein the first detection portion is enhanced cyan fluorescent protein (ECFP) and the second detection portion is enhanced yellow fluorescent protein (EYFP).

5. The method of any one of claims 1 to 4, wherein the fusion protein further comprises a targeting signal sequence.

6. The method of any one of claims 1 to 5, wherein the PBP portion is modified with respect to the corresponding wild-type PBP so that the binding affinity to the compound of said PBP portion is altered compared to the wild-type PBP.

7. The method of any one of claims 1 to 6, wherein the cell is a bacterial, fungus, insect, plant, yeast or animal cell.

8. The method of claim 7, wherein the animal cell is that of a mouse, rat or zebra fish.

9. The method according to claim 1, wherein each linker peptide comprises between 5 and 15 amino acid residues.

## Patentansprüche

1. In-vitro-Verfahren zum Nachweisen einer Verbindung in einer Zelle, umfassend:
(a) Versorgen einer Zelle, die mit einem Nukleinsäuremolekül gentechnisch so verändert wurde, dass sie ein Fusionsprotein exprimiert, wobei das Fusionsprotein umfasst:
(i) zwei fluoreszierende Proteinnachweisabschnitte, wobei die Nachweisabschnitte grün fluoreszierendes Protein oder eine Mutante davon umfassen, das bzw. die von einem Nukleinsäuremolekül codiert wird, das unter stringenten Hybridisierungsbedingungen mit der SEQ ID NO: 5 hybridisiert;
(ii) einen periplasmatischen Bindungsprotein-(PBP-) Abschnitt zwischen den zwei Nachweisabschnitten, wobei der PBP-Abschnitt eine Struktur aus erstem Lappen, Hinge und zweitem Lappen aufweist, und
(iii) entsprechende Linkerpeptide, die den ersten und zweiten Nachweisabschnitt mit dem PBP-Abschnitt verbinden, wobei jedes Linkerpeptid zwischen 3 und 35 Aminosäurereste umfasst, wobei die Linkerpeptide eine Konformation des Fusionsproteins bereitstellen, das eine verbesserte Energieemission hat; wobei der erste Nachweisabschnitt an den ersten Lappen des PBP-Abschnitts fusioniert ist und der zweite Nachweisabschnitt an den zweiten Lappen des PBP-Abschnitts fusioniert ist;
mit Energie, die sich zum Anregen der Energieemission durch den ersten und/oder zweiten Nachweisabschnitt eignet;
(b) Zusammenbringen der Zelle mit der Verbindung, wobei der PBP-Abschnitt eine Konformationsänderung beim Binden der Verbindung durchläuft; und
(c) Messen eines Anstiegs oder einer Abnahme der Fluoreszenzresonanzenergieübertragung zwischen den beiden Nachweisabschnitten und **dadurch** Nachweisen der Verbindung.

2. Verfahren nach Anspruch 1, wobei die Verbindung ausgewählt ist aus der Gruppe bestehend aus Zuckern, Aminosäuren, Peptiden, organischen Säuren, Metallen oder Ionen, Oxiden, Hydroxiden oder Konjugaten davon, anorganischen Ionen, (Poly)aminen und Vitaminen.

3. Verfahren nach Anspruch 1 oder 2, wobei der PBP-Abschnitt, der erste Nachweisabschnitt und/oder der zweite Nachweisabschnitt im Vergleich zu dem entsprechenden Wildtyp-PBP-Abschnitt, dem ersten Nachweisabschnitt und/oder dem zweiten Nachweisabschnitt verkürzt sind.

4. Verfahren nach einem der Ansprüche 1 bis 3, wobei der erste Nachweisabschnitt verstärktes cyan fluoreszierendes Protein (ECFP) ist und der zweite Nachweisabschnitt verstärktes gelb fluoreszierendes Protein (EYFP) ist.

5. Verfahren nach einem der Ansprüche 1 bis 4, wobei das Fusionsprotein zudem eine Targeting-Signalsequenz umfasst.

6. Verfahren nach einem der Ansprüche 1 bis 5, wobei der PBP-Abschnitt in Bezug auf das entsprechende Wildtyp-PBP so modifiziert ist, dass die Bindungsaffinität zu der Verbindung des PBP-Abschnitts im Vergleich zu dem Wildtyp-PBP verändert ist.

7. Verfahren nach einem der Ansprüche 1 bis 6, wobei die Zelle eine Bakterien-, Pilz-, Insekten-, Pflanzen-, Hefe- oder Tierzelle ist.

8. Verfahren nach Anspruch 7, wobei die Tierzelle aus einer Maus, einer Ratte oder einem Zebrafisch stammt.

9. Verfahren nach Anspruch 1, wobei jedes Linkerpeptid zwischen 5 und 15 Aminosäurereste umfasst.

## Revendications

1. Procédé in vitro pour détecter un composé dans une cellule, comprenant :
(a) la fourniture d'une cellule qui est génétiquement modifiée par une molécule d'acide nucléique pour exprimer une protéine de fusion, ladite protéine de fusion comprenant :
(i) deux portions de détection de type protéine fluorescente, lesdites portions de détection comprenant une protéine fluorescente verte ou un mutant de cette dernière, codé par une molécule d'acide nucléique qui s'hybride dans des conditions d'hybridation stringentes avec la séquence SEQ ID N°5 ;
(ii) une portion protéine de liaison périplasmique (PBP) entre lesdites deux portions de détection, la portion PBP ayant une structure premier lobe-charnière-second lobe ; et
(iii) des peptides lieurs respectifs, connectant la première et la deuxième portions de détection à la portion PBP, chaque peptide lieur comprenant de 3 à 35 résidus d'acides aminés, lesdits peptides lieurs fournissant une conformation de la protéine de fusion qui présente une émission d'énergie améliorée ; la première portion de détection étant fusionnée au premier lobe de la portion PBP, et la deuxième portion de détection étant fusionnée au deuxième lobe de la portion PBP ;
avec une énergie convenant à l'excitation de l'émission d'énergie par ladite première et/ou ladite deuxième portions de détection ;
(b) la mise en contact de la cellule avec le composé, la portion PBP subissant un changement de conformation après liaison du composé ; et
(c) la mesure d'une augmentation ou d'une diminution du transfert d'énergie de fluorescence par résonance entre lesdites deux portions de détection, de façon à détecter le composé.

2. Procédé de la revendication 1, dans lequel le composé est choisi dans le groupe consistant en les sucres, les acides aminés, les peptides, les acides organiques, les métaux ou ions, les oxydes, les hydroxydes ou les conjugués de ceux-ci, les ions inorganiques, les (poly)amines et les vitamines.

3. Procédé de la revendication 1 ou 2, dans lequel la portion PBP, la première portion de détection et/ou la deuxième portion de détection sont tronquées par comparaison avec la portion PBP, la première portion de détection et/ou la deuxième portion de détection de type sauvage correspondantes.

4. Procédé de l'une quelconque des revendications 1 à 3, dans lequel la première portion de détection est une protéine de fluorescence cyan renforcée (ECFP), et la deuxième portion de détection est une protéine de fluorescence jaune renforcée (EYFP).

5. Procédé de l'une quelconque des revendications 1 à 4, dans lequel la protéine de fusion comprend en outre une séquence signal de ciblage.

6. Procédé de l'une quelconque des revendications 1 à 5, dans lequel la portion PBP est modifiée par rapport au PBP de type sauvage correspondant, de telle sorte que l'affinité de liaison au composé de ladite portion PBP soit modifiée par comparaison avec le PBP de type sauvage.

7. Procédé de l'une quelconque des revendications 1 à 6, dans lequel la cellule est une cellule bactérienne, fongique, d'insecte, de plante, de levure ou d'animal.

8. Procédé de la revendication 7, dans lequel la cellule animale est celle d'une souris, d'un rat ou d'un fouille-roche.

9. Procédé selon la revendication 1, dans lequel chaque peptide de liaison comprend de 5 à 15 résidus d'acides aminés.


### SEQUENCE LISTING

<110> Frommer, Wolf
<120> Fusion proteins useful for detecting analytes
<130> F 2159 PCT
<160> 49
<170> PatentIn version 3.1
<210> 1
   <211> 1113
   <212> DNA
   <213> Escherichia coli
<220>
   <221> CDS
   <222> (1)..(1110)
   <223>
<400> 1
   aaa atc gaa gaa ggt aaa ctg gta atc tgg att aac ggc gat aaa ggc        48
   tat aac ggt ctc gct gaa gtc ggt aag aaa ttc gag aaa gat acc gga        96
   att aaa gtc acc gtt gag cat ccg gat aaa ctg gaa gag aaa ttc cca       144
   cag gtt gcg gca act ggc gat ggc cct gac att atc ttc tgg gca cac       192
   gac cgc ttt ggt ggc tac gct caa tct ggc ctg ttg gct gaa atc acc       240
   ccg gac aaa gcg ttc cag gac aag ctg tat ccg ttt acc tgg gat gcc       288
   gta cgt tac aac ggc aag ctg att gct tac ccg atc gct gtt gaa gcg       336
   tta tcg ctg att tat aac aaa gat ctg ctg ccg aac ccg cca aaa acc       384
   tgg gaa gag atc ccg gcg ctg gat aaa gaa ctg aaa gcg aaa ggt aag       432
   agc gcg ctg atg ttc aac ctg caa gaa ccg tac ttc acc tgg ccg ctg      480
   att gct gct gac ggg ggt tat gcg ttc aag tat gaa aac ggc aag tac      528
   gac att aaa gac gtg ggc gtg gat aac gct ggc gcg aaa gcg ggt ctg      576
   acc ttc ctg gtt gac ctg att aaa aac aaa cac atg aat gca gac acc      624
   gat tac tcc atc gca gaa gct gcc ttt aat aaa ggc gaa aca gcg atg      672
   acc atc aac ggc ccg tgg gca tgg tcc aac atc gac acc agc aaa gtg      720
   aat tat ggt gta acg gta ctg ccg acc ttc aag ggt caa cca tcc aaa      768
   ccg ttc gtt ggc gtg ctg agc gca ggt att aac gcc gcc agt ccg aac      816
   aaa gag ctg gcg aaa gag ttc ctc gaa aac tat ctg ctg act gat gaa      864
   ggt ctg gaa gcg gtt aat aaa gac aaa ccg ctg ggt gcc gta gcg ctg      912
   aag tct tac gag gaa gag ttg gcg aaa gat cca cgt att gcc gcc acc      960
   atg gaa aac gcc cag aaa ggt gaa atc atg ccg aac atc ccg cag atg     1008
   tcc gct ttc tgg tat gcc gtg cgt act gcg gtg atc aac gcc gcc agc     1056
   ggt cgt cag act gtc gat gaa gcc ctg aaa gac gcg cag act cgt atc     1104
   acc aag taa                                                         1113
<210> 2
   <211> 370
   <212> PRT
   <213> Escherichia coli
<400> 2
<210> 3
   <211> 930
   <212> DNA
   <213> Escherichia coli
<220>
   <221> CDS
   <222> (1)..(927)
   <223>
<400> 3
   gct gat act cgc att ggt gta aca atc tat aag tac gac gat aac ttt        48
   atg tct gta gtg cgc aag gct att gag caa gat gcg aaa gcc gcg cca      96
   gat gtt cag ctg ctg atg aat gat tct cag aat gac cag tcc aag cag      144
   aac gat cag atc gac gta ttg ctg gcg aaa ggg gtg aag gca ctg gca      192
   atc aac ctg gtt gac ccg gca gct gcg ggt acg gtg att gag aaa gcg      240
   cgt ggg caa aac gtg ccg gtg gtt ttc ttc aac aaa gaa ccg tct cgt      288
   aag gcg ctg gat agc tac gac aaa gcc tac tac gtt ggc act gac tcc      336
   aaa gag tcc ggc att att caa ggc gat ttg att gct aaa cac tgg gcg      384
   gcg aat cag ggt tgg gat ctg aac aaa gac ggt cag att cag ttc gta      432
   ctg ctg aaa ggt gaa ccg ggc cat ccg gat gca gaa gca cgt acc act      480
   tac gtg att aaa gaa ttg aac gat aaa ggc atc aaa act gaa cag tta      528
   cag tta gat acc gca atg tgg gac acc gct cag gcg aaa gat aag atg      576
   gac gcc tgg ctg tct ggc ccg aac gcc aac aaa atc gaa gtg gtt atc      624
   gcc aac aac gat gcg atg gca atg ggc gcg gtt gaa gcg ctg aaa gca      672
   cac aac aag tcc agc att ccg gtg ttt ggc gtc gat gcg ctg cca gaa      720
   gcg ctg gcg ctg gtg aaa tcc ggt gca ctg gcg ggc acc gta ctg aac      768
   gat gct aac aac cag gcg aaa gcg acc ttt gat ctg gcg aaa aac ctg      816
   gcc gat ggt aaa ggt gcg gct gat ggc acc aac tgg aaa atc gac aac      864
   aaa gtg gtc cgc gta cct tat gtt ggc gta gat aaa gac aac ctg gct      912
   gaa ttc agc aag aaa taa                                              930
<210> 4
   <211> 309
   <212> PRT
   <213> Escherichia coli
<400> 4
<210> 5
   <211> 238
   <212> PRT
   <213> Aequorea Victoria
<400> 5
   Met Gly Lys Gly Glu Glu Leu Phe Thr Gly Val Val Pro Ile Leu Val
   1 5 10 15
   Glu Leu Asp Gly Asp Val Asn Gly His Lys Phe Ser Val Ser Gly Glu
   20 25 30
   Gly Glu Gly Asp Ala Thr Tyr Gly Lys Leu Thr Leu Lys Phe Ile Cys
               35                  40                  45
   Thr Thr Gly Lys Leu Pro Val Pro Trp Pro Thr Leu Val Thr Thr Phe
       50                  55                  60
   Ser Tyr Gly Val Gln Cys Phe Ser Arg Tyr Pro Asp His Met Lys Arg
   65                  70                  75                  80
   His Asp Phe Phe Lys Ser Ala Met Pro Glu Gly Tyr Val Gln Glu Arg
                   85                  90                  95
   Thr Ile Phe Phe Lys Asp Asp Gly Asn Tyr Lys Thr Arg Ala Glu Val
               100                 105                 110
   Lys Phe Glu Gly Asp Thr Leu Val Asn Arg Ile Glu Leu Lys Gly Ile
           115                 120                 125
   Asp Phe Lys Glu Asp Gly Asn Ile Leu Gly His Lys Leu Glu Tyr Asn
       130                 135                 140
   Tyr Asn Ser His Asn Val Tyr Ile Met Ala Asp Lys Gln Lys Asn Gly
   145                 150                 155                 160
   Ile Lys Val Asn Phe Lys Ile Arg His Asn Ile Glu Asp Gly Ser Val
                   165                 170                 175
   Gln Leu Ala Asp His Tyr Gln Gln Asn Thr Pro Ile Gly Asp Gly Pro
               180                 185                 190
   Val Leu Leu Pro Asp Asn His Tyr Leu Ser Thr Gln Ser Ala Leu Ser
           195                 200                 205
   Lys Asp Pro Asn Glu Lys Arg Asp His Met Val Leu Leu Glu Phe Val
       210                 215                 220
   Thr Ala Ala Gly Ile Thr His Gly Met Asp Glu Leu Tyr Lys
   225                 230                 235
<210> 6
   <211> 22
   <212> PRT
   <213> Artificial sequence
<220>
   <223> target peptide sequence
<400> 6
   His Lys Thr Met Leu Pro Leu Pro Leu Ile Pro Ser Leu Leu Leu Ser
   1 5 10 15
   Leu Ser Ser Ala Glu Phe
   20
<210> 7
   <211> 4
   <212> PRT
   <213> Artificial sequence
<220>
   <223> target peptide sequence
<400> 7
   His Asp Glu Leu
   1
<210> 8
   <211> 15
   <212> PRT
   <213> Artificial sequence
<220>
   <223> target peptide sequence
<400> 8
   Gln Pro Ser Leu Lys Arg Met Lys Ile Gln Pro Ser Ser Gln Pro
   1 5 10 15
<210> 9
   <211> 7
   <212> PRT
   <213> Artificial sequence
<220>
   <223> target peptide sequence
<400> 9
   Met Gly Ser Ser Lys Ser Lys
   1 5
<210> 10
   <211> 4
   <212> PRT
   <213> Artificial sequence
<220>
   <223> target peptide sequence
<220>
   <221> MISC_FEATURE
   <222> (2)..(3)
   <223> Xaa = Val, Ile or Leu
<220>
   <221> MISC_FEATURE
   <222> (4)..(4)
   <223> Xaa = any amino acid
<400> 10
   Cys Xaa Xaa Xaa
   1
<210> 11
   <211> 5
   <212> PRT
   <213> Artificial sequence
<220>
   <223> target peptide sequence
<220>
   <221> MISC_FEATURE
   <222> (2)..(2)
   <223> Xaa = none or any amino acid
<220>
   <221> MISC_FEATURE
   <222> (4)..(5)
   <223> Xaa = any amino acid
<400> 11
   Lys Xaa Lys Xaa Xaa
   1               5
<210> 12
   <211> 7
   <212> PRT
   <213> Artificial sequence
<220>
   <223> target peptide sequence
<400> 12
   Pro Lys Lys Lys Arg Lys Val
   1 5
<210> 13
   <211> 28
   <212> PRT
   <213> Artificial sequence
<220>
   <223> target peptide sequence
<400> 13
   Met Ser Val Leu Thr Pro Leu Leu Leu Arg Gly Leu Thr Gly Ser Ala
   1 5 10 15
   Arg Arg Leu Pro Val Pro Arg Ala Lys Ile Ser Leu
   20 25
<210> 14
   <211> 2583
   <212> DNA
   <213> Artificial sequence
<220>
   <223> fusion protein FLIPmal
<220>
   <221> CDS
   <222> (1)..(2580)
   <223>
<220>
   <221> misc_feature
   <222> (1864)..(2580)
   <223> EYFP
<220>
   <221> misc_feature
   <222> (1846)..(1863)
   <223> linker peptide
<220>
   <221> misc_feature
   <222> (736)..(1845)
   <223> MBP
<220>
   <221> misc_feature
   <222> (718)..(735)
   <223> linker peptide
<220>
   <221> misc_feature
   <222> (1)..(717)
   <223> ECFP
<400> 14
   atg gtg agc aag ggc gag gag ctg ttc acc ggg gtg gtg ccc atc ctg      48
   Met Val Ser Lys Gly Glu Glu Leu Phe Thr Gly Val Val Pro Ile Leu
   1               5                   10                  15
   gtc gag ctg gac ggc gac gta aac ggc cac aag ttc agc gtg tcc ggc      96
   Val Glu Leu Asp Gly Asp Val Asn Gly His Lys Phe Ser Val Ser Gly
               20                  25                  30
   gag ggc gag ggc gat gcc acc tac ggc aag ctg acc ctg aag ttc atc     144
   Glu Gly Glu Gly Asp Ala Thr Tyr Gly Lys Leu Thr Leu Lys Phe Ile
           35                  40                  45
   tgc acc acc ggc aag ctg ccc gtg ccc tgg ccc acc ctc gtg acc acc     192
   Cys Thr Thr Gly Lys Leu Pro Val Pro Trp Pro Thr Leu Val Thr Thr
       50                  55                  60
   ctg acc tgg ggc gtg cag tgc ttc agc cgc tac ccc gac cac atg aag     240
   Leu Thr Trp Gly Val Gln Cys Phe Ser Arg Tyr Pro Asp His Met Lys
   65                  70                  75                  80
   cag cac gac ttc ttc aag tcc gcc atg ccc gaa ggc tac gtc cag gag     288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag     336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc     384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac     432
   Ile Asp Phe Lys Glu Asp Gly Asn Ile Leu Gly His Lys Leu Glu Tyr
       130                 135                 140
   aac tac atc agc cac aac gtc tat atc acc gcc gac aag cag aag aac     480
   Asn Tyr Ile Ser His Asn Val Tyr Ile Thr Ala Asp Lys Gln Lys Asn
   145                 150                 155                 160
   ggc atc aag gcc aac ttc aag atc cgc cac aac atc gag gac ggc agc     528
   Gly Ile Lys Ala Asn Phe Lys Ile Arg His Asn Ile Glu Asp Gly Ser
                   165                 170                 175
   gtg cag ctc gcc gac cac tac cag cag aac acc ccc atc ggc gac ggc     576
   Val Gln Leu Ala Asp His Tyr Gln Gln Asn Thr Pro Ile Gly Asp Gly
               180                 185                 190
   ccc gtg ctg ctg ccc gac aac cac tac ctg agc acc cag tcc gcc ctg     624
   Pro Val Leu Leu Pro Asp Asn His Tyr Leu Ser Thr Gln Ser Ala Leu
           195                 200                 205
   agc aaa gac ccc aac gag aag cgc gat cac atg gtc ctg ctg gag ttc     672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag ggt     720
   Val Thr Ala Ala Gly Ile Thr Leu Gly Met Asp Glu Leu Tyr Lys Gly
   225                 230                 235                 240
   ggt acc gga ggc gcc aaa atc gaa gaa ggt aaa ctg gta atc tgg att      768
   Gly Thr Gly Gly Ala Lys Ile Glu Glu Gly Lys Leu Val Ile Trp Ile
               245                 250                 255
   aac ggc gat aaa ggc tat aac ggt ctc gct gaa gtc ggt aag aaa ttc      816
   Asn Gly Asp Lys Gly Tyr Asn Gly Leu Ala Glu Val Gly Lys Lys Phe
           260                 265                 270
   gag aaa gat acc gga att aaa gtc acc gtt gag cat ccg gat aaa ctg      864
   Glu Lys Asp Thr Gly Ile Lys Val Thr Val Glu His Pro Asp Lys Leu
       275                 280                 285
   gaa gag aaa ttc cca cag gtt gcg gca act ggc gat ggc cct gac att      912
   Glu Glu Lys Phe Pro Gln Val Ala Ala Thr Gly Asp Gly Pro Asp Ile
   290                 295                 300
   atc ttc tgg gca cac gac cgc ttt ggt ggc tac gct caa tct ggc ctg      960
   Ile Phe Trp Ala His Asp Arg Phe Gly Gly Tyr Ala Gln Ser Gly Leu
   305                 310                 315                 320
   ttg gct gaa atc acc ccg gac aaa gcg ttc cag gac aag ctg tat ccg     1008
   Leu Ala Glu Ile Thr Pro Asp Lys Ala Phe Gln Asp Lys Leu Tyr Pro
               325                 330                 335
   ttt acc tgg gat gcc gta cgt tac aac ggc aag ctg att gct tac ccg     1056
   Phe Thr Trp Asp Ala Val Arg Tyr Asn Gly Lys Leu Ile Ala Tyr Pro
           340                 345                 350
   atc gct gtt gaa gcg tta tcg ctg att tat aac aaa gat ctg ctg ccg     1104
   Ile Ala Val Glu Ala Leu Ser Leu Ile Tyr Asn Lys Asp Leu Leu Pro
       355                 360                 365
   aac ccg cca aaa acc tgg gaa gag atc ccg gcg ctg gat aaa gaa ctg     1152
   Asn Pro Pro Lys Thr Trp Glu Glu Ile Pro Ala Leu Asp Lys Glu Leu
       370                 375                 380
   aaa gcg aaa ggt aag agc gcg ctg atg ttc aac ctg caa gaa ccg tac     1200
   Lys Ala Lys Gly Lys Ser Ala Leu Met Phe Asn Leu Gln Glu Pro Tyr
   385                 390                 395                 400
   ttc acc tgg ccg ctg att gct gct gac ggg ggt tat gcg ttc aag tat     1248
   Phe Thr Trp Pro Leu Ile Ala Ala Asp Gly Gly Tyr Ala Phe Lys Tyr
               405                 410                 415
   gaa aac ggc aag tac gac att aaa gac gtg ggc gtg gat aac gct ggc     1296
   Glu Asn Gly Lys Tyr Asp Ile Lys Asp Val Gly Val Asp Asn Ala Gly
           420                 425                 430
   gcg aaa gcg ggt ctg acc ttc ctg gtt gac ctg att aaa aac aaa cac     1344
   Ala Lys Ala Gly Leu Thr Phe Leu Val Asp Leu Ile Lys Asn Lys His
       435                 440                 445
   atg aat gca gac acc gat tac tcc atc gca gaa gct gcc ttt aat aaa     1392
   Met Asn Ala Asp Thr Asp Tyr Ser Ile Ala Glu Ala Ala Phe Asn Lys
       450                 455                 460
   ggc gaa aca gcg atg acc atc aac ggc ccg tgg gca tgg tcc aac atc     1440
   Gly Glu Thr Ala Met Thr Ile Asn Gly Pro Trp Ala Trp Ser Asn Ile
   465                 470                 475                 480
   gac acc agc aaa gtg aat tat ggt gta acg gta ctg ccg acc ttc aag     1488
   Asp Thr Ser Lys Val Asn Tyr Gly Val Thr Val Leu Pro Thr Phe Lys
                   485                 490                 495
   ggt caa cca tcc aaa ccg ttc gtt ggc gtg ctg agc gca ggt att aac     1536
   Gly Gln Pro Ser Lys Pro Phe Val Gly Val Leu Ser Ala Gly Ile Asn
               500                 505                 510
   gcc gcc agt ccg aac aaa gag ctg gcg aaa gag ttc ctc gaa aac tat     1584
   Ala Ala Ser Pro Asn Lys Glu Leu Ala Lys Glu Phe Leu Glu Asn Tyr
           515                 520                 525
   ctg ctg act gat gaa ggt ctg gaa gcg gtt aat aaa gac aaa ccg ctg     1632
   Leu Leu Thr Asp Glu Gly Leu Glu Ala Val Asn Lys Asp Lys Pro Leu
       530                 535                 540
   ggt gcc gta gcg ctg aag tct tac gag gaa gag ttg gcg aaa gat cca     1680
   Gly Ala Val Ala Leu Lys Ser Tyr Glu Glu Glu Leu Ala Lys Asp Pro
   545                 550                 555                 560
   cgt att gcc gcc acc atg gaa aac gcc cag aaa ggt gaa atc atg ccg     1728
   Arg Ile Ala Ala Thr Met Glu Asn Ala Gln Lys Gly Glu Ile Met Pro
                   565                 570                 575
   aac atc ccg cag atg tcc gct ttc tgg tat gcc gtg cgt act gcg gtg     1776
   Asn Ile Pro Gln Met Ser Ala Phe Trp Tyr Ala Val Arg Thr Ala Val
               580                 585                 590
   atc aac gcc gcc agc ggt cgt cag act gtc gat gaa gcc ctg aaa gac     1824
   Ile Asn Ala Ala Ser Gly Arg Gln Thr Val Asp Glu Ala Leu Lys Asp
           595                 600                 605
   gcg cag act cgt atc acc aag ggc gcc ggt acc ggt gga atg gtg agc     1872
   Ala Gln Thr Arg Ile Thr Lys Gly Ala Gly Thr Gly Gly Met Val Ser
       610                 615                 620
   aag ggc gag gag ctg ttc acc ggg gtg gtg ccc atc ctg gtc gag ctg     1920
   Lys Gly Glu Glu Leu Phe Thr Gly Val Val Pro Ile Leu Val Glu Leu
   625                 630                 635                 640
   gac ggc gac gta aac ggc cac aag ttc agc gtg tcc ggc gag ggc gag     1968
   Asp Gly Asp Val Asn Gly His Lys Phe Ser Val Ser Gly Glu Gly Glu
                   645                 650                 655
   ggc gat gcc acc tac ggc aag ctg acc ctg aag ttc atc tgc acc acc     2016
   Gly Asp Ala Thr Tyr Gly Lys Leu Thr Leu Lys Phe Ile Cys Thr Thr
               660                 665                 670
   ggc aag ctg ccc gtg ccc tgg ccc acc ctc gtg acc acc ttc ggc tac     2064
   Gly Lys Leu Pro Val Pro Trp Pro Thr Leu Val Thr Thr Phe Gly Tyr
           675                 680                 685
   ggc ctg cag tgc ttc gcc cgc tac ccc gac cac atg aag cag cac gac     2112
   Gly Leu Gln Cys Phe Ala Arg Tyr Pro Asp His Met Lys Gln His Asp
       690                 695                 700
   ttc ttc aag tcc gcc atg ccc gaa ggc tac gtc cag gag cgc acc atc     2160
   Phe Phe Lys Ser Ala Met Pro Glu Gly Tyr Val Gln Glu Arg Thr Ile
   705                 710                 715                 720
   ttc ttc aag gac gac ggc aac tac aag acc cgc gcc gag gtg aag ttc      2208
   Phe Phe Lys Asp Asp Gly Asn Tyr Lys Thr Arg Ala Glu Val Lys Phe
                   725                 730                 735
   gag ggc gac acc ctg gtg aac cgc atc gag ctg aag ggc atc gac ttc      2256
   Glu Gly Asp Thr Leu Val Asn Arg Ile Glu Leu Lys Gly Ile Asp Phe
               740                 745                 750
   aag gag gac ggc aac atc ctg ggg cac aag ctg gag tac aac tac aac      2304
   Lys Glu Asp Gly Asn Ile Leu Gly His Lys Leu Glu Tyr Asn Tyr Asn
           755                 760                 765
   agc cac aac gtc tat atc atg gcc gac aag cag aag aac ggc atc aag      2352
   Ser His Asn Val Tyr Ile Met Ala Asp Lys Gln Lys Asn Gly Ile Lys
       770                 775                 780
   gtg aac ttc aag atc cgc cac aac atc gag gac ggc agc gtg cag ctc      2400
   Val Asn Phe Lys Ile Arg His Asn Ile Glu Asp Gly Ser Val Gln Leu
   785                 790                 795                 800
   gcc gac cac tac cag cag aac acc ccc atc ggc gac ggc ccc gtg ctg      2448
   Ala Asp His Tyr Gln Gln Asn Thr Pro Ile Gly Asp Gly Pro Val Leu
                   805                 810                 815
   ctg ccc gac aac cac tac ctg agc tac cag tcc gcc ctg agc aaa gac      2496
   Leu Pro Asp Asn His Tyr Leu Ser Tyr Gln Ser Ala Leu Ser Lys Asp
               820                 825                 830
   ccc aac gag aag cgc gat cac atg gtc ctg ctg gag ttc gtg acc gcc      2544
   Pro Asn Glu Lys Arg Asp His Met Val Leu Leu Glu Phe Val Thr Ala
           835                 840                 845
   gcc ggg atc act ctc ggc atg gac gag ctg tac aag taa                  2583
   Ala Gly Ile Thr Leu Gly Met Asp Glu Leu Tyr Lys
       850                 855                 860
<210> 15
   <211> 860
   <212> PRT
   <213> Artificial sequence
<220>
   <223> fusion protein FLIPmal
<220>
   <221> misc_feature
   <222> (1864)..(2580)
   <223> EYFP
<220>
   <221> misc_feature
   <222> (1896)..(1863)
   <223> linker peptide
<220>
   <221> misc_feature
   <222> (736)..(1845)
   <223> MBP
<220>
   <221> misc_feature
   <222> (718)..(735)
   <223> linker peptide
<220>
   <221> misc_feature
   <222> (1)..(717)
   <223> ECFP
<400> 15
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Trp Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Ile Ser His Asn Val Tyr Ile Thr Ala Asp Lys Gln Lys Asn
   145                 150                 155                 160
   Gly Ile Lys Ala Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys Gly
   225                 230                 235                 240
   Gly Thr Gly Gly Ala Lys Ile Glu Glu Gly Lys Leu Val Ile Trp Ile
                   245                 250                 255
   Asn Gly Asp Lys Gly Tyr Asn Gly Leu Ala Glu Val Gly Lys Lys Phe
               260                 265                 270
   Glu Lys Asp Thr Gly Ile Lys Val Thr Val Glu His Pro Asp Lys Leu
           275                 280                 285
   Glu Glu Lys Phe Pro Gln Val Ala Ala Thr Gly Asp Gly Pro Asp Ile
       290                 295                 300
   Ile Phe Trp Ala His Asp Arg Phe Gly Gly Tyr Ala Gln Ser Gly Leu
   305                 310                 315                 320
   Leu Ala Glu Ile Thr Pro Asp Lys Ala Phe Gln Asp Lys Leu Tyr Pro
                   325                 330                 335
   Phe Thr Trp Asp Ala Val Arg Tyr Asn Gly Lys Leu Ile Ala Tyr Pro
               340                 345                 350
   Ile Ala Val Glu Ala Leu Ser Leu Ile Tyr Asn Lys Asp Leu Leu Pro
           355                 360                 365
   Asn Pro Pro Lys Thr Trp Glu Glu Ile Pro Ala Leu Asp Lys Glu Leu
       370                 375                 380
   Lys Ala Lys Gly Lys Ser Ala Leu Met Phe Asn Leu Gln Glu Pro Tyr
   385                 390                 395                 400
   Phe Thr Trp Pro Leu Ile Ala Ala Asp Gly Gly Tyr Ala Phe Lys Tyr
                   405                 410                 415
   Glu Asn Gly Lys Tyr Asp Ile Lys Asp Val Gly Val Asp Asn Ala Gly
               420                 425                 430
   Ala Lys Ala Gly Leu Thr Phe Leu Val Asp Leu Ile Lys Asn Lys His
           435                 440                 445
   Met Asn Ala Asp Thr Asp Tyr Ser Ile Ala Glu Ala Ala Phe Asn Lys
       450                 455                 460
   Gly Glu Thr Ala Met Thr Ile Asn Gly Pro Trp Ala Trp Ser Asn Ile
   465                 470                 475                 480
   Asp Thr Ser Lys Val Asn Tyr Gly Val Thr Val Leu Pro Thr Phe Lys
                   485                 490                 495
   Gly Gln Pro Ser Lys Pro Phe Val Gly Val Leu Ser Ala Gly Ile Asn
               500                 505                 510
   Ala Ala Ser Pro Asn Lys Glu Leu Ala Lys Glu Phe Leu Glu Asn Tyr
           515                 520                 525
   Leu Leu Thr Asp Glu Gly Leu Glu Ala Val Asn Lys Asp Lys Pro Leu
       530                 535                 540
   Gly Ala Val Ala Leu Lys Ser Tyr Glu Glu Glu Leu Ala Lys Asp Pro
   545                 550                 555                 560
   Arg Ile Ala Ala Thr Met Glu Asn Ala Gln Lys Gly Glu Ile Met Pro
                   565                 570                 575
   Asn Ile Pro Gln Met Ser Ala Phe Trp Tyr Ala Val Arg Thr Ala Val
               580                 585                 590
   Ile Asn Ala Ala Ser Gly Arg Gln Thr Val Asp Glu Ala Leu Lys Asp
           595                 600                 605
   Ala Gln Thr Arg Ile Thr Lys Gly Ala Gly Thr Gly Gly Met Val Ser
       610                 615                 620
   Lys Gly Glu Glu Leu Phe Thr Gly Val Val Pro Ile Leu Val Glu Leu
   625                 630                 635                 640
   Asp Gly Asp Val Asn Gly His Lys Phe Ser Val Ser Gly Glu Gly Glu
                   645                 650                 655
   Gly Asp Ala Thr Tyr Gly Lys Leu Thr Leu Lys Phe Ile Cys Thr Thr
               660                 665                 670
   Gly Lys Leu Pro Val Pro Trp Pro Thr Leu Val Thr Thr Phe Gly Tyr
         675                 680                 685
   Gly Leu Gln Cys Phe Ala Arg Tyr Pro Asp His Met Lys Gln His Asp
       690                 695                 700
   Phe Phe Lys Ser Ala Met Pro Glu Gly Tyr Val Gln Glu Arg Thr Ile
   705                 710                 715                 720
   Phe Phe Lys Asp Asp Gly Asn Tyr Lys Thr Arg Ala Glu Val Lys Phe
                   725                 730                 735
   Glu Gly Asp Thr Leu Val Asn Arg Ile Glu Leu Lys Gly Ile Asp Phe
               740                 745                 750
   Lys Glu Asp Gly Asn Ile Leu Gly His Lys Leu Glu Tyr Asn Tyr Asn
           755                 760                 765
   Ser His Asn Val Tyr Ile Met Ala Asp Lys Gln Lys Asn Gly Ile Lys
       770                 775                 780
   Val Asn Phe Lys Ile Arg His Asn Ile Glu Asp Gly Ser Val Gln Leu
   785                 790                 795                 800
   Ala Asp His Tyr Gln Gln Asn Thr Pro Ile Gly Asp Gly Pro Val Leu
                   805                 810                 815
   Leu Pro Asp Asn His Tyr Leu Ser Tyr Gln Ser Ala Leu Ser Lys Asp
               820                 825                 830
   Pro Asn Glu Lys Arg Asp His Met Val Leu Leu Glu Phe Val Thr Ala
           835                 840                 845
   Ala Gly Ile Thr Leu Gly Met Asp Glu Leu Tyr Lys
       850                 855                 860
<210> 16
   <211> 2400
   <212> DNA
   <213> Artificial sequence
<220>
   <223> fusion protein FLIPmal
<220>
   <221> CDS
   <222> (1)..(2397)
   <223>
<220>
   <221> misc_feature
   <222> (1681)..(2397)
   <223> EYFP
<220>
   <221> misc_feature
   <222> (1663)..(1680)
   <223> linker peptide
<220>
   <221> misc_feature
   <222> (736)..(1662)
   <223> GGBP
<220>
   <221> misc_feature
   <222> (718)..(735)
   <223> linker peptide
<220>
   <221> misc_feature
   <222> (1)..(717)
   <223> ECFP
<400> 16
   atg gtg agc aag ggc gag gag ctg ttc acc ggg gtg gtg ccc atc ctg       48
   Met Val Ser Lys Gly Glu Glu Leu Phe Thr Gly Val Val Pro Ile Leu
   1               5                   10                  15
   gtc gag ctg gac ggc gac gta aac ggc cac aag ttc agc gtg tcc ggc       96
   Val Glu Leu Asp Gly Asp Val Asn Gly His Lys Phe Ser Val Ser Gly
               20                  25                  30
   gag ggc gag ggc gat gcc acc tac ggc aag ctg acc ctg aag ttc atc      144
   Glu Gly Glu Gly Asp Ala Thr Tyr Gly Lys Leu Thr Leu Lys Phe Ile
           35                  40                  45
   tgc acc acc ggc aag ctg ccc gtg ccc tgg ccc acc ctc gtg acc acc      192
   Cys Thr Thr Gly Lys Leu Pro Val Pro Trp Pro Thr Leu Val Thr Thr
       50                  55                  60
   ctg acc tgg ggc gtg cag tgc ttc agc cgc tac ccc gac cac atg aag      240
   Leu Thr Trp Gly Val Gln Cys Phe Ser Arg Tyr Pro Asp His Met Lys
   65                  70                  75                  80
   cag cac gac ttc ttc aag tcc gcc atg ccc gaa ggc tac gtc cag gag      288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag      336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc      384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac      432
   Ile Asp Phe Lys Glu Asp Gly Asn Ile Leu Gly His Lys Leu Glu Tyr
       130                 135                 140
   aac tac atc agc cac aac gtc tat atc acc gcc gac aag cag aag aac      480
   Asn Tyr Ile Ser His Asn Val Tyr Ile Thr Ala Asp Lys Gln Lys Asn
   145                 150                 155                 160
   ggc atc aag gcc aac ttc aag atc cgc cac aac atc gag gac ggc agc      528
   Gly Ile Lys Ala Asn Phe Lys Ile Arg His Asn Ile Glu Asp Gly Ser
                   165                 170                 175
   gtg cag ctc gcc gac cac tac cag cag aac acc ccc atc ggc gac ggc      576
   Val Gln Leu Ala Asp His Tyr Gln Gln Asn Thr Pro Ile Gly Asp Gly
               180                 185                 190
   ccc gtg ctg ctg ccc gac aac cac tac ctg agc acc cag tcc gcc ctg      624
   Pro Val Leu Leu Pro Asp Asn His Tyr Leu Ser Thr Gln Ser Ala Leu
           195                 200                 205
   agc aaa gac ccc aac gag aag cgc gat cac atg gtc ctg ctg gag ttc      672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag ggt      720
   Val Thr Ala Ala Gly Ile Thr Leu Gly Met Asp Glu Leu Tyr Lys Gly
   225                 230                 235                 240
   ggt acc gga ggc gcc gct gat act cgc att ggt gta aca atc tat aag      768
   Gly Thr Gly Gly Ala Ala Asp Thr Arg Ile Gly Val Thr Ile Tyr Lys
                   245                 250                 255
   tac gac gat aac ttt atg tct gta gtg cgc aag gct att gag caa gat      816
   Tyr Asp Asp Asn Phe Met Ser Val Val Arg Lys Ala Ile Glu Gln Asp
               260                 265                 270
   gcg aaa gcc gcg cca gat gtt cag ctg ctg atg aat gat tct cag aat      864
   Ala Lys Ala Ala Pro Asp Val Gln Leu Leu Met Asn Asp Ser Gln Asn
           275                 280                 285
   gac cag tcc aag cag aac gat cag atc gac gta ttg ctg gcg aaa ggg      912
   Asp Gln Ser Lys Gln Asn Asp Gln Ile Asp Val Leu Leu Ala Lys Gly
       290                 295                 300
   gtg aag gca ctg gca atc aac ctg gtt gac ccg gca gct gcg ggt acg      960
   Val Lys Ala Leu Ala Ile Asn Leu Val Asp Pro Ala Ala Ala Gly Thr
   305                 310                 315                 320
   gtg att gag aaa gcg cgt ggg caa aac gtg ccg gtg gtt ttc ttc aac     1008
   Val Ile Glu Lys Ala Arg Gly Gln Asn Val Pro Val Val Phe Phe Asn
                   325                 330                 335
   aaa gaa ccg tct cgt aag gcg ctg gat agc tac gac aaa gcc tac tac     1056
   Lys Glu Pro Ser Arg Lys Ala Leu Asp Ser Tyr Asp Lys Ala Tyr Tyr
               340                 345                 350
   gtt ggc act gac tcc aaa gag tcc ggc att att caa ggc gat ttg att      1104
   Val Gly Thr Asp Ser Lys Glu Ser Gly Ile Ile Gln Gly Asp Leu Ile
           355                 360                 365
   gct aaa cac tgg gcg gcg aat cag ggt tgg gat ctg aac aaa gac ggt      1152
   Ala Lys His Trp Ala Ala Asn Gln Gly Trp Asp Leu Asn Lys Asp Gly
       370                 375                 380
   cag att cag ttc gta ctg ctg aaa ggt gaa ccg ggc cat ccg gat gca      1200
   Gln Ile Gln Phe Val Leu Leu Lys Gly Glu Pro Gly His Pro Asp Ala
   385                 390                 395                 400
   gaa gca cgt acc act tac gtg att aaa gaa ttg aac gat aaa ggc atc      1248
   Glu Ala Arg Thr Thr Tyr Val Ile Lys Glu Leu Asn Asp Lys Gly Ile
                   405                 410                 415
   aaa act gaa cag tta cag tta gat acc gca atg tgg gac acc gct cag      1296
   Lys Thr Glu Gln Leu Gln Leu Asp Thr Ala Met Trp Asp Thr Ala Gln
               420                 425                 430
   gcg aaa gat aag atg gac gcc tgg ctg tct ggc ccg aac gcc aac aaa      1344
   Ala Lys Asp Lys Met Asp Ala Trp Leu Ser Gly Pro Asn Ala Asn Lys
           435                 440                 445
   atc gaa gtg gtt atc gcc aac aac gat gcg atg gca atg ggc gcg gtt      1392
   Ile Glu Val Val Ile Ala Asn Asn Asp Ala Met Ala Met Gly Ala Val
       450                 455                 460
   gaa gcg ctg aaa gca cac aac aag tcc agc att ccg gtg ttt ggc gtc      1440
   Glu Ala Leu Lys Ala His Asn Lys Ser Ser Ile Pro Val Phe Gly Val
   465                 470                 475                 480
   gat gcg ctg cca gaa gcg ctg gcg ctg gtg aaa tcc ggt gca ctg gcg      1488
   Asp Ala Leu Pro Glu Ala Leu Ala Leu Val Lys Ser Gly Ala Leu Ala
                   485                 490                 495
   ggc acc gta ctg aac gat gct aac aac cag gcg aaa gcg acc ttt gat      1536
   Gly Thr Val Leu Asn Asp Ala Asn Asn Gln Ala Lys Ala Thr Phe Asp
               500                 505                 510
   ctg gcg aaa aac ctg gcc gat ggt aaa ggt gcg gct gat ggc acc aac      1584
   Leu Ala Lys Asn Leu Ala Asp Gly Lys Gly Ala Ala Asp Gly Thr Asn
           515                 520                 525
   tgg aaa atc gac aac aaa gtg gtc cgc gta cct tat gtt ggc gta gat      1632
   Trp Lys Ile Asp Asn Lys Val Val Arg Val Pro Tyr Val Gly Val Asp
       530                 535                 540
   aaa gac aac ctg gct gaa ttc agc aag aaa ggc gcc ggt acc ggt gga      1680
   Lys Asp Asn Leu Ala Glu Phe Ser Lys Lys Gly Ala Gly Thr Gly Gly
   545                 550                 555                 560
   atg gtg agc aag ggc gag gag ctg ttc acc ggg gtg gtg ccc atc ctg      1728
   Met Val Ser Lys Gly Glu Glu Leu Phe Thr Gly Val Val Pro Ile Leu
                   565                 570                 575
   gtc gag ctg gac ggc gac gta aac ggc cac aag ttc agc gtg tcc ggc      1776
   Val Glu Leu Asp Gly Asp Val Asn Gly His Lys Phe Ser Val Ser Gly
               580                 585                 590
    gag ggc gag ggc gat gcc acc tac ggc aag ctg acc ctg aag ttc atc     1824
   Glu Gly Glu Gly Asp Ala Thr Tyr Gly Lys Leu Thr Leu Lys Phe Ile
           595                 600                 605
   tgc acc acc ggc aag ctg ccc gtg ccc tgg ccc acc ctc gtg acc acc     1872
   Cys Thr Thr Gly Lys Leu Pro Val Pro Trp Pro Thr Leu Val Thr Thr
       610                 615                 620
   ttc ggc tac ggc ctg cag tgc ttc gcc cgc tac ccc gac cac atg aag     1920
   Phe Gly Tyr Gly Leu Gln Cys Phe Ala Arg Tyr Pro Asp His Met Lys
   625                 630                 635                 640
   cag cac gac ttc ttc aag tcc gcc atg ccc gaa ggc tac gtc cag gag     1968
   Gln His Asp Phe Phe Lys Ser Ala Met Pro Glu Gly Tyr Val Gln Glu
                   645                 650                 655
   cgc acc atc ttc ttc aag gac gac ggc aac tac aag acc cgc gcc gag     2016
   Arg Thr Ile Phe Phe Lys Asp Asp Gly Asn Tyr Lys Thr Arg Ala Glu
               660                 665                 670
   gtg aag ttc gag ggc gac acc ctg gtg aac cgc atc gag ctg aag ggc     2064
   Val Lys Phe Glu Gly Asp Thr Leu Val Asn Arg Ile Glu Leu Lys Gly
           675                 680                 685
   atc gac ttc aag gag gac ggc aac atc ctg ggg cac aag ctg gag tac     2112
   Ile Asp Phe Lys Glu Asp Gly Asn Ile Leu Gly His Lys Leu Glu Tyr
       690                 695                 700
   aac tac aac agc cac aac gtc tat atc atg gcc gac aag cag aag aac     2160
   Asn Tyr Asn Ser His Asn Val Tyr Ile Met Ala Asp Lys Gln Lys Asn
   705                 710                 715                 720
   ggc atc aag gtg aac ttc aag atc cgc cac aac atc gag gac ggc agc     2208
   Gly Ile Lys Val Asn Phe Lys Ile Arg His Asn Ile Glu Asp Gly Ser
                   725                 730                 735
   gtg cag ctc gcc gac cac tac cag cag aac acc ccc atc ggc gac ggc     2256
   Val Gln Leu Ala Asp His Tyr Gln Gln Asn Thr Pro Ile Gly Asp Gly
               740                 745                 750
   ccc gtg ctg ctg ccc gac aac cac tac ctg agc tac cag tcc gcc ctg     2304
   Pro Val Leu Leu Pro Asp Asn His Tyr Leu Ser Tyr Gln Ser Ala Leu
           755                 760                 765
   agc aaa gac ccc aac gag aag cgc gat cac atg gtc ctg ctg gag ttc     2352
   Ser Lys Asp Pro Asn Glu Lys Arg Asp His Met Val Leu Leu Glu Phe
       770                 775                 780
   gtg acc gcc gcc ggg atc act ctc ggc atg gac gag ctg tac aag taa     2400
   Val Thr Ala Ala Gly Ile Thr Leu Gly Met Asp Glu Leu Tyr Lys
   785                 790                 795
<210> 17
   <211> 799
   <212> PRT
   <213> Artificial sequence
<220>
   <223> fusion protein FLIPmal
<220>
   <221> misc_feature
   <222> (1681)..(2397)
   <223> EYFP
<220>
   <221> misc_feature
   <222> (1663)..(1680)
   <223> linker peptide
<220>
   <221> misc_feature
   <222> (736)..(1662)
   <223> GGBP
<220>
   <221> misc_feature
   <222> (718)..(735)
   <223> linker peptide
<220>
   <221> misc_feature
   <222> (1)..(717)
   <223> ECFP
<400> 17
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Trp Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
   130 135 140
   Asn Tyr Ile Ser His Asn Val Tyr Ile Thr Ala Asp Lys Gln Lys Asn
   145 150 155 160
   Gly Ile Lys Ala Asn Phe Lys Ile Arg His Asn Ile Glu Asp Gly Ser
   165 170 175
   Val Gln Leu Ala Asp His Tyr Gln Gln Asn Thr Pro Ile Gly Asp Gly
   180 185 190
   Pro Val Leu Leu Pro Asp Asn His Tyr Leu Ser Thr Gln Ser Ala Leu
   195 200 205
   Ser Lys Asp Pro Asn Glu Lys Arg Asp His Met Val Leu Leu Glu Phe
   210 215 220
   Val Thr Ala Ala Gly Ile Thr Leu Gly Met Asp Glu Leu Tyr Lys Gly
   225 230 235 240
   Gly Thr Gly Gly Ala Ala Asp Thr Arg Ile Gly Val Thr Ile Tyr Lys
   245 250 255
   Tyr Asp Asp Asn Phe Met Ser Val Val Arg Lys Ala Ile Glu Gln Asp
   260 265 270
   Ala Lys Ala Ala Pro Asp Val Gln Leu Leu Met Asn Asp Ser Gln Asn
   275 280 285
   Asp Gln Ser Lys Gln Asn Asp Gln Ile Asp Val Leu Leu Ala Lys Gly
   290 295 300
   Val Lys Ala Leu Ala Ile Asn Leu Val Asp Pro Ala Ala Ala Gly Thr
   305 310 315 320
   Val Ile Glu Lys Ala Arg Gly Gln Asn Val Pro Val Val Phe Phe Asn
   325 330 335
   Lys Glu Pro Ser Arg Lys Ala Leu Asp Ser Tyr Asp Lys Ala Tyr Tyr
   340 345 350
   Val Gly Thr Asp Ser Lys Glu Ser Gly Ile Ile Gln Gly Asp Leu Ile
   355 360 365
   Ala Lys His Trp Ala Ala Asn Gln Gly Trp Asp Leu Asn Lys Asp Gly
       370                 375                 380
   Gln Ile Gln Phe Val Leu Leu Lys Gly Glu Pro Gly His Pro Asp Ala
   385                 390                 395                 400
   Glu Ala Arg Thr Thr Tyr Val Ile Lys Glu Leu Asn Asp Lys Gly Ile
                   405                 410                 415
   Lys Thr Glu Gln Leu Gln Leu Asp Thr Ala Met Trp Asp Thr Ala Gln
               420                 425                 430
   Ala Lys Asp Lys Met Asp Ala Trp Leu Ser Gly Pro Asn Ala Asn Lys
           435                 440                 445
   Ile Glu Val Val Ile Ala Asn Asn Asp Ala Met Ala Met Gly Ala Val
       450                 455                 460
   Glu Ala Leu Lys Ala His Asn Lys Ser Ser Ile Pro Val Phe Gly Val
   465                 470                 475                 480
   Asp Ala Leu Pro Glu Ala Leu Ala Leu Val Lys Ser Gly Ala Leu Ala
                   485                 490                 495
   Gly Thr Val Leu Asn Asp Ala Asn Asn Gln Ala Lys Ala Thr Phe Asp
               500                 505                 510
   Leu Ala Lys Asn Leu Ala Asp Gly Lys Gly Ala Ala Asp Gly Thr Asn
           515                 520                 525
   Trp Lys Ile Asp Asn Lys Val Val Arg Val Pro Tyr Val Gly Val Asp
       530                 535                 540
   Lys Asp Asn Leu Ala Glu Phe Ser Lys Lys Gly Ala Gly Thr Gly Gly
   545                 550                 555                 560
   Met Val Ser Lys Gly Glu Glu Leu Phe Thr Gly Val Val Pro Ile Leu
                   565                 570                 575
   Val Glu Leu Asp Gly Asp Val Asn Gly His Lys Phe Ser Val Ser Gly
               580                 585                 590
   Glu Gly Glu Gly Asp Ala Thr Tyr Gly Lys Leu Thr Leu Lys Phe Ile
           595                 600                 605
   Cys Thr Thr Gly Lys Leu Pro Val Pro Trp Pro Thr Leu Val Thr Thr
       610                 615                 620
   Phe Gly Tyr Gly Leu Gln Cys Phe Ala Arg Tyr Pro Asp His Met Lys
   625                 630                 635                 640
   Gln His Asp Phe Phe Lys Ser Ala Met Pro Glu Gly Tyr Val Gln Glu
                   645                 650                 655
   Arg Thr Ile Phe Phe Lys Asp Asp Gly Asn Tyr Lys Thr Arg Ala Glu
               660                 665                 670
   Val Lys Phe Glu Gly Asp Thr Leu Val Asn Arg Ile Glu Leu Lys Gly
           675                 680                 685
   Ile Asp Phe Lys Glu Asp Gly Asn Ile Leu Gly His Lys Leu Glu Tyr
       690                 695                 700
   Asn Tyr Asn Ser His Asn Val Tyr Ile Met Ala Asp Lys Gln Lys Asn
   705                 710                 715                 720
   Gly Ile Lys Val Asn Phe Lys Ile Arg His Asn Ile Glu Asp Gly Ser
                   725                 730                 735
   Val Gln Leu Ala Asp His Tyr Gln Gln Asn Thr Pro Ile Gly Asp Gly
               740                 745                 750
   Pro Val Leu Leu Pro Asp Asn His Tyr Leu Ser Tyr Gln Ser Ala Leu
           755                 760                 765
   Ser Lys Asp Pro Asn Glu Lys Arg Asp His Met Val Leu Leu Glu Phe
       770                 775                 780
   Val Thr Ala Ala Gly Ile Thr Leu Gly Met Asp Glu Leu Tyr Lys
   785                 790                 795
<210> 18
   <211> 50
   <212> DNA
   <213> Artificial sequence
<220>
   <223> oligonucleotide primer
<400> 18
   ccggtggtac cggaggcgcc aaaatcgaag aaggtaaact ggtaatctgg 50
<210> 19
   <211> 42
   <212> DNA
   <213> Artificial sequence
<220>
   <223> oligonucleotide primer
<400> 19
   catccaccgg taccggcgcc cttggtgata cgagtctgcg cg 42
<210> 20
   <211> 44
   <212> DNA
   <213> Artificial sequence
<220>
   <223> oligonucleotide primer
<400> 20
   ctggtggtac cggaggcgcc gctgatactc gcattggtgt aaca 44
<210> 21
   <211> 43
   <212> DNA
   <213> Artificial sequence
<220>
   <223> oligonucleotide primer
<400> 21
   tctccaccgg taccggcgcc tttcttgctg aattcagcca ggt 43
<210> 22
   <211> 34
   <212> DNA
   <213> Artificial sequence
<220>
   <223> oligonucleotide primer
<400> 22
   caccgcggcc gcatggtgag caagggcgag gagc 34
<210> 23
   <211> 50
   <212> DNA
   <213> Artificial sequence
<220>
   <223> oligonucleotide primer
<400> 23
   accaactagt ggcgccggta ccggtggaat ggtgagcaag ggcgaggagc 50
<210> 24
   <211> 50
   <212> DNA
   <213> Artificial sequence
<220>
   <223> oligonucleotide primer
<400> 24
   cgctactagt ggcgcctccg gtaccaccct tgtacagctc gtccatgccg 50
<210> 25
   <211> 35
   <212> DNA
   <213> Artificial sequence
<220>
   <223> oligonucleotide primer
<400> 25
   cgctaagctt ttacttgtac agctcgtcca tgccg 35
<210> 26
   <211> 43
   <212> DNA
   <213> Artificial sequence
<220>
   <223> oligonucleotide primer
<400> 26
   cctgacatta tcttcgcggc acacgaccgc tttggtggct acg 43
<210> 27
   <211> 38
   <212> DNA
   <213> Artificial sequence
<220>
   <223> oligonucleotide primer
<400> 27
   gcggtcgtgt gccgcgaaga taatgtcagg gccatcgc 38
<210> 28
   <211> 36
   <212> DNA
   <213> Artificial sequence
<220>
   <223> oligonucleotide primer
<400> 28
   catcaacggc ccggcggcat ggtccaacat cgacac 36
<210> 29
   <211> 38
   <212> DNA
   <213> Artificial sequence
<220>
   <223> oligonucleotide primer
<400> 29
   gatgttggac catgccgccg ggccgttgat ggtcatcg 38
<210> 30
   <211> 42
   <212> DNA
   <213> Artificial sequence
<220>
   <223> oligonucleotide primer
<400> 30
   cagatgtccg ctttcgcgta tgccgtgcgt actgcggtga tc 42
<210> 31
   <211> 42
   <212> DNA
   <213> Artificial sequence
<220>
   <223> oligonucleotide primer
<400> 31
   gtacgcacgg catacgcgaa agcggacatc tgcgggatgt tc 42
<210> 32
   <211> 38
   <212> DNA
   <213> Artificial sequence
<220>
   <223> oligonucleotide primer
<400> 32
   tacgacgata acgcgatgtc tgtagtgcgc aaggctat 38
<210> 33
   <211> 41
   <212> DNA
   <213> Artificial sequence
<220>
   <223> oligonucleotide primer
<400> 33
   gcgcactaca gacatcgcgt tatcgtcgta cttatagatt g 41
<210> 34
   <211> 38
   <212> DNA
   <213> Artificial sequence
<220>
   <223> oligonucleotide primer
<400> 34
   gataccgcaa tggcggacac cgctcaggcg aaagataa 38 <210> 35

   <211> 39
   <212> DNA
   <213> Artificial sequence
<220>
   <223> oligonucleotide primer <400> 35

   ctgagcggtg tccgccattg cggtatctaa ctgtaactg 39
<210> 36
   <211> 38
   <212> DNA
   <213> Artificial sequence
<220>
   <223> oligonucleotide primer
<400> 36
   cggtgtttgg cgtcgcggcg ctgccagaag cgctggcg 38
<210> 37
   <211> 42
   <212> DNA
   <213> Artificial sequence
<220>
   <223> oligonucleotide primer
<400> 37
   gcgcttctgg cagcgccgcg acgccaaaca ccggaatgct gg 42
<210> 38
   <211> 39
   <212> DNA
   <213> Artificial sequence
<220>
   <223> oligonucleotide primer
<400> 38
   gtggatccgg gccgcatggt gagcaagggc gaggagctg 39
<210> 39
   <211> 35
   <212> DNA
   <213> Artificial sequence
<220>
   <223> oligonucleotide primer
<400> 39
   cgctaagctt ttacttgtac agctcgtcca tgccg 35
<210> 40
   <211> 28
   <212> DNA
   <213> Artificial sequence
<220>
   <223> oligonucleotide primer
<400> 40
   tcctcgagat ggtgagcaag ggcgagga 28
<210> 41
   <211> 58
   <212> DNA
   <213> Artificial sequence
<220>
   <223> oligonucleotide primer
<400> 41
   ttaagctagc tggcgatgtt gagtctaacc ctggtcatat ggtgagcaag ggcgagga 58
<210> 42
   <211> 42
   <212> DNA
   <213> Artificial sequence
<220>
   <223> oligonucleotide primer
<400> 42
   gccagctagc ttaaggagat cgaagttgag gagctgcttg ta 42
<210> 43
   <211> 31
   <212> DNA
   <213> Artificial sequence
<220>
   <223> oligonucleotide primer
<400> 43
   cgggatcctt acttgtacag ctcgtccatg c 31
<210> 44
   <211> 891
   <212> DNA
   <213> Escherichia coli
<220>
   <221> CDS
   <222> (1) .. (888)
   <223>
<220>
   <221> misc_feature
   <222> (1)..(69)
   <223> periplasm targeting sequence
<400> 44
   atg aac atg aaa aaa ctg gct acc ctg gtt tcc gct gtt gcg cta agc       48
   Met Asn Met Lys Lys Leu Ala Thr Leu Val Ser Ala Val Ala Leu Ser
   1               5                   10                  15
   gcc acc gtc agt gcg aat gcg atg gca aaa gac acc atc gcg ctg gtg       96
   Ala Thr Val Ser Ala Asn Ala Met Ala Lys Asp Thr Ile Ala Leu Val
               20                  25                  30
   gtc tcc acg ctt aac aac ccg ttc ttt gta tcg ctg aaa gat ggc gcg      144
   Val Ser Thr Leu Asn Asn Pro Phe Phe Val Ser Leu Lys Asp Gly Ala
           35                  40                  45
   cag aaa gag gcg gat aaa ctt ggc tat aac ctg gtg gtg ctg gac tcc      192
   Gln Lys Glu Ala Asp Lys Leu Gly Tyr Asn Leu Val Val Leu Asp Ser
       50                  55                  60
   cag aac aac ccg gcg aaa gag ctg gcg aac gtg cag gac tta acc gtt      240
   Gln Asn Asn Pro Ala Lys Glu Leu Ala Asn Val Gln Asp Leu Thr Val
   65                  70                  75                  80
   cgc ggc aca aaa att ctg ctg att aac ccg acc gac tcc gac gca gtg      288
   Arg Gly Thr Lys Ile Leu Leu Ile Asn Pro Thr Asp Ser Asp Ala Val
                   85                  90                  95
   ggt aat gct gtg aag atg gct aac cag gcg aac atc ccg gtt atc act      336
   Gly Asn Ala Val Lys Met Ala Asn Gln Ala Asn Ile Pro Val Ile Thr
               100                 105                 110
   ctt gac cgc cag gca acg aaa ggt gaa gtg gtg agc cac att gct tct      384
   Leu Asp Arg Gln Ala Thr Lys Gly Glu Val Val Ser His Ile Ala Ser
           115                 120                 125
   gat aac gta ctg ggc ggc aaa atc gct ggt gat tac atc gcg aag aaa      432
   Asp Asn Val Leu Gly Gly Lys Ile Ala Gly Asp Tyr Ile Ala Lys Lys
       130                 135                 140
   gcg ggt gaa ggt gcc aaa gtt atc gag ctg caa ggc att gct ggt aca      480
   Ala Gly Glu Gly Ala Lys Val Ile Glu Leu Gln Gly Ile Ala Gly Thr
   145                 150                 155                 160
   tcc gca gcc cgt gaa cgt ggc gaa ggc ttc cag cag gcc gtt gct gct      528
    Ser Ala Ala Arg Glu Arg Gly Glu Gly Phe Gln Gln Ala Val Ala Ala
                    165                 170                 175
   cac aag ttt aat gtt ctt gcc agc cag cca gca gat ttt gat cgc att       576
   His Lys Phe Asn Val Leu Ala Ser Gln Pro Ala Asp Phe Asp Arg Ile
               180                 185                 190
   aaa ggt ttg aac gta atg cag aac ctg ttg acc gct cat ccg gat gtt       624
   Lys Gly Leu Asn Val Met Gln Asn Leu Leu Thr Ala His Pro Asp Val
           195                 200                 205
   cag gct gta ttc gcg cag aat gat gaa atg gcg ctg ggg gcg ctg cgc       672
   Gln Ala Val Phe Ala Gln Asn Asp Glu Met Ala Leu Gly Ala Leu Arg
       210                 215                 220
   gca ctg caa act gcc ggt aaa tcg gat gtg atg gtc gtc gga ttt gac       720
   Ala Leu Gln Thr Ala Gly Lys Ser Asp Val Met Val Val Gly Phe Asp
   225                 230                 235                 240
   ggt aca ccg gat ggc gaa aaa gcg gtg aat gat ggc aaa cta gca gcg       768
   Gly Thr Pro Asp Gly Glu Lys Ala Val Asn Asp Gly Lys Leu Ala Ala
                   245                 250                 255
   act atc gct cag cta ccc gat cag att ggc gcg aaa ggc gtc gaa acc       816
   Thr Ile Ala Gln Leu Pro Asp Gln Ile Gly Ala Lys Gly Val Glu Thr
               260                 265                 270
   gca gat aaa gtg ctg aaa ggc gag aaa gtt cag gct aag tat ccg gtt       864
   Ala Asp Lys Val Leu Lys Gly Glu Lys Val Gln Ala Lys Tyr Pro Val
           275                 280                 285
   gat ctg aaa ctg gtt gtt aag cag tag                                   891
   Asp Leu Lys Leu Val Val Lys Gln
       290                 295
<210> 45
   <211> 296
   <212> PRT
   <213> Escherichia coli
<220>
   <221> misc_feature
   <222> (1)..(69)
   <223> periplasm targeting sequence
<400> 45
   Met Asn Met Lys Lys Leu Ala Thr Leu Val Ser Ala Val Ala Leu Ser
   1               5                   10                  15
   Ala Thr Val Ser Ala Asn Ala Met Ala Lys Asp Thr Ile Ala Leu Val
               20                  25                  30
   Val Ser Thr Leu Asn Asn Pro Phe Phe Val Ser Leu Lys Asp Gly Ala
           35                  40                  45
   Gln Lys Glu Ala Asp Lys Leu Gly Tyr Asn Leu Val Val Leu Asp Ser
       50                  55                  60
   Gln Asn Asn Pro Ala Lys Glu Leu Ala Asn Val Gln Asp Leu Thr Val
   65                  70                  75                  80
   Arg Gly Thr Lys Ile Leu Leu Ile Asn Pro Thr Asp Ser Asp Ala Val
                   85                  90                  95
   Gly Asn Ala Val Lys Met Ala Asn Gln Ala Asn Ile Pro Val Ile Thr
               100                 105                 110
   Leu Asp Arg Gln Ala Thr Lys Gly Glu Val Val Ser His Ile Ala Ser
           115                 120                 125
   Asp Asn Val Leu Gly Gly Lys Ile Ala Gly Asp Tyr Ile Ala Lys Lys
       130                 135                 140
   Ala Gly Glu Gly Ala Lys Val Ile Glu Leu Gln Gly Ile Ala Gly Thr
   145                 150                 155                 160
   Ser Ala Ala Arg Glu Arg Gly Glu Gly Phe Gln Gln Ala Val Ala Ala
                   165                 170                 175
   His Lys Phe Asn Val Leu Ala Ser Gln Pro Ala Asp Phe Asp Arg Ile
               180                 185                 190
   Lys Gly Leu Asn Val Met Gln Asn Leu Leu Thr Ala His Pro Asp Val
           195                 200                 205
   Gln Ala Val Phe Ala Gln Asn Asp Glu Met Ala Leu Gly Ala Leu Arg
       210                 215                 220
   Ala Leu Gln Thr Ala Gly Lys Ser Asp Val Met Val Val Gly Phe Asp
   225                 230                 235                 240
   Gly Thr Pro Asp Gly Glu Lys Ala Val Asn Asp Gly Lys Leu Ala Ala
                   245                 250                 255
   Thr Ile Ala Gln Leu Pro Asp Gln Ile Gly Ala Lys Gly Val Glu Thr
               260                 265                 270
   Ala Asp Lys Val Leu Lys Gly Glu Lys Val Gln Ala Lys Tyr Pro Val
           275                 280                 285
   Asp Leu Lys Leu Val Val Lys Gln
       290                 295
<210> 46
   <211> 2292
   <212> DNA
   <213> Artificial sequence
<220>
   <223> fusion protein FLIPrbs
<220>
   <221> CDS
   <222> (1)..(2289)
   <223>
<220>
   <221> misc_feature
   <222> (1573)..(2289)
   <223> EYFP
<220>
   <221> misc_feature
   <222> (1555)..(1572)
   <223> linker peptide
<220>
   <221> misc_feature
   <222> (736)..(1554)
   <223>
<220>
   <221> misc_feature
   <222> (718)..(735)
   <223> linker peptide
<220>
   <221> misc_feature
   <222> (1)..(717)
   <223> ECFP
<400> 46
   atg gtg agc aag ggc gag gag ctg ttc acc ggg gtg gtg ccc atc ctg        48
   Met Val Ser Lys Gly Glu Glu Leu Phe Thr Gly Val Val Pro Ile Leu
   1               5                   10                  15
   gtc gag ctg gac ggc gac gta aac ggc cac aag ttc agc gtg tcc ggc        96
   Val Glu Leu Asp Gly Asp Val Asn Gly His Lys Phe Ser Val Ser Gly
               20                  25                  30
   gag ggc gag ggc gat gcc acc tac ggc aag ctg acc ctg aag ttc atc       144
   Glu Gly Glu Gly Asp Ala Thr Tyr Gly Lys Leu Thr Leu Lys Phe Ile
           35                  40                  45
   tgc acc acc ggc aag ctg ccc gtg ccc tgg ccc acc ctc gtg acc acc      192
   Cys Thr Thr Gly Lys Leu Pro Val Pro Trp Pro Thr Leu Val Thr Thr
       50                  55                  60
   ctg acc tgg ggc gtg cag tgc ttc agc cgc tac ccc gac cac atg aag      240
   Leu Thr Trp Gly Val Gln Cys Phe Ser Arg Tyr Pro Asp His Met Lys
   65                  70                  75                  80
   cag cac gac ttc ttc aag tcc gcc atg ccc gaa ggc tac gtc cag gag      288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag      336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc      384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac      432
   Ile Asp Phe Lys Glu Asp Gly Asn Ile Leu Gly His Lys Leu Glu Tyr
       130                 135                 140
   aac tac atc agc cac aac gtc tat atc acc gcc gac aag cag aag aac      480
   Asn Tyr Ile Ser His Asn Val Tyr Ile Thr Ala Asp Lys Gln Lys Asn
   145                 150                 155                 160
   ggc atc aag gcc aac ttc aag atc cgc cac aac atc gag gac ggc agc      528
   Gly Ile Lys Ala Asn Phe Lys Ile Arg His Asn Ile Glu Asp Gly Ser
                   165                 170                 175
   gtg cag ctc gcc gac cac tac cag cag aac acc ccc atc ggc gac ggc      576
   Val Gln Leu Ala Asp His Tyr Gln Gln Asn Thr Pro Ile Gly Asp Gly
               180                 185                 190
   ccc gtg ctg ctg ccc gac aac cac tac ctg agc acc cag tcc gcc ctg      624
   Pro Val Leu Leu Pro Asp Asn His Tyr Leu Ser Thr Gln Ser Ala Leu
           195                 200                 205
   agc aaa gac ccc aac gag aag cgc gat cac atg gtc ctg ctg gag ttc      672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag ggt      720
   Val Thr Ala Ala Gly Ile Thr Leu Gly Met Asp Glu Leu Tyr Lys Gly
   225                 230                 235                 240
   ggt acc gga ggc gcc atg gca aaa gac acc atc gcg ctg gtg gtc tcc      768
   Gly Thr Gly Gly Ala Met Ala Lys Asp Thr Ile Ala Leu Val Val Ser
                   245                 250                 255
   acg ctt aac aac ccg ttc ttt gta tcg ctg aaa gat ggc gcg cag aaa      816
   Thr Leu Asn Asn Pro Phe Phe Val Ser Leu Lys Asp Gly Ala Gln Lys
               260                 265                 270
   gag gcg gat aaa ctt ggc tat aac ctg gtg gtg ctg gac tcc cag aac      864
   Glu Ala Asp Lys Leu Gly Tyr Asn Leu Val Val Leu Asp Ser Gln Asn
           275                 280                 285
   aac ccg gcg aaa gag ctg gcg aac gtg cag gac tta acc gtt cgc ggc      912
   Asn Pro Ala Lys Glu Leu Ala Asn Val Gln Asp Leu Thr Val Arg Gly
       290                 295                 300
   aca aaa att ctg ctg att aac ccg acc gac tcc gac gca gtg ggt aat      960
   Thr Lys Ile Leu Leu Ile Asn Pro Thr Asp Ser Asp Ala Val Gly Asn
   305                 310                 315                 320
   gct gtg aag atg gct aac cag gcg aac atc ccg gtt atc act ctt gac     1008
   Ala Val Lys Met Ala Asn Gln Ala Asn Ile Pro Val Ile Thr Leu Asp
                   325                 330                 335
   cgc cag gca acg aaa ggt gaa gtg gtg agc cac att gct tct gat aac     1056
   Arg Gln Ala Thr Lys Gly Glu Val Val Ser His Ile Ala Ser Asp Asn
               340                 345                 350
   gta ctg ggc ggc aaa atc gct ggt gat tac atc gcg aag aaa gcg ggt     1104
   Val Leu Gly Gly Lys Ile Ala Gly Asp Tyr Ile Ala Lys Lys Ala Gly
           355                 360                 365
   gaa ggt gcc aaa gtt atc gag ctg caa ggc att gct ggt aca tcc gca     1152
   Glu Gly Ala Lys Val Ile Glu Leu Gln Gly Ile Ala Gly Thr Ser Ala
       370                 375                 380
   gcc cgt gaa cgt ggc gaa ggc ttc cag cag gcc gtt gct gct cac aag     1200
   Ala Arg Glu Arg Gly Glu Gly Phe Gln Gln Ala Val Ala Ala His Lys
   385                 390                 395                 400
   ttt aat gtt ctt gcc agc cag cca gca gat ttt gat cgc att aaa ggt     1248
   Phe Asn Val Leu Ala Ser Gln Pro Ala Asp Phe Asp Arg Ile Lys Gly
                   405                 410                 415
   ttg aac gta atg cag aac ctg ttg acc gct cat ccg gat gtt cag gct     1296
   Leu Asn Val Met Gln Asn Leu Leu Thr Ala His Pro Asp Val Gln Ala
               420                 425                 430
   gta ttc gcg cag aat gat gaa atg gcg ctg ggg gcg ctg cgc gca ctg     1344
   Val Phe Ala Gln Asn Asp Glu Met Ala Leu Gly Ala Leu Arg Ala Leu
           435                 440                 445
   caa act gcc ggt aaa tcg gat gtg atg gtc gtc gga ttt gac ggt aca     1392
   Gln Thr Ala Gly Lys Ser Asp Val Met Val Val Gly Phe Asp Gly Thr
       450                 455                 460
   ccg gat ggc gaa aaa gcg gtg aat gat ggc aaa cta gca gcg act atc     1440
   Pro Asp Gly Glu Lys Ala Val Asn Asp Gly Lys Leu Ala Ala Thr Ile
   465                 470                 475                 480
   gct cag cta ccc gat cag att ggc gcg aaa ggc gtc gaa acc gca gat     1488
   Ala Gln Leu Pro Asp Gln Ile Gly Ala Lys Gly Val Glu Thr Ala Asp
                   485                 490                 495
   aaa gtg ctg aaa ggc gag aaa gtt cag gct aag tat ccg gtt gat ctg     1536
   Lys Val Leu Lys Gly Glu Lys Val Gln Ala Lys Tyr Pro Val Asp Leu
               500                 505                 510
   aaa ctg gtt gtt aag cag ggc gcc ggt acc ggt gga atg gtg agc aag     1584
   Lys Leu Val Val Lys Gln Gly Ala Gly Thr Gly Gly Met Val Ser Lys
           515                 520                 525
   ggc gag gag ctg ttc acc ggg gtg gtg ccc atc ctg gtc gag ctg gac      1632
   Gly Glu Glu Leu Phe Thr Gly Val Val Pro Ile Leu Val Glu Leu Asp
       530                 535                 540
   ggc gac gta aac ggc cac aag ttc agc gtg tcc ggc gag ggc gag ggc      1680
   Gly Asp Val Asn Gly His Lys Phe Ser Val Ser Gly Glu Gly Glu Gly
   545                 550                 555                 560
   gat gcc acc tac ggc aag ctg acc ctg aag ttc atc tgc acc acc ggc      1728
   Asp Ala Thr Tyr Gly Lys Leu Thr Leu Lys Phe Ile Cys Thr Thr Gly
                   565                 570                 575
   aag ctg ccc gtg ccc tgg ccc acc ctc gtg acc acc ttc ggc tac ggc      1776
   Lys Leu Pro Val Pro Trp Pro Thr Leu Val Thr Thr Phe Gly Tyr Gly
               580                 585                 590
   ctg cag tgc ttc gcc cgc tac ccc gac cac atg aag cag cac gac ttc      1824
   Leu Gln Cys Phe Ala Arg Tyr Pro Asp His Met Lys Gln His Asp Phe
           595                 600                 605
   ttc aag tcc gcc atg ccc gaa ggc tac gtc cag gag cgc acc atc ttc      1872
   Phe Lys Ser Ala Met Pro Glu Gly Tyr Val Gln Glu Arg Thr Ile Phe
       610                 615                 620
   ttc aag gac gac ggc aac tac aag acc cgc gcc gag gtg aag ttc gag      1920
   Phe Lys Asp Asp Gly Asn Tyr Lys Thr Arg Ala Glu Val Lys Phe Glu
   625                 630                 635                 640
   ggc gac acc ctg gtg aac cgc atc gag ctg aag ggc atc gac ttc aag      1968
   Gly Asp Thr Leu Val Asn Arg Ile Glu Leu Lys Gly Ile Asp Phe Lys
                   645                 650                 655
   gag gac ggc aac atc ctg ggg cac aag ctg gag tac aac tac aac agc      2016
   Glu Asp Gly Asn Ile Leu Gly His Lys Leu Glu Tyr Asn Tyr Asn Ser
               660                 665                 670
   cac aac gtc tat atc atg gcc gac aag cag aag aac ggc atc aag gtg      2064
   His Asn Val Tyr Ile Met Ala Asp Lys Gln Lys Asn Gly Ile Lys Val
           675                 680                 685
   aac ttc aag atc cgc cac aac atc gag gac ggc agc gtg cag ctc gcc      2112
   Asn Phe Lys Ile Arg His Asn Ile Glu Asp Gly Ser Val Gln Leu Ala
       690                 695                 700
   gac cac tac cag cag aac acc ccc atc ggc gac ggc ccc gtg ctg ctg      2160
   Asp His Tyr Gln Gln Asn Thr Pro Ile Gly Asp Gly Pro Val Leu Leu
   705                 710                 715                 720
   ccc gac aac cac tac ctg agc tac cag tcc gcc ctg agc aaa gac ccc      2208
   Pro Asp Asn His Tyr Leu Ser Tyr Gln Ser Ala Leu Ser Lys Asp Pro
                   725                 730                 735
   aac gag aag cgc gat cac atg gtc ctg ctg gag ttc gtg acc gcc gcc      2256
   Asn Glu Lys Arg Asp His Met Val Leu Leu Glu Phe Val Thr Ala Ala
               740                 745                 750
   ggg atc act ctc ggc atg gac gag ctg tac aag taa                      2292
   Gly Ile Thr Leu Gly Met Asp Glu Leu Tyr Lys
           755                 760
<210> 47
   <211> > 763
   <212> > PRT
   <213> Artificial sequence
<220>
   <223> fusion protein FLIPrbs
<220>
   <221> misc_feature
   <222> (1573)..(2289)
   <223> EYFP
<220>
   <221> misc_feature
   <222> (1555)..(1572)
   <223> linker peptide
<220>
   <221> misc_feature
   <222> (736)..(1554)
   <223>
<220>
   <221> misc_feature
   <222> (718)..(735)
   <223> linker peptide
<220>
   <221> misc_feature
   <222> (1)..(717)
   <223> ECFP
<400> 47
<210> 48
   <211> 63
   <212> DNA
   <213> Artificial sequence
<220>
   <223> oligonucleotide primer
<400> 48
   cggcatggac gagctgtaca agggtggtac cggaggcgcc atggcaaaag acaccatcgc     60
   gct                                                                   63
<210> 49
   <211> 65
   <212> DNA
   <213> Artificial sequence
<220>
   <223> oligonucleotide primer
<400> 49
   gctcctcgcc cttgctcacc attccaccgg taccggcgcc ctgcttaaca accagtttca     60
   gatca                                                                 65